# EUROPEAN PATENT SPECIFICATION

(11) **EP 3 234 133 B1**
(45) Date of publication and mention of the grant of the patent: **11.11.2020**
(21) Application number: 15828610.4
(22) Date of filing: 18.12.2015
(51) Int. Cl.: C12N 15/11

(54) **CRISPR-BASED COMPOSITIONS AND METHODS OF USE**
ZUSAMMENSETZUNGEN AUF CRISPR-BASIS UND VERFAHREN ZUR VERWENDUNG
COMPOSITIONS À BASE DE CRISPR ET LEURS PROCÉDÉS D'UTILISATION

(30) Priority: 18.12.2014 US 201462093588 P; 09.10.2015 US 201562239546 P
(43) Date of publication of application: 25.10.2017
(73) Proprietor: Integrated DNA Technologies, Inc., Coralville, IA 52241 (US)
(72) Inventor: COLLINGWOOD, Michael, Allen, North Liberty, IA 52317 (US); JACOBI, Ashley, Mae, North Liberty, IA 52317 (US); RETTIG, Garrett, Richard, Coralville, IA 52241 (US); SCHUBERT, Mollie, Sue, Cedar Rapids, IA 52402 (US); BEHLKE, Mark, Aaron, Coralville, IA 62241 (US)
(74) Representative: Modiano, Micaela Nadia
(86) International application number: PCT/US2015/066942
(87) International publication number: WO 2016/100951

(56) References cited:
- WO-A1-2014/065596
- WO-A1-2014/124226
- WO-A1-2016/080097
- WO-A1-2016/089433
- WO-A2-2014/144592
- US-A1- 2014 273 232
- M. JINEK ET AL: "A Programmable Dual-RNA-Guided DNA Endonuclease in Adaptive Bacterial Immunity", SCIENCE, vol. 337, no. 6096, 17 August 2012 (2012-08-17), pages 816-821, XP055229606, US ISSN: 0036-8075, DOI: 10.1126/science.1225829 cited in the application -& M. JINEK ET AL: "A Programmable Dual-RNA-Guided DNA Endonuclease in Adaptive Bacterial Immunity (Supplementary Material)", SCIENCE, vol. 337, no. 6096, 28 June 2012 (2012-06-28), XP055067747, US ISSN: 0036-8075, DOI: 10.1126/science.1225829
- REGINA CENCIC ET AL: "Protospacer Adjacent Motif (PAM)-Distal Sequences Engage CRISPR Cas9 DNA Target Cleavage", PLOS ONE, vol. 9, no. 10, 2 October 2014 (2014-10-02), page e109213, XP055175713, DOI: 10.1371/journal.pone.0109213
- MEGHDAD RAHDAR ET AL: "Synthetic CRISPR RNA-Cas9-guided genome editing in human cells", PROCEEDINGS OF THE NATIONAL ACADEMY OF SCIENCES, 16 November 2015 (2015-11-16), XP055264777, US ISSN: 0027-8424, DOI: 10.1073/pnas.1520883112
- AYAL HENDEL ET AL: "Chemically modified guide RNAs enhance CRISPR-Cas genome editing in human primary cells", NATURE BIOTECHNOLOGY, vol. 33, no. 9, 29 June 2015 (2015-06-29), pages 985-989, XP055233915, US ISSN: 1087-0156, DOI: 10.1038/nbt.3290

## Description

### SEQUENCE LISTING

The instant application contains a Sequence Listing that has been submitted in ASCII format via EFS-Web. The ASCII copy, created on December 18, 2015, is named IDT01-008-US_ST25.txt, and is 177,163 bytes in size.

### FIELD OF THE INVENTION

This disclosure pertains to modified compositions for use in CRISPR systems, and their methods of use.

### BACKGROUND OF THE INVENTION

The use of clustered regularly interspaced short palindromic repeats (CRISPR) and associated Cas proteins (CRISPR-Cas system) for site-specific DNA cleavage has shown great potential for a number of biological applications. CRISPR is used for genome editing; the genome-scale-specific targeting of transcriptional repressors (CRISPRi) and activators (CRISPRa) to endogenous genes; and other applications of RNA-directed DNA targeting with Cas enzymes.

CRISPR-Cas systems are native to bacteria and Archaea to provide adaptive immunity against viruses and plasmids. There are three classes of CRISPR-Cas systems that could potentially be adapted for research and therapeutic reagents, but Type-II CRISPR systems have a desirable characteristic in utilizing a single CRISPR associated (Cas) nuclease (specifically Cas9) in a complex with the appropriate guide RNAs - either a 2-part RNA system similar to the natural complex in bacteria comprising a CRISPR-activating RNA:trans-activating crRNA (crRNA:tracrRNA) pair or an artificial chimeric single-guide-RNA (sgRNA) - to mediate double-stranded cleavage of target DNA. In mammalian systems, these RNAs have been introduced by transfection of DNA cassettes containing RNA Pol III promoters (such as U6 or H1) driving RNA transcription, viral vectors, and single-stranded RNA following *in vitro* transcription (see Xu, T., et al., Appl Environ Microbiol, 2014. 80(5): p. 1544-52).

In the CRISPR-Cas9 system, using, for example, the system present in *Streptococcus pyogenes* as an example (*S.py.* or Spy), native crRNAs are about 42 bp long, containing a 5'-region of about 20 bases complementary to a target sequence (also referred to as a protospacer sequence) and a 3' region typically about 22 bases long that corresponds to a complementary region of the tracrRNA sequence. The native tracrRNAs are about 85-90 bases long, having a 5'-region containing the region complementary to the crRNA as well as about a 10-base region 5'-upstream. The remaining 3' region of the tracrRNA includes secondary structures (herein referred to as the "tracrRNA 3'-tail").

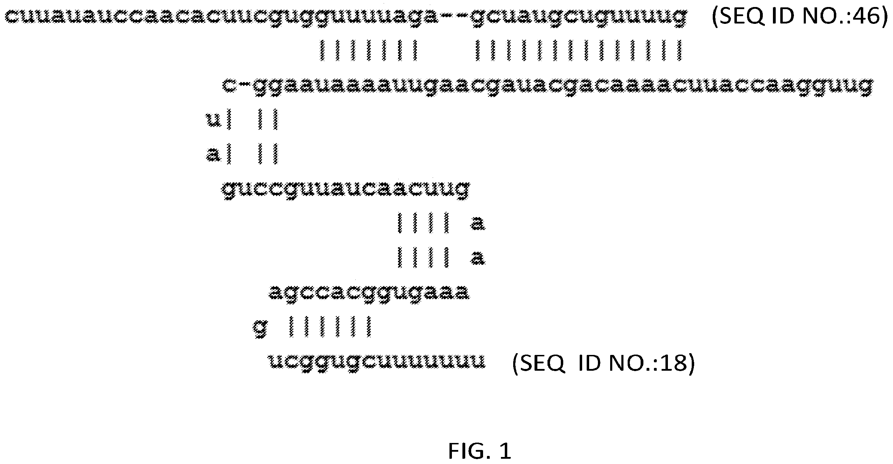

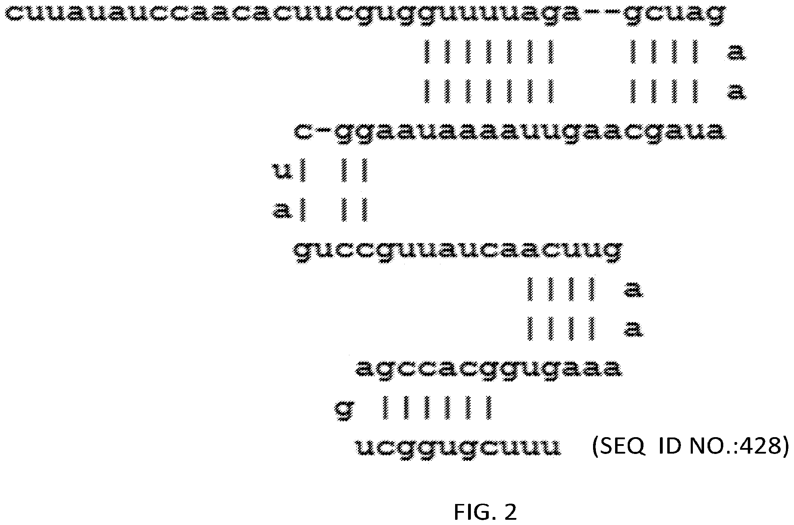

Jinek et al. extensively investigated the portions of the crRNA and tracrRNA that are required for proper functioning of the CRISPR-Cas9 system (Science, 2012. 337(6096): p. 816-21). They devised a truncated crRNA:tracrRNA fragment that could still function in CRISPR-Cas9 wherein the crRNA was the wild type 42 nucleotides and the tracrRNA was truncated to 75 nucleotides. They also developed an embodiment wherein the crRNA and tracrRNA are attached with a linker loop, forming a single guide RNA (sgRNA), which varies between 99-123 nucleotides in different embodiments. The configuration of the native 2-part crRNA:tracrRNA complex is shown in FIG. 1 and the 99 nucleotide embodiment of the artificial sgRNA single guide is shown in FIG. 2.

At least two groups have elucidated the crystal structure of *Streptococcus pyogenes* Cas9 (SpyCas9). In Jinek, M., et al., the structure did not show the nuclease in complex with either a guide RNA or target DNA. They carried out molecular modeling experiments to reveal predictive interactions between the protein in complex with RNA and DNA (Science, 2014. 343, p. 1215, DOI: 10.1126/science/1247997).

In Nishimasu, H., et al., the crystal structure of SpyCas9 is shown in complex with sgRNA and its target DNA at 2.5 angstrom resolution (Cell, 2014. 156(5): p. 935-49). The crystal structure identified two lobes to the Cas9 enzyme: a recognition lobe (REC) and a nuclease lobe (NUC). The sgRNA:target DNA heteroduplex (negatively charged) sits in the positively charged groove between the two lobes. The REC lobe, which shows no structural similarity with known proteins and therefore likely a Cas9-specific functional domain, interacts with the portions of the crRNA and tracrRNA that are complementary to each other.

Another group, Briner et al. (Mol Cell, 2014. 56(2): p. 333-9), identified and characterized the six conserved modules within native crRNA:tracrRNA duplexes and sgRNA.

US2014273232 (A1) discloses vectors and vector systems, some of which encode one or more components of a CRISPR complex, as well as methods for the design and use of such vectors. Also provided are methods of directing CRISPR complex formation in eukaryotic cells and methods for selecting specific cells by introducing precise mutations utilizing the CRISPR-Cas system.

WO2014144592 (A2) discloses methods for increasing specificity of RNA-guided genome editing, e.g., editing using CRISPR/Cas9 systems, using truncated guide RNAs (tru-gRNAs).

WO2014065596 (A1) relates to a composition for cleaving a target DNA in eukaryotic cells or organisms comprising a guide RNA specific for the target DNA and Cas protein-encoding nucleic acid or Cas protein, and use thereof.

WO2014124226 (A1) discloses compositions and methods for selectively reducing the amount of antibiotic resistant and/or virulent bacteria in a mixed bacteria population, or for reducing any other type of unwanted bacteria in a mixed bacteria population. The compositions and methods involve targeting bacteria that are differentiated from other members of the population by at least one unique clustered regularly interspaced short palindromic repeats (CRISPR) targeted DNA sequence.

Regina Cencic et al, "Protospacer Adjacent Motif (PAM)-Distal Sequences Engage CRISPR Cas9 DNA Target Cleavage", PLOS ONE, (20141002), vol. 9, no. 10, teach using chromatin immunoprecipitation followed by sequencing (ChIP-seq), to delineate the genome-wide binding panorama of catalytically inactive Cas9 directed by two different single guide (sg) RNAs targeting the Trp53 locus. Interactions between the 5' end of the guide and PAM-distal target sequences are found necessary to efficiently engage Cas9 nucleolytic activity, providing an explanation for why off-target editing is significantly lower than expected from ChIP-seq data.

**The** CRISPR-Cas9 system is utilized in genomic engineering as follows: a portion of the crRNA hybridizes to a target sequence, a portion of the tracrRNA hybridizes to a portion of the crRNA, and the Cas9 nuclease binds to the entire construct and directs cleavage. The Cas9 contains two domains homologous to endonucleases HNH and RuvC, wherein the HNH domain cleaves the DNA strand complementary to the crRNA and the RuvC-like domain cleaves the noncomplementary strand. This results in a double-stranded break in the genomic DNA. When repaired by non-homologous end joining (NHEJ) the break is typically shifted by 1 or more bases, leading to disruption of the natural DNA sequence and in many cases leading to a frameshift mutation if the event occurs in the coding exon of a protein-encoding gene. The break by also be repaired by homology dependent recombination (HDR), which permits insertion of new genetic material via experimental manipulation into the cut site created by Cas9 cleavage.

Some of the current methods for guide RNA delivery into mammalian cells include transfection of double-stranded DNA (dsDNA) containing RNA Pol III promoters for endogenous transcription, viral delivery, transfection of RNAs as *in vitro* transcription (IVT) products, or microinjection of IVT products. There are disadvantages to each of these methods. Unmodified exogenous RNA introduced into mammalian cells is known to initiate the innate immune response via recognition by Toll-like Receptors (TLRs), RIG-I, OASI and others receptors that recognize pathogen-associated molecular patterns (PAMPs). However, in most published studies, RNA which has been *in vitro* transcribed (IVT) by a T7 RNA polymerase is delivered to the cells. This type of RNA payload has been shown to be a trigger for the innate immune response. The alternative delivery methods described above each have their own disadvantages as well. For example, dsDNA cassettes can lead to integration, guide RNA transcription driven endogenously by a RNA Pol II promoter can persist constitutively, and the amount of RNA transcribed is uncontrollable.

RNA is quickly degraded by nucleases present in serum and in cells. Unmodified CRISPR RNA triggers (crRNAs, tracrRNAs, and sgRNAs) made by IVT methods or chemical synthesis are quickly degraded during delivery or after delivery to mammalian cells. Greater activity would be realized if the RNA was chemically modified to gain nuclease resistance. The most potent degradative activity present in serum and in cells is a 3'-exonuclease (Eder et al., Antisense Research and Development 1:141-151, 1991). Thus "end blocking" a synthetic oligonucleotide often improves nuclease stability. Chemical modification of single-stranded antisense oligonucleotides (ASOs) and double-stranded small interfering RNAs (siRNAs) has been well studied and successful approaches are in practice today (for reviews, see: Kurreck, Eur. J. Biochem., 270:1628-1644, 2003; Behlke, Oligonucleotides, 18:305-320, 2008; Lennox et al., Gene Therapy, 18:1111-1120, 2011). It is therefore desirable to devise chemical modification strategies for use with the RNA components of CRISPR/Cas. While the basic toolbox of chemical modifications available is well known to those with skill in the art, the effects that site-specific modification have on the interaction of a RNA species and an effector protein are not easily predicted and effective modification patterns usually must be empirically determined. In some cases, sequence of the RNA may influence the effectiveness of a modification pattern, requiring adjustment of the modification pattern employed for different sequence contexts, making practical application of such methods more challenging.

There is therefore a need to modify the guide RNA to reduce its toxicity to cells and to extend lifespan and functionality in mammalian cells while still performing their intended purpose in the CRISPR-Cas system. The methods and compositions described herein provide RNA and modified RNA oligonucleotides for use in a CRISPR-Cas system.

### SUMMARY OF THE INVENTION

In a first embodiment, the instant invention provides an isolated tracrRNA comprising a chemically-modified form of one of SEQ ID NOs.:2, 18, 30-33 and 36-39, wherein the isolated tracrRNA displays activity in a Clustered Regularly Interspaced Short Palindromic Repeats (CRISPR)-CRISPR associated (Cas) (CRISPR-Cas) endonuclease system, wherein the chemically-modified form of one of SEQ ID NOs.:2, 18, 30-33 and 36-39 comprises a chemically-modified nucleotide having an end-modifying group selected from a group consisting of a propanediol (C3) spacer, N,N-diethyl-4-(4-nitronaphthalen-1-ylazo)-phenylamine ("ZEN"), and an inverted-dT residue

In a second embodiment, the instant invention provides an isolated crRNA comprising a chemically-modified form of formula (I):

5'-X-Z-3' (I),

wherein X represents sequences comprising a target-specific protospacer domain comprising from 17 nucleotides to 20 nucleotides and Z represents sequences comprising a tracrRNA-binding domain comprising from 12 nucleotides to 19 nucleotides,
wherein the isolated crRNA displays activity in a Clustered Regularly Interspaced Short Palindromic Repeats (CRISPR)-CRISPR associated (Cas) (CRISPR-Cas) endonuclease system¬,
wherein the chemically-modified form of formula (I) comprises a chemically-modified nucleotide having an end-modifying group selected from the group consisting of a propanediol (C3) spacer, N,N-diethyl-4-(4-nitronaphthalen-1-ylazo)-phenylamine ("ZEN"), and an inverted-dT residue

In a third embodiment, the instant invention provides an isolated tracrRNA selected from the group consisting of SEQ ID NOs.:11-13, 17, 87-106, 110-114, 116, 117, 126, 129, 130, 131, 132, 134, 136, 144, 145, and 147-156, 159, 160, 449 and 451, wherein the isolated tracrRNA is active in a Clustered Regularly Interspaced Short Palindromic Repeats (CRISPR)/CRISPR-associated protein endonuclease system.

In a fourth embodiment, the instant invention provides an isolated crRNA selected from the group consisting of SEQ ID NOs.: 14, 164, 166-169, 171, 174-178, 186, 187, 189-207, 212-214, 221-224, 226-228, 233-249, 252, 256-259, 264-271, 291-374, 376, 377, 379-384, 430-437, and 445-448, wherein the isolated crRNA is active in a Clustered Regularly Interspaced Short Palindromic Repeats (CRISPR)/CRISPR-associated protein endonuclease system.

### BRIEF DESCRIPTION OF THE DRAWINGS

FIG. 1 [reference] is an illustration of a wild-type (WT) natural 2-part crRNA:tracrRNA complex with a 42 base unmodified crRNA (SEQ ID No. 46_) and an 89 base unmodified tracrRNA (SEQ ID No. 18). Lowercase letters represent RNA.
FIG. 2 [reference] is an illustration of a 99 base artificial single-guide RNA (SEQ ID NO: 428_) (sgRNA) that fuses the crRNA and tracrRNA elements into a single sequence through the addition of a new hairpin loop. Lowercase letters represent RNA.

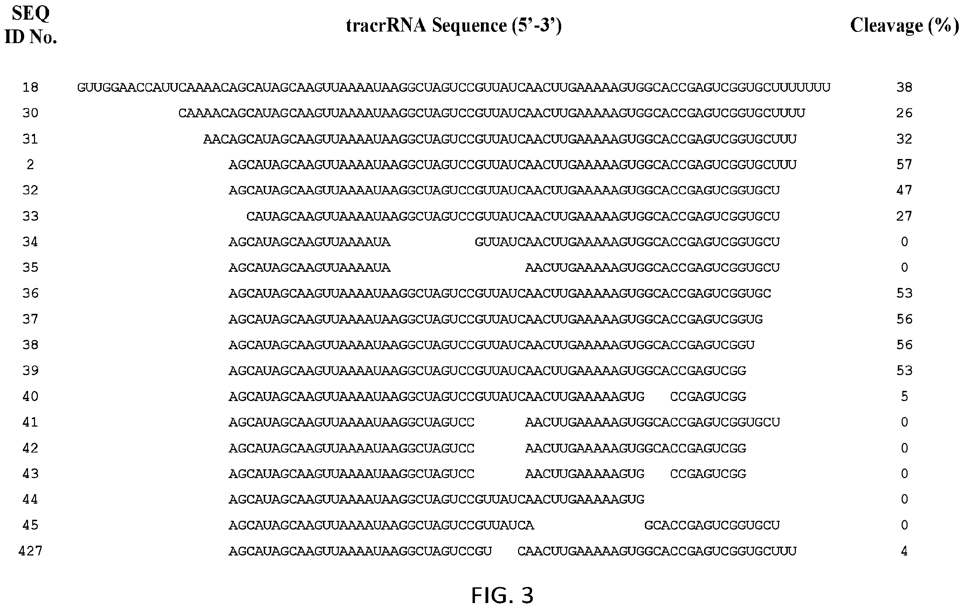

FIG. 3 [reference] shows an alignment of the full-length and truncated tracrRNA species studied in Example 2. Sequences are RNA and are shown 5'-3'. Alignment is based upon the 89 base WT tracrRNA sequence at the top (SEQ ID No. 18). Internal gaps represent sites of internal truncation/deletion. Uppercase letters represent RNA.

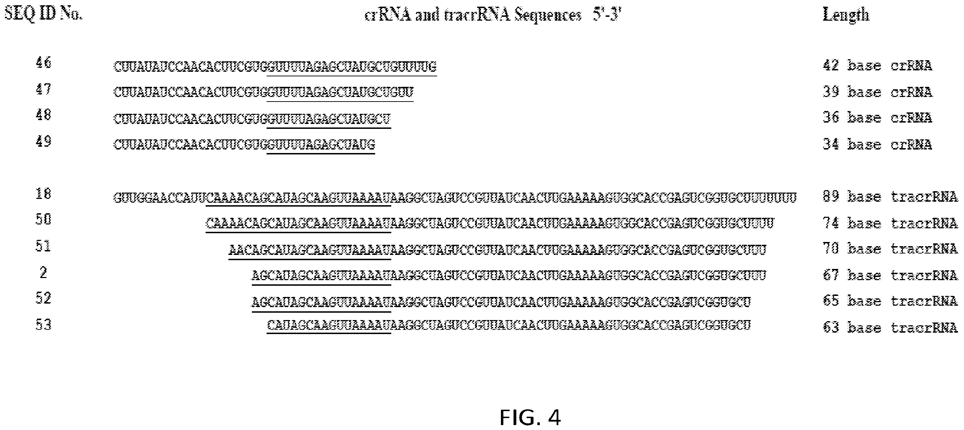

FIG. 4 [reference] shows an alignment of the full-length and truncated crRNA and tracrRNA species studied in Example 3. Alignment is based upon the 42 base WT crRNA (SEQ ID No. 46) and 89 base WT tracrRNA (SEQ ID No. 18) sequences at the top of their respective groupings. The 20 base 5'-domain in the crRNAs is sequence-specific and targets human *HPRT1.* The 3'-domain in underlined and binds to a region towards the 5'-end of the tracrRNA. The 5'-domain in the tracrRNA is underlined that binds the 3'-end of the crRNA. Uppercase letters represent RNA.

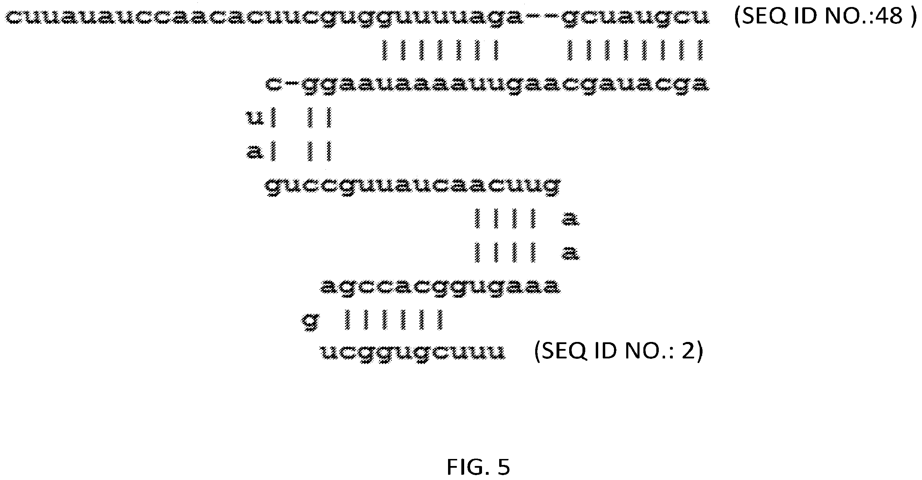

FIG. 5 [reference] is an illustration of a truncated 2-part crRNA:tracrRNA complex with a 36 base crRNA (SEQ ID No. 48) and a 67 base tracrRNA (SEQ ID No. 2). Lowercase letters represent RNA.

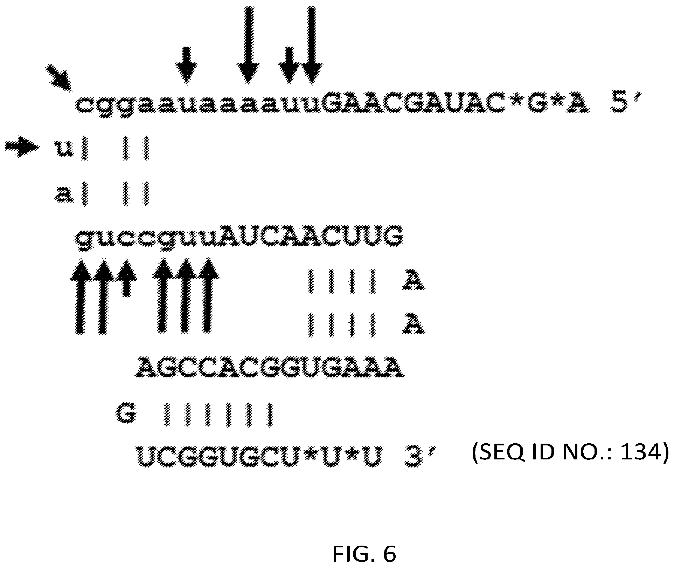

FIG. 6 [invention] is a schematic showing structure of one embodiment of an optimized truncated and chemically-modified tracrRNA (SEQ ID No. 134). Length is 67 bases. RNA is lower case and 2'OMe RNA is uppercase. Phosphorothioate (PS) internucleotide linkages are indicated by "*". Residues which lead to substantial loss of function when converted from RNA to 2'OMe RNA are identified by large arrows and residues which lead to a moderate loss of function when converted from RNA to 2'OMe RNA are identified by small arrows.

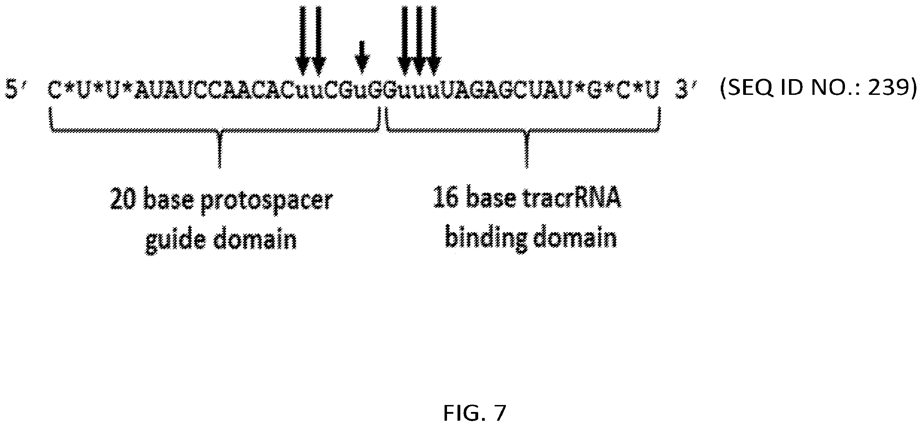

FIG. 7 [invention] is a schematic showing structure of one embodiment of an optimized truncated and chemically-modified crRNA (SEQ ID No. 239). Length is 36 bases. RNA is lower case and 2'OMe RNA is uppercase. Phosphorothioate (PS) internucleotide linkages are indicated by "*". Residues which lead to substantial loss of function when converted from RNA to 2'OMe RNA are identified by large arrows and residues which lead to a moderate loss of function when converted from RNA to 2'OMe RNA are identified by small arrows. The 5'-end 20 base protospacer target-specific guide domain is indicated, which in this case is sequence specific to the human *HPRT1* gene. The 3'-end 16 base tracrRNA binding domain is indicated.

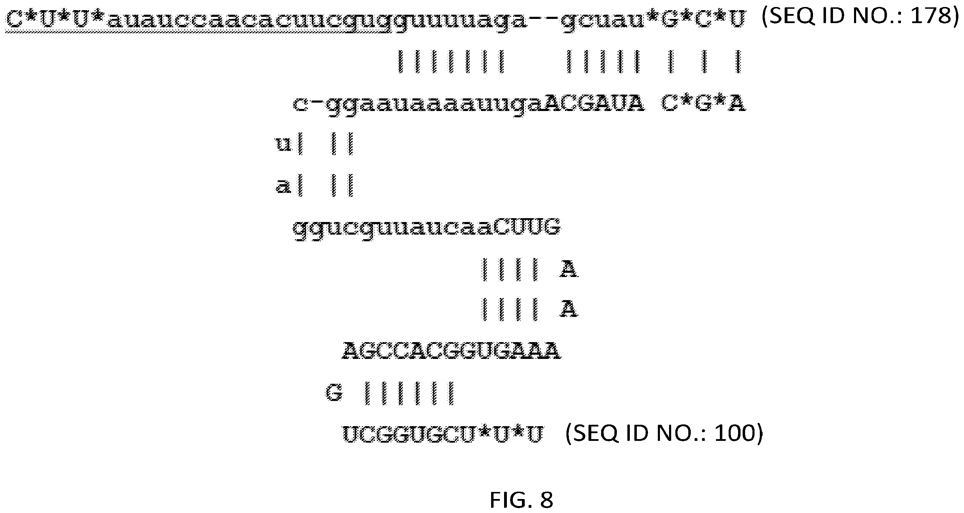

FIG. 8 [invention] is a schematic showing structure of one embodiment of the optimized truncated/modified crRNA:tracrRNA complex as employed in Example 8. The crRNA is positioned at the top with the 5'-protospacer domain 20 base underlined, which in this case is specific for target human *HPRT1* site 38285; the 3'-end is the 16 base tracrRNA binding domain. The tracrRNA is aligned below. RNA is lower case, 2'OMe RNA is uppercase, and "*" indicates a phosphorothioate internucleotide linkage modification. This figure shows the complex formed by crRNA SEQ ID No. 178 and tracrRNA SEQ ID No. 100.

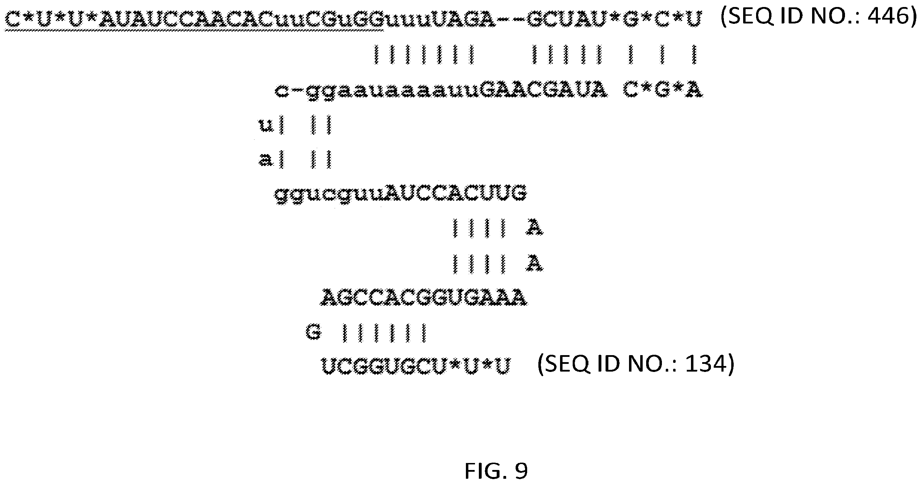

FIG. 9 [invention] is a schematic showing structure of one embodiment of the optimized truncated/modified crRNA:tracrRNA complex that is highly modified. The crRNA is positioned at the top with the 5'-protospacer domain 20 base underlined, which in this case is specific for target human *HPRT1* site 38285; the 3'-end is the 16 base tracrRNA binding domain. The tracrRNA is aligned below. RNA is lower case, 2'OMe RNA is uppercase, and "*" indicates a phosphorothioate internucleotide linkage modification. This figure shows the complex formed by crRNA SEQ ID No. 446 and tracrRNA SEQ ID No. 134.

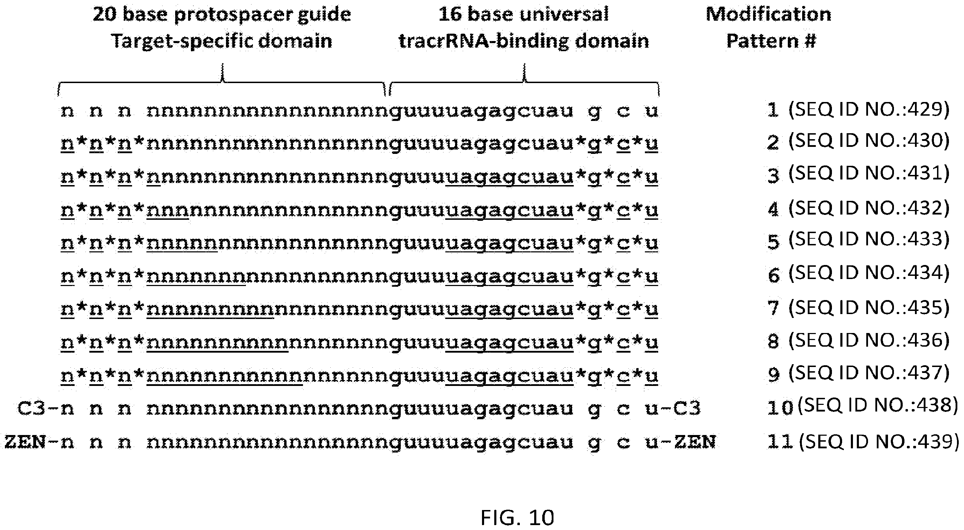

FIG. 10 [invention] is a schematic showing the crRNA modification patterns employed in Example 10. Oligonucleotide sequences (SEQ ID NOS 429-439, respectively, in order of appearance) are shown 5'-3'. Lowercase = RNA; Underlined = 2'-O-methyl RNA; C3 = C3 spacer (propanediol modifier); * = phosphorothioate internucleotide linkage; ZEN = napthyl-azo modifier. The 5'-target specific protospacer domain is indicated. Bases are indicated by "N" in this domain as sequence is different for each target site, although the modification pattern employed remains constant. The 3'-universal tracrRNA binding domain is indicated. Modification patterns are numbered for reference between Table 10 and FIG. 10.

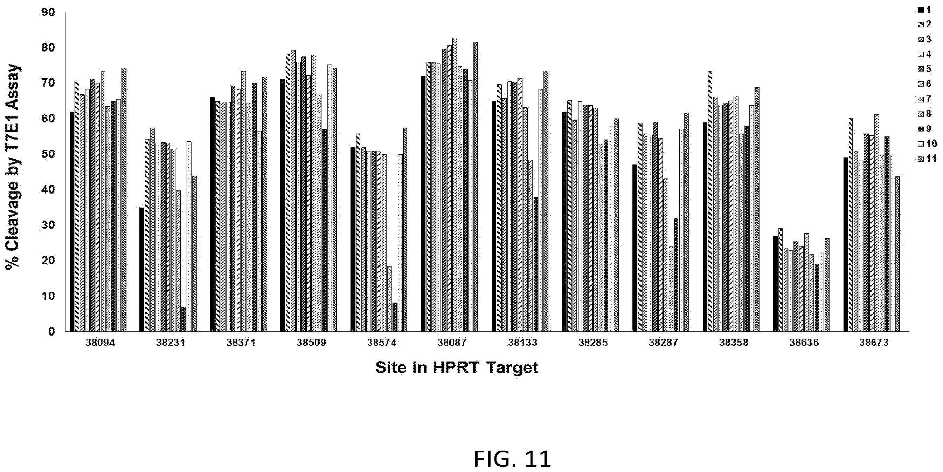

FIG. 11 [invention] is a plot of the data in Table 10 showing the functional gene editing observed using the T7E1 assay in mammalian cells using crRNAs made with 11 different modification patterns tested at 12 different sites in the human *HPRT1* gene. All crRNA variants were paired with an optimized, modified tracrRNA (SEQ ID No. 100).

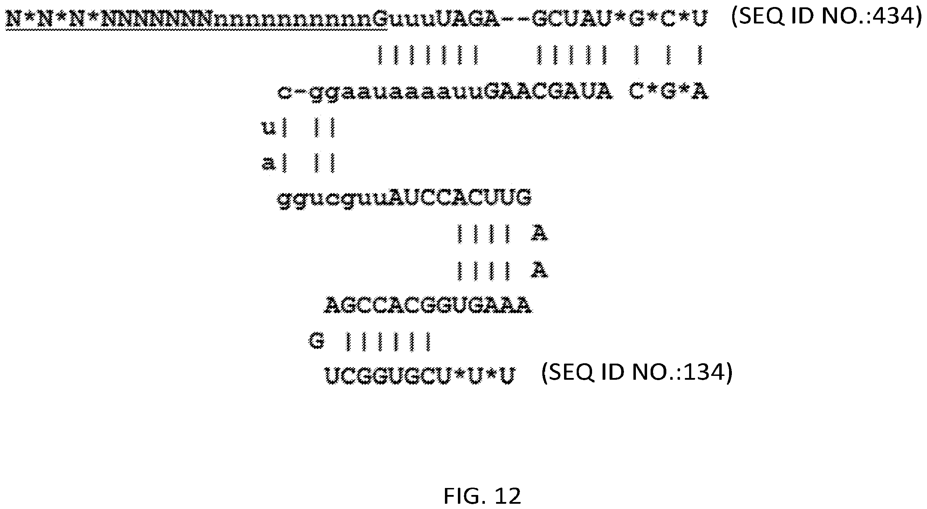

FIG. 12 [invention] is a schematic showing structure of one embodiment of the optimized truncated/modified crRNA:tracrRNA complex that is highly modified using crRNA Mod Pattern 6 that is universal and can be applied in any sequence context. The crRNA (SEQ ID NO: 440) is positioned at the top with the 5'-protospacer domain 20 base underlined (N-bases); the 3'-end is the 16 base tracrRNA binding domain. The tracrRNA is aligned below (SEQ ID No. 134). RNA is lower case, 2'OMe RNA is uppercase, and "*" indicates a phosphorothioate internucleotide linkage modification.

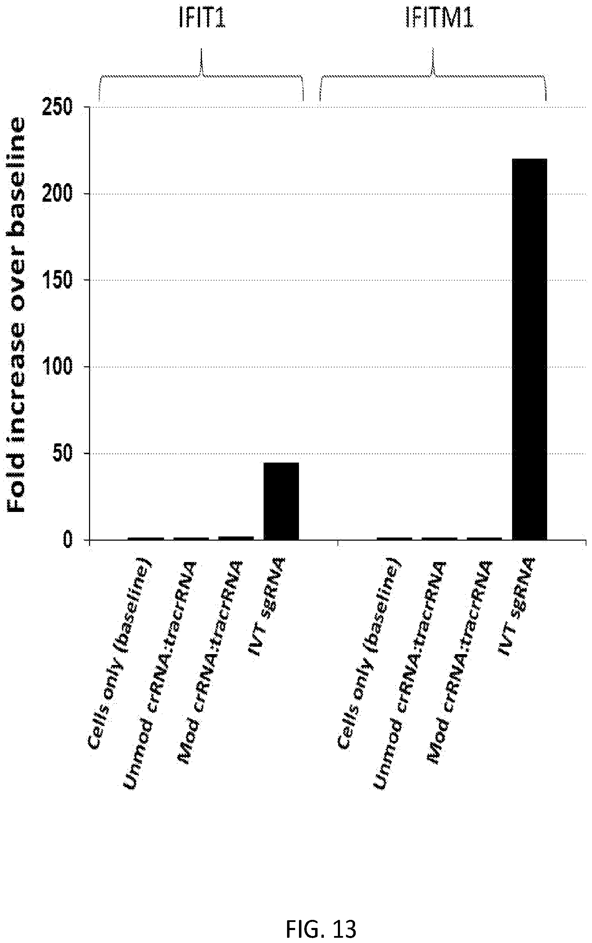

FIG. 13 [invention] shows a plot of RT-qPCR data from HEK-Cas9 cells transfected with different CRISPR gRNAs showing relative expression levels of IFIT1 and IFITM1, 2 genes involved in interferon signaling pathways.

### DETAILED DESCRIPTION OF THE INVENTION

Aspects of this disclosure relate to modified compositions for use in CRISPR systems, and their methods of use.

The term "oligonucleotide," as used herein, refer to polydeoxyribonucleotides (containing 2-deoxy-D-ribose), polyribonucleotides (containing D-ribose), and to any other type of polynucleotide which is an N glycoside of a purine or pyrimidine base (a single nucleotide is also referred to as a "base" or "residue"). There is no intended distinction in length between the terms "nucleic acid", "oligonucleotide" and "polynucleotide", and these terms can be used interchangeably. These terms refer only to the primary structure of the molecule. Thus, these terms include double- and single-stranded DNA, as well as double- and single-stranded RNA. For use in the present disclosure, an oligonucleotide also can comprise nucleotide analogs in which the base, sugar or phosphate backbone is modified as well as non-purine or non-pyrimidine nucleotide analogs. An oligonucleotide may comprise ribonucleotides, deoxyribonucleotides, modified nucleotides (e.g., nucleotides with 2' modifications, synthetic base analogs, etc.) or combinations thereof.

Compositions of the present disclosure include any modification that potentially reduces activation of the innate immune system. Modifications can be placed or substituted at a conventional phosphodiester linkage, at the ribose sugar, or at the nucleobase of RNA. Such compositions could include, for example, a modified nucleotide such as 2'-O-methly-modified RNAs.

More broadly, the term "modified nucleotide" refers to a nucleotide that has one or more modifications to the nucleoside, the nucleobase, pentose ring, or phosphate group. For example, modified nucleotides exclude ribonucleotides containing adenosine monophosphate, guanosine monophosphate, uridine monophosphate, and cytidine monophosphate and deoxyribonucleotides containing deoxyadenosine monophosphate, deoxyguanosine monophosphate, deoxythymidine monophosphate, and deoxycytidine monophosphate. Modifications include those naturally occurring that result from modification by enzymes that modify nucleotides, such as methyltransferases. Modified nucleotides also include synthetic or non-naturally occurring nucleotides. Modifications also include base analogs and universal bases. Synthetic or non-naturally occurring modifications in nucleotides include those with 2' modifications, e.g., 2'-O-alkyl (including 2'-O-methyl), 2'-fluoro, 2'-methoxyethoxy, 2'-allyl, 2'-O-[2-(methylamino)-2-oxoethyl], 4'-thio, bicyclic nucleic acids, 4'-CH2-O-2'-bridge, 4'-(CH2)2-O-2'-bridge, 2'-LNA, and 2'-O-(N-methylcarbamate) or those comprising base analogs. Such modified groups are described, e.g., in Eckstein et al., U.S. Pat. No. 5,672,695 and Matulic-Adamic et al., U.S. Pat. No. 6,248,878.

The use of 2'-O-methyl has been documented in siRNA literature (See Behlke, M.A., Oligonucleotides, 2008. 18(4): p. 305-19) as well as in mRNA delivery (see Sahin, U. et al., Nat Rev Drug Discov, 2014. 13(10): p. 759-80). Sahin et al., describes modifications of mRNA therapeutics that extend beyond 2'-OMe modification and "non-immunogenic" mRNA.

The term "ribonucleotide" encompasses natural and synthetic, unmodified and modified ribonucleotides. Modifications include changes to the sugar moiety, to the base moiety and/or to the linkages between ribonucleotides in the oligonucleotide.

The term "Cas9 protein" encompasses wild-type and mutant forms of Cas9 having biochemical and biological activity when combined with a suitable guide RNA (for example sgRNA or dual crRNA:tracrRNA compositions) to form an active CRISPR-Cas endonuclease system. This includes orthologs and Cas9 variants having different amino acid sequences from the *Streptococcus pyogenese* Cas9 employed as example in the present disclosure.

The term "length-modified," as that term modifies RNA, refers to a shortened or truncated form of a reference RNA lacking nucleotide sequences or an elongated form of a reference RNA including additional nucleotide sequences.

The term "chemically-modified," as that term modifies RNA, refers to a form of a reference RNA containing a chemically-modified nucleotide or a non-nucleotide chemical group covalently linked to the RNA. Chemically-modified RNA, as described herein, generally refers to synthetic RNA prepared using oligonucleotide synthesis procedures wherein modified nucleotides are incorporated during synthesis of an RNA oligonucleotide. However, chemically-modified RNA also includes synthetic RNA oligonucleotides modified with suitable modifying agents post-synthesis.

Applicants have discovered novel crRNA and tracrRNA oligonucleotide compositions that display robust activity in the Clustered Regularly Interspaced Short Palindromic Repeats (CRISPR)-CRISPR associated (Cas) (CRISPR-Cas) endonuclease system. The oligonucleotide compositions include length-modified forms of crRNA and tracrRNA, as well as chemically-modified forms of crRNA and tracrRNA. The length-modified forms of crRNA and tracrRNA enable one to prepare active forms of these RNAs with cost-effective and efficient oligonucleotide synthesis protocols routinely available. The chemically-modified forms of crRNA and tracrRNA provide one with active agents tunable with certain specific properties, such as improved stability in cellular and *in vivo* contexts. The length-modified forms of crRNA and tracrRNA can also include modifications, thereby enabling access to a broad range of compositions having activity in CRISPR-Cas endonuclease system contexts. These oligonucleotide compositions and their properties in the CRISPR-Cas endonuclease system are described below.

### Length-modified forms of crRNA and tracrRNA

FIG.1 depicts a representation of the wild-type *S. pyogenes* crRNA:tracrRNA complex, wherein an exemplary isolated crRNA (SEQ ID No. 46) is paired with an isolated tracrRNA (SEQ ID No. 18). In a first aspect of the instant disclosure, an isolated tracrRNA including a length-modified form of SEQ ID NO.:18 is provided. The isolated tracrRNA displays activity in the CRISPR-Cas endonuclease system. In one respect, the isolated tracrRNA includes a length-modified form of SEQ ID NO.:18 nucleotide having deleted sequence information. In some aspects of the instant disclosure, the length-modified form of SEQ ID NO.:18 includes shortened or truncated forms of SEQ ID NO.:18, wherein SEQ ID NO.:18 can be shortened by 1 to 20 nucleotides at the 5'-end and by 1-10 nucleotides at the 3'-end. Such shortened or truncated forms of SEQ ID NO.:18 retain activity when paired with a functionally competent crRNA in the CRISPR-Cas endonuclease system. Where shortening of the 5'-end of the tracrRNA is performed and extends into sequence that pairs with the 3'-end of the crRNA, improved activity may be obtained using chemical modification that enhance binding affinity in these domains. Where shortening of the 3'-end of the crRNA is performed and extends into sequence that pairs with the 5'-end of the tracrRNA, improved activity may be obtained using chemical modification that enhance binding affinity in these domains. Preferred examples according to the instant disclosure of a length-modified form of SEQ ID NO.:18 having a shortened or truncated form include SEQ ID NOs:2, 30-33 and 36-39. A highly preferred example of a length-modified form of SEQ ID NO.:18 having a shortened or truncated form includes SEQ ID NO:2. For each of the foregoing exemplary length-modified forms of SEQ ID NO.: 18 having a shortened or truncated form, SEQ ID NOs.:2, 30-33 and 36- 39 according to the instant disclosure can consist of chemically non-modified nucleotides.

According to the first embodiment of the instant invention, an isolated tracrRNA comprising a chemically-modified form of one of SEQ ID NOs.:2, 18, 30-33 and 36-39, wherein the isolated tracrRNA displays activity in a Clustered Regularly Interspaced Short Palindromic Repeats (CRISPR)-CRISPR associated (Cas) (CRISPR-Cas) endonuclease system, wherein the chemically-modified form of one of SEQ ID NOs.:2, 18, 30-33 and 36-39 comprises a chemically-modified nucleotide having an end-modifying group selected from a group consisting of a propanediol (C3) spacer, N,N-diethyl-4-(4-nitronaphthalen-1-ylazo)-phenylamine ("ZEN"), and an inverted-dT residue.

Particularly preferred are isolated tracrRNA , wherein the isolated tracrRNA is selected from the group consisting of SEQ ID NOs.: 97, 99, 104, 105, 130, 131, and 132.
The third embodiment of the invention is directed to an isolated crRNA selected from the group consisting of SEQ ID NOs.: 14, 164, 166-169, 171, 174-178, 186, 187, 189-207, 212-214, 221-224, 226-228, 233-249, 252, 256-259, 264-271, 291-374, 376, 377, 379-384, 430-437, and 445-448, wherein the isolated crRNA is active in a Clustered Regularly Interspaced Short Palindromic Repeats (CRISPR)/CRISPR-associated protein endonuclease system.

In a second aspect of the instant disclosure, an isolated crRNA comprising a length-modified form of formula (I) is provided:

5'-X-Z-3' (I),

wherein X represents sequences including a target-specific protospacer domain, and Z represents sequences including a tracrRNA-binding domain.

The target-specific protospacer domain (X domain of formula (I)) typically includes about twenty nucleotides having complementarity to a region of DNA targeted by the CRISPR-Cas endonuclease system. The tracrRNA-binding domain (the Z domain of formula (I)) typically includes about 20 nucleotides in most CRISPR endonuclease systems (in the native *S.py.* version, this domain is 22 nucleotides). The isolated crRNA displays activity in the CRISPR-Cas endonuclease system.

In one respect, the isolated crRNA includes a length-modified form of formula (I) having deleted sequence information. In some aspects of the instant disclosure, the length-modified form of formula (I) includes shortened or truncated forms of formula (I), wherein formula (I) can be shortened by 1-8 nucleotides at the 3'-end of the Z domain. The length-modified form of formula (I) can be shortened at the 5-end of the X-domain to accommodate a target-specific protospacer domain having 17, 18, 19 or 20 nucleotides. Highly preferred examples of such length-modified form of formula (I) include target-specific protospacer domain having 19 or 20 nucleotides. The exemplary length-modified forms of formula (I) having a shortened or truncated form with a target-specific protospacer (X-domain) of 17-20 nucleotides in length and/or lacking 1-8 nucleotides at the 3'-end of the Z-domain can consist of chemically non-modified nucleotides.

Such shortened or truncated forms of formula (I) according to the instant disclosure retain activity when paired with a competent tracrRNA in the CRISPR-Cas endonuclease system. Preferred spects of isolated crRNA of formula (I) having a length-modified form of formula (I) can include chemically non-modified nucleotides and chemically modified nucleotides.

### Chemically-modified forms of crRNA and tracrRNA

In a third aspect of the instant disclosure, an isolated tracrRNA including a chemically-modified nucleotide or a non-nucleotide chemical modifier is provided. The isolated tracrRNA displays activity in the CRISPR-Cas endonuclease system. In one respect, the isolated tracrRNA includes a chemically-modified nucleotide having a modification selected from a group consisting of a ribose modification, an end-modifying group, and internucleotide modifying linkages. Exemplary ribose modifications include 2'O-alkyl (e.g., 2'OMe), 2'F, bicyclic nucleic acid, and locked nucleic acid (LNA). Exemplary end-modifying groups include a propanediol (C3) spacer and napthyl-azo modifier (N,N-diethyl-4-(4-nitronaphthalen-1-ylazo)-phenylamine, or "ZEN"), and an inverted-dT residue. Exemplary internucleotide modifying linkages include phosphorothioate modification. In one respect, the isolated tracrRNA having a chemically-modified form include SEQ ID NO.:46 and length-modified forms thereof, such as shortened or truncated forms of SEQ ID NO.:46. Preferred shortened or truncated forms of SEQ ID NO.:46 having a chemically-modified nucleotide include SEQ ID NOs:2, 30-33 and 36-39 having a chemically-modified nucleotide. Yet other examples of isolated tracrRNA having a chemically-modified nucleotide with robust activity in the CRISPR-Cas endonuclease system are presented in the Examples.

In a fourth aspect of the instant disclosure, an isolated crRNA including a chemically-modified nucleotide is provided. The isolated crRNA displays activity in the CRISPR-Cas endonuclease system. In one respect, the isolated crRNA includes a chemically-modified nucleotide having a modification selected from a group consisting of a ribose modification, an end-modifying group, and internucleotide modifying linkage. Exemplary ribose modifications include 2'O-alkyl (e.g., 2'OMe), 2'F, bicyclic nucleic acid, and locked nucleic acid (LNA). Exemplary end-modifying groups include a propanediol (C3) spacer and napthyl-azo modifier (N,N-diethyl-4-(4-nitronaphthalen-1-ylazo)-phenylamine, or "ZEN"), and an inverted-dT residue. Exemplary internucleotide modifying linkages include phosphorothioate modification. In one respect, the isolated crRNA having a chemically-modified form include crRNA of formula (I) and length-modified forms thereof.

More in particular, according to the second embodiment of the instant invention, an isolated crRNA comprising a chemically-modified form of formula (I):

5'-X-Z-3' (I)

is provided,
wherein X represents sequences comprising a target-specific protospacer domain comprising from 17 nucleotides to 20 nucleotides and Z represents sequences comprising a tracrRNA-binding domain comprising from 12 nucleotides to 19 nucleotides, wherein the isolated crRNA displays activity in a Clustered Regularly Interspaced Short Palindromic Repeats (CRISPR)-CRISPR associated (Cas) (CRISPR-Cas) endonuclease system, wherein the chemically-modified form of formula (I) comprises a chemically-modified nucleotide having an end-modifying group selected from the group consisting of a propanediol (C3) spacer, N,N-diethyl-4-(4-nitronaphthalen-1-ylazo)-phenylamine ("ZEN"), and an inverted-dT residue.

Particularly preferred is isolated crRNA, wherein the chemically-modified form of formula (I) is selected from SEQ ID NOs.: 163, 179-181, 235-237, 240-243, 256, 257, 268-271, 385-406, 438 and 439.

According to the fourth embodiment of the instant invention, an isolated crRNA selected from the group consisting of SEQ ID NOs.: 14, 164, 166-169, 171, 174-178, 186, 187, 189-207, 212-214, 221-224, 226-228, 233-249, 252, 256-259, 264-271, 291-374, 376, 377, 379-384, 430-437, and 445-448, wherein the isolated crRNA is active in a Clustered Regularly Interspaced Short Palindromic Repeats (CRISPR)/CRISPR-associated protein endonuclease system is provided.

Preferred shortened or truncated forms of crRNA of formula (I) having a chemically-modified nucleotide according to the instant disclosure include SEQ ID NOs.:429-439, with SEQ ID NOs. 438 and 439 being preferred by the instant invention. Highly preferred examples of an isolated crRNA according to the invention having a chemically-modified nucleotide include SEQ ID NOs.:434 and 435. These particular isolated crRNA species represent "universal" crRNAs having a chemically-modified nucleotide showing high activity when combined with a competent tracrRNA in the CRISPR-Cas endonuclease system. Yet other examples of isolated crRNA having a chemically-modified nucleotide with robust activity in the CRISPR-Cas endonuclease system are presented in the Examples.

The foregoing isolated, length-modified and chemically-modified of crRNA and tracrRNA preferably include chemical modifications at the 2'-OH groups (for example, 2'OMe, 2'F, bicyclic nucleic acid, locked nucleic acid, among others) and end-blocking modifications (for example, ZEN, C3 spacer, inverted-dT). Use of both types of general modifications provides isolated, length-modified and chemically-modified of crRNA and tracrRNA with biochemical stability and immunologic tolerance for isolated, length-modified and chemically-modified of crRNA and tracrRNA in biological contexts.

The foregoing isolated, length-modified and chemically-modified of crRNA and tracrRNA can be mixed in different combinations to form active crRNA:tracrRNA as the guide RNA for Cas9. For example, an isolated, length-modified tracrRNA can be combined with an isolated chemically-modified crRNA to form an active crRNA:tracrRNA as the guide RNA for Cas9. The Examples provide illustrations of different combinations of isolated, length-modified and chemically-modified of crRNA and tracrRNA resulting in active crRNA:tracrRNA as the guide RNA for Cas9.

The extent to which one needs particular chemically-modified nucleotides included in one (or both) of the isolated, length-modified and chemically-modified crRNA and tracrRNA depends upon the application for which the resultant active crRNA:tracrRNA serves as the guide RNA for Cas9. In certain biochemical assays of the CRISPR-Cas endonuclease system, particularly where nucleases can be minimized or absent, one may not need extensively chemically-modified crRNA and tracrRNA to effect robust activity of the resultant guide RNA for Cas9 of the CRISPR-Cas endonuclease system. This is attributed to the fact that chemically-modified nucleotides that confer resistance to nucleases are not necessary when nucleases are minimal or absent. In certain biological *(in vivo)* contexts, wherein a mixture including crRNA and tracrRNA is delivered to cells inside carrier vehicles, such as liposome nanoparticles, the isolated length-modified and chemically-modified crRNA and tracrRNA may require less extensive chemically-modified nucleotides than mixtures of crRNA and tracrRNA delivered directly into the blood stream or injected into organ systems as isolated, "naked," RNA mixtures. The extent of chemical modification present in chemically-modified crRNA and tracrRNA can dictate the half-life of the relevant RNA molecules *in vivo* (that is, in the relevant biological context, such as, for example, in the blood stream or inside cells). Accordingly, the modification profile of chemically-modified crRNA and tracrRNA can be used to fine tune the biochemical and biological activity of the resultant crRNA:tracrRNA duplexes as a guide RNA for Cas9 in the CRISPR-Cas endonuclease system.

Although the prior art focuses on the structure of Cas9 as it interacts with a sgRNA, the disclosed design patterns described herein contemplate the aforementioned crRNA:tracrRNA dual RNA systems. A single strand guide RNA offers several benefits, such as simplicity of a therapeutic design. However, standard solid phase phosphoramidite RNA synthesis shows diminishing yields for oligonucleotides as length increases and this problem becomes more apparent as length exceeds 60-70 bases. This precludes robust, cost-effective synthesis of some tracrRNAs as well as the chimeric sgRNA, especially at larger scales needed for some commercial or therapeutic applications. For this reason, the disclosure contemplates aspects of not only sgRNA, but also alternate dual crRNA:tracrRNA as the guide RNA for Cas9. However, an isolated guide RNA having robust activity when combined with Cas9 in the CRISPR-Cas endonuclease system can be engineered by linkage or synthesis of appropriate crRNA and tracrRNA as an artificial, unimolecular sgRNA based upon the isolated, length-modified and chemically-modified forms of crRNA and tracrRNA provided herein. Long single guides of this type may be obtained by direct synthesis or by post-synthetic chemical conjugation of shorter strands.

The design of length-modified and chemically-modified tracrRNA compositions addresses the potential synthetic issues associated with tracrRNA oligonucleotides that are >80 nucleotides in length. The coupling efficiency of 2'-OMe-modified RNA monomers (effectively containing a protecting group on the 2'-OH) is greater than RNA monomer coupling. Incorporating 2'-OMe modified RNAs provides some advantages. First, it allows for longer oligonucleotides to be synthesized as either full 2'-OMe or RNA/2'-OMe mixed oligonucleotides. Secondly, the methods and compositions of the disclosure lead to synthesis and transfection of crRNA:tracrRNA that can evade detection by the immune system. It is well known that exogenous, unmodified RNAs trigger an innate immune response in mammalian cells as well as whole animals. Using 2'OMe-modified oligonucleotides can confer RNA stability to nucleases (a third advantage) as well as reduce cell death and toxicity associated with immunogenic triggers. These advantages are not unique to 2'-OMe modification, per se, as the other disclosed modified nucleotides having different chemical moieties (for example, 2'F, other 2'O-alkyls, LNA, and other bicyclic nucleotides) can offer similar benefits and advantages in terms of conferring resistance to nucleases.

In another embodiment of the instant invention, the tracrRNA portion according to the instant invention complementary to the crRNA according to the instant invention contains at least one modified nucleotide, and in a further embodiment the tracrRNA portion according to the invention complementary to the crRNA according to the invention is comprised of more than 10% modified residues, and in a further embodiment the tracrRNA portion according to the invention not complementary to the crRNA according to the invention is comprised of more than 50% modified residues, and in a further embodiment the tracrRNA portion according to the invention not complementary to the crRNA according to the invention is comprised of more than 90% modified residues.

In another aspect, the crRNA portion is unmodified and the tracrRNA portion is comprised of at least one modified nucleotide. In a further aspect the crRNA portion is unmodified and the tracrRNA portion is comprised of more than 10% modified bases.

In another aspect of the instant disclosure, an isolated crRNA of formula (I) is designed with modifications that are empirically determined. As depicted in FIGs. 7 and 10, the 12 nucleotides at the 3'-end of the Z domain (the tracrRNA-binding domain) and the 10-12 nucleotides at the 5'-end of the X domain (within the protospacer domain) represent universal nucleotides amenable to substitution with chemically-modified nucleotides, wherein the resultant RNAs retain robust activity in the CRISPR-Cas endonuclease system. Yet other nucleotides within the 5'-end of the Z domain (the tracrRNA-binding domain) are intolerant to substitution with chemically-modified nucleotides (FIG. 7). Yet the ability of other sites within an isolated crRNA of formula (I) to accept chemically-modified nucleotides and retain activity in the CRISPR-Cas endonuclease system is largely determined empirically. The tracrRNA binding domain (Z domain) of the crRNA is constant (i.e., sequence does not change as target site varies), so the modifications patterns described herein are universal to all crRNAs regardless of target site and can be broadly applied. The protospacer (X domain) of the crRNA varies with target, and the tolerance of some of the base positions within this domain to chemical modification vary with sequence context and, if maximal chemical modification of a site is desired, may benefit from empiric optimization. However, some of the residues within the target-specific protospacer (X) domain can be modified without consideration to sequence context. The 10-12 residues at the 5'-end of this domain can be substituted with 2'-modified residues with the expectation that full activity of the modified crRNA will be maintained. The remaining 8-10 bases towards the 3'-end of the protospacer (X) domain may tolerate modification or may not, depending on sequence context. One sequence context where 17 out of the 20 bases of the protospacer (X) domain can be modified while maintaining full activity are shown in FIG. 7. Sites were modification compromised activity are indicated.

The applications of Cas9-based tools are many and varied. They include, but are not limited to: plant gene editing, yeast gene editing, rapid generation of knockout/knockin animal lines, generating an animal model of disease state, correcting a disease state, inserting a reporter gene, and whole genome functional screening.

The utility of the present disclosure is further expanded by including mutant versions of Cas enzymes, such as a D10A and H840a double mutant of Cas9 as a fusion protein with transcriptional activators (CRISPRa) and repressors (CRISPRi) (see Xu, T., et al., Appl Environ Microbiol, 2014. 80(5): p. 1544-52). The Cas9-sgRNA complex also can be used to target single-stranded mRNA as well (see O'Connell, M.R., et al., Nature, 516:263, 2014). In the same way as targeting dsDNA, crRNA:tracrRNA can be used with a PAMmer DNA oligonucleotide to direct Cas9 cleavage to the target mRNA or use it in the mRNA capture assay described by O'Connell.

By utilizing an approach to deliver synthetic RNA oligonucleotides for CRISPR/Cas9 applications, it is possible to 1) use mass spectroscopy to confirm discrete RNA sequences, 2) selectively insert 2'-OMe modified RNAs in well-tolerated locations to confer stability and avoid immunogenicity yet retain functional efficacy, 3) specifically control the amount of RNA that is introduced into cells for a controlled transient effect, and 4) eliminate concern over introducing dsDNA that would be endogenously transcribed to RNA but could also become substrate in either homology-directed repair pathway or in non-homologous end joining resulting in an integration event. These integration events can lead to long term undesired expression of crRNA or tracrRNA elements. Further, integration can disrupt other genes in a random and unpredictable fashion, changing the genetic material of the cell in undesired and potentially deleterious ways. The present disclosure is therefore desirable as a means to introduce transient expression of elements of the CRISPR pathway in cells in a way which is transient and leaves no lasting evidence or change in the genome outside of whatever alteration is intended as directed by the crRNA guide.

### CRISPR-Cas endonuclease systems

A competent CRISPR-Cas endonuclease system includes a ribonucleoprotein (RNP) complex formed with isolated Cas9 protein and isolated guide RNA selected from one of a dual crRNA:tracrRNA combination and a chimeric sgRNA. In some aspects, isolated length-modified and/or chemically-modified forms of crRNA and tracrRNA are combined with purified Cas9 protein (for example, SEQ ID NOs.:407-410), an isolated mRNA encoding Cas9 protein (for example, SEQ ID NO.:413), or a gene encoding Cas9 protein (for example, SEQ ID NOs.: 411 and 412) in an expression vector. In certain assays, isolated length-modified and/or chemically-modified forms of crRNA and tracrRNA can be introduced into cell lines that stably express Cas9 protein from an endogenous expression cassette encoding the Cas9 gene. In other assays, a mixture of length-modified and/or chemically-modified forms of crRNA and tracrRNA in combination with either Cas9 mRNA or Cas9 protein can be introduced into cells.

### EXAMPLE 1

This example illustrates functioning of chemically modified and truncated guide RNAs in an *in vitro* Cas9 DNA cleavage assay.

CrRNA and tracrRNA oligonucleotides were synthesized having various chemical modifications and truncations relative to the WT sequences as indicated (Table 1).

**Table 1: In vitro biochemical studies of cleavage of HPRT1 target DNA by Cas9 endonuclease with various crRNA and tracrRNA pairs.**

| **cr/tracr RNA pair** | **SEQ ID No.** | **crRNA Sequence** | **Length** | **Cleavage** |
|---|---|---|---|---|
| | | **tracrRNA Sequence** | | |
| 1A | 1 | uuauauccaacacuucgugguuuuagagcuaugcu | 35 | +++ |
| | 2 | | 67 | |
| 1B | 1 | uuauauccaacacuucgugguuuuagagcuaugcu | 35 | - |
| | 3 | | 89 | |
| 1C | 1 | uuauauccaacacuucgugguuuuagagcuaugcu | 35 | - |
| | 4 | | 67 | |
| 2A | 5 | uuauauccaacacuucgugguuuuagagcuaugcuguuuug | 41 | +++ |
| | 2 | | 67 | |
| 2B | 5 | uuauauccaacacuucgugguuuuagagcuaugcuguuuug | 41 | - |
| | 6 | | 89 | |
| 2C | 5 | uuauauccaacacuucgugguuuuagagcuaugcuguuuug | 41 | - |
| | 4 | | 67 | |
| 3A | 7 | uuauauccaacacuucgugguuuuagagcuaugcuguuuug | 41 | - |
| | 2 | | 67 | |
| 3B | 7 | uuauauccaacacuucgugguuuuagagcuaugcuguuuug | 41 | - |
| | 6 | | 89 | |
| 3C | 7 | uuauauccaacacuucgugguuuuagagcuaugcuguuuug | 41 | - |
| | 4 | | 67 | |
| 4A | 8 | uuauauccaacacuucgugguuuuagagcuaugcuguuuug | 41 | - |
| | 2 | | 67 | |
| 4B | 8 | uuauauccaacacuucgugguuuuagagcuaugcuguuuug | 41 | - |
| | 6 | | 89 | |
| 4C | 8 | uuauauccaacacuucgugguuuuagagcuaugcuguuuug | 41 | - |
| | 4 | | 67 | |
| 5A | 9 | uuauauccaacacuucgugguuuuagagcuaugcu | 35 | - |
| | 2 | | 67 | |
| 5B | 9 | uuauauccaacacuucgugguuuuagagcuaugcu | 35 | - |
| | 6 | | 89 | |
| 5C | 9 | uuauauccaacacuucgugguuuuagagcuaugcu | 35 | - |
| | 4 | | 67 | |
| 6A | 10 | uuauauccaacacuucgugguuuuagagcuaugcu | 35 | - |
| | 2 | | 67 | |
| 6B | 10 | uuauauccaacacuucgugguuuuagagcuaugcu | 35 | - |
| | 6 | | 89 | |
| 6C | 10 | uuauauccaacacuucgugguuuuagagcuaugcu | 35 | - |
| | 4 | | 67 | |
| 1G | 1 | uuauauccaacacuucgugguuuuagagcuaugcu | 35 | +++ |
| | 11 | | 67 | |
| 1K | 1 | uuauauccaacacuucgugguuuuagagcuaugcu | 35 | ++ |
| | 12 | | 67 | |
| 1L | 1 | uuauauccaacacuucgugguuuuagagcuaugcu | 35 | +++ |
| | 13 | | 67 | |
| 14A | 14 | uuauauccaacacuucgugguuuuagagcuaugcu | 35 | +++ |
| | 2 | | 67 | |
| 14G | 14 | uuauauccaacacuucgugguuuuagagcuaugcu | 35 | +++ |
| | 11 | | 67 | |
| 14K | 14 | uuauauccaacacuucgugguuuuagagcuaugcu | 35 | +++ |
| | 12 | | 67 | |
| 14L | 14 | uuauauccaacacuucgugguuuuagagcuaugcu | 35 | +++ |
| | 13 | | 67 | |
| 15A | 15 | uuauauccaacacuucgugguuuuagagcuaugcu | 35 | +++ |
| | 2 | | 67 | |
| 15G | 15 | uuauauccaacacuucgugguuuuagagcuaugcu | 35 | +++ |
| | 11 | | 67 | |
| 15K | 15 | uuauauccaacacuucgugguuuuagagcuaugcu | 35 | +++ |
| | 12 | | 67 | |
| 15L | 15 | uuauauccaacacuucgugguuuuagagcuaugcu | 35 | +++ |
| | 13 | | 67 | |
| 16A | 16 | uuauauccaacacuucgugguuuuagagcuaugcu | 35 | +++ |
| | 2 | | 67 | |
| 16G | 16 | uuauauccaacacuucgugguuuuagagcuaugcu | 35 | +++ |
| | 11 | | 67 | |
| 16K | 16 | uuauauccaacacuucgugguuuuagagcuaugcu | 35 | +++ |
| | 12 | | 67 | |
| 16L | 16 | uuauauccaacacuucgugguuuuagagcuaugcu | 35 | +++ |
| | 13 | | 67 | |
| 1H | 1 | uuauauccaacacuucgugguuuuagagcuaugcu | 35 | +++ |
| | 17 | | 67 | |
| 2D | 5 | uuauauccaacacuucgugguuuuagagcuaugcuguuuug | 41 | +++ |
| | 18 | | 89 | |
| 2E | 5 | uuauauccaacacuucgugguuuuagagcuaugcuguuuug | 41 | +++ |
| | 19 | | 89 | |
| 2F | 5 | uuauauccaacacuucgugguuuuagagcuaugcuguuuug | 41 | +++ |
| | 20 | | 89 | |
| 21 | 5 | uuauauccaacacuucgugguuuuagagcuaugcuguuuug | 41 | ++ |
| | 21 | | 89 | |
| 7A | 22 | uuauauccaacacuucgugguuuuagagcuaugcu | 35 | - |
| | 2 | | 67 | |
| 9A | 23 | uuauauccaacacuucgugguuuuagagcuaugcu | 35 | +++ |
| | 2 | | 67 | |
| 10A | 24 | *uuauauccaacacuucgugguuuuagagcuaugcu* | 35 | +++ |
| | 2 | | 67 | |
| 3D | 7 | uuauauccaacacuucgugguuuuagagcuaugcuguuuug | 41 | - |
| | 18 | | 89 | |
| 4D | 8 | uuauauccaacacuucgugguuuuagagcuaugcuguuuug | 41 | - |
| | 18 | | 89 | |
| 8D | 25 | uuauauccaacacuucgugguuuuagagcuaugcuguuuug | 41 | - |
| | 18 | | 89 | |
| 13D | 26 | uuauauccaacacuucgugguuuuagagcuaugcuguuuug | 41 | +++ |
| | 18 | | 89 | |
| 131 | 26 | uuauauccaacacuucgugguuuuagagcuaugcuguuuug | 41 | +++ |
| | 21 | | 89 | |

| | | | | |
|---|---|---|---|---|
| Oligonucleotide sequences are shown 5'-3'. Lowercase = RNA, Underlined = 2'-O-methyl RNA, Italics = 2'-fluoro RNA. Lengths of the RNA oligonucleotides are indicated (bases). The relative efficiency of cleavage of the DNA target by recombinant Cas9 with each of the crRNA:tracrRNA pairs as visualized by agarose gel electrophoresis is indicated with "+++" indicating complete cleavage, "++" and "+" indicating intermediate levels of cleavage, and "-" indicating no cleavage. | | | | |

The crRNAs contained a 19 base protospacer guide sequence matching a site in the human *HPRT1* gene adjacent to a suitable 'NGG" PAM site. A 938 base pair region from the human *HPRT1* gene was cloned into the pCR-Blunt vector (Life Technologies). The plasmid was linearized by digestion with the restriction endonuclease XmaI (New England BioLabs) prior to use in the Cas9 cleavage assay. Sequence of the *HPRT1* target fragment is shown below. The target PAM site is indicated in bold font and the protospacer guide sequence binding site is underlined.
HPRT1 target sequence. SEQ ID No. 27.

The crRNA and tracrRNA pairs were tested for the ability to direct degradation of a linearized plasmid DNA containing a cloned fragment of the human *HPRT1* gene by recombinant Spy Cas9 (New England BioLabs). The crRNA:tracrRNA were annealed in Duplex Buffer (30 mM HEPES pH 7.5, 100 mM potassium acetate) at 150 nM concentration. Spy Cas9 (15 nM) was preincubated with the crRNA:tracrRNA for 10 min at 37°C at a 1:1 molar ratio. Subsequently, 3 nM of the linearized target plasmid was added and incubated at 37°C for 1 hour. The reaction products were separated on a 1% agarose gel at 125 V for 1 hour. Bands were visualized by GelRed (Biotium) post-staining according to the manufacturer's protocol. The gel was imaged on a UV-transilluminator and results are summarized in Table 1 above.

Native wild-type (WT) CRISPR RNAs have a 19-20 base protospacer domain (guide, which binds to a target nucleic acid) at the 5'-end and a 22 base domain at the 3'-end that binds to the tracrRNA. Thus WT crRNAs are 41-42 bases long. The WT tracrRNA is 89 bases long. We observed that a WT type crRNA:tracrRNA pair supported full cleavage of the target DNA (cr/tracrRNA pair 2D). We additionally observed that a truncated version of the reagents with a 35 base crRNA (19 base protospacer and 16 base tracrRNA binding domain) paired with a 67 base tracrRNA supported full cleavage of the target RNA (cr/tracrRNA pair 1A). The crRNA tracrRNA binding region was truncated 6 bases at the 3'-end (SEQ ID No. 1). The tracrRNA was truncated at both ends (SEQ ID No. 2). Pairwise combinations of the short crRNA with the long tracrRNA showed cleavage as well as the long crRNA with the short tracrRNA (pair 2A). These findings are significant as it permits use of shorter RNA components to direct Cas9 target recognition and cleavage. Shorter RNA oligonucleotides are less expensive and less difficult for chemical synthesis, requiring less purification and giving higher yields than longer RNA oligonucleotides.

Some elements of the native crRNA and tracrRNA (Fig. 1) were deleted to make a functional sgRNA (Fig. 2). However, the amount of duplex nucleic acid binding the crRNA to the tracrRNA in the sgRNA is limited to 11 base pairs, which is typically too short for duplex formation in biological salt conditions. The complex is stable in sgRNA format due to the unimolecular hairpin structure, however the same sequences split into 2 RNAs would be unstable. It was therefore unclear what length of duplex domain was needed to make a minimal yet functional 2-molecule (2-part) CRISPR complex, or if this complex would function to direct target cleavage by Cas9. The present example demonstrates that having as little of 15 bases base paired permits a function 2-part crRNA:tracrRNA complex that is competent to direct Cas9 nuclease activity against a target complementary to the crRNA protospacer domain (SEQ ID Nos. 1 and 2).

Complete chemical modification of the crRNA with 2'OMe RNA was not tolerated (pair 3A and pair 5A). Further, complete 2'OMe modification of the 22 base tracrRNA binding domain of the crRNA did not support target cleavage (pair 4A, pair 6A) and complete 2'OMe modification of the protospacer guide domain did not support cleavage (pair 7A). Complete chemical modification of the tracrRNA with 2'OMe RNA was also not tolerated (pair 1B, 1C and pair 2B, 2C).

Importantly, some highly 2'OMe-modified versions of both CRISPR RNA species did support cleavage. Pair 1K shows high cleavage activity with a tracrRNA having 29 2'OMe residues at the 3'-end (SEQ ID No. 11). Pair 1L shows high cleavage activity with 9 2'OMe residues at the 5'-end and 29 2'OMe residues at the 3'-end (SEQ ID No. 13). Thus 38 out of 67 RNA residues in the short version of the tracrRNA can be converted to 2'OMe RNA (57%) with no loss of activity in an *in vitro* cleavage assay.

Pair 14A demonstrates that 11 bases at the 3'-end of the crRNA (50% of the 22 base tracrRNA binding domain) can be modified with 2'OMe RNA and support target cleavage (SEQ ID No. 14). The modified crRNA retains full activity when paired with the modified tracrRNA (pair 14L, Seq ID Nos. 13 and 14). Modification of 11 bases towards the 5'-end of the crRNA (in the guide, protospacer domain, bases 2-12) supports target cleavage (pair 15A) and this modification is also functional when paired with the modified tracrRNA (pair 15L, SEQ ID Nos. 13 and 15). The 2'OMe modifications towards the 5'-end and 3'-end of the crRNA can be combined (SEQ ID No. 16) such that 22 out of 35 residues are modified (63%) and still support cleavage (pair 16A), even when paired with the modified tracrRNA (pair 16L, SEQ ID Nos. 13 and 16).

The crRNA:tracrRNA pairs mentioned above all employed 2'OMe RNA as the modifier. Additional studies showed that 2'F modification was also tolerated by Cas9 and enabled cleavage of a target DNA. Pair 9A employs a crRNA with 2'F modification at all pyrimidine bases (SEQ ID No. 23) and this design supported complete target cleavage. Likewise complete 2'F modification of the crRNA supported complete target cleavage (pair 10A, SEQ ID No. 24). Combined use of 2'OMe and 2'F modifications may permit complete modification of both the crRNA and tracrRNA. The studies in the present example utilized *in vitro* biochemical analyses. Performance may vary in the context of mammalian gene editing where the sequences have to function in the milieu of a cell nucleus.

### EXAMPLE 2

This example demonstrates functioning of truncated tracrRNAs to direct genome editing by the Spy Cas9 nuclease in mammalian cells.

**Both** functional Cas9 nuclease and the RNA triggers (a single sgRNA or dual crRNA:tracrRNA pair) must be present in the nucleus of mammalian cells for CRISPR genome editing to take place. Transfection of large plasmid vectors expressing Cas9 is inefficient and adds variability to experimental results. In order to accurately assess the impact that changes in length and chemical composition of the crRNA and tracrRNA have in mammalian cells in the absence of other variables, a cell line that stably expresses Spy Cas9 was constructed.

A HEK293 cell line having constitutive expression of SpyCas9 (human codon-optimized) with stable vector integration and selection under G418 was developed as described below. Human optimized Spy Cas9 was ligated into a pcDNA3.1 expression vector (Life Technologies) and transfected into HEK293 cells using Lipofectamine2000 (Life Technologies). The transfected cells were allowed to grow for 2 days before being placed under selective pressure using Neomycin. After 7 days, cells were plated to single colonies using limiting dilution techniques. Monoclonal colonies were screened for Cas9 activity and the clone having highest level of expression was used for future studies. A single copy integration event for Cas9 was determined using droplet digital PCR (ddPCR). Western blot using an anti-Cas9 antibody showed low but constant expression of Cas9 protein. This cell line is henceforth referred to as "HEK-Cas9".

The HEK-Cas9 cell line was employed in subsequent studies. In a reverse transfection format, anti-*HPRT1* crRNA:tracrRNA complexes were mixed with Lipofectamine RNAiMAX (Life Technologies) and transfected into the HEK-Cas9 cells. Transfections were done with 40,000 cells per well in 96 well plate format. RNAs were introduced at a final concentration of 30 nM in 0.75 µl of the lipid reagent. Cells were incubated at 37°C for 48 hours. Genomic DNA was isolated using QuickExtract solution (Epicentre). Genomic DNA was amplified with KAPA HiFi DNA Polymerase (Roche) and primers targeting the HPRT region of interest (HPRT forward primer: AAGAATGTTGTGATAAAAGGTGATGCT (SEQ ID No. 28); HPRT reverse primer: ACACATCCATGGGACTTCTGCCTC (SEQ ID No. 29)). PCR products were melted and re-annealed in NEB buffer 2 (New England BioLabs) to allow for heteroduplex formation followed by digestion with 2 units of T7 endonuclease 1 (T7EI; New England BioLabs) for 1 hour at 37°C. The digested products were visualized on a Fragment Analyzer (Advanced Analytics). Percent cleavage of targeted DNA was calculated as the average molar concentration of the cut products / (average molar concentration of the cut products + molar concentration of the uncut band) × 100.

TracrRNAs (Table 2) were synthesized having deletions at the 5'-end, 3'-end, internal or combinations thereof. The tracrRNAs were complexed with unmodified truncated anti-*HPRT1* crRNA SEQ ID No. 1 (Table 1) which has a 19 base protospacer domain targeting *HPRT1* at the 5'-end and a 16 base tracrRNA binding domain at the 3'-end. The paired crRNA:tracrRNA RNA oligonucleotides were transfected into the HEK-Cas9 cells and processed as described above. Relative gene editing activities were assessed by comparing cleavage rates in the *HPRT1* gene using the T7EI mismatch endonuclease cleavage assay with quantitative measurement of products done using the Fragment Analyzer. A representation of the wild-type S. pyogenes crRNA:tracrRNA complex is shown in FIG. 1 (which pairs crRNA SEQ ID No. 46 with tracrRNA SEQ ID No. 18). The relative location of deletions in the tracrRNA tested in this example are shown in sequence alignment format in FIG. 3.

**Table 2: Effect of length truncations in the tracrRNA on efficiency of gene editing in mammalian cells by Cas9 endonuclease.**

| **SEQ ID No.** | **tracrRNA Sequence 5'-3**' | **Cleavage (%)** | **Length** | **Truncation positions** |
|---|---|---|---|---|
| 18 | | 38 | 89 | - |
| 30 | | 26 | 74 | 5' - 12 bases |
| | | | | 3' - 3 bases |
| 31 | | 32 | 70 | 5' - 15 bases |
| | | | | 3' - 4 bases |
| 2 | | 57 | 67 | 5' - 18 bases |
| | | | | 3' - 4 bases |
| 32 | | 47 | 65 | 5' - 18 bases |
| | | | | 3' - 6 bases |
| 33 | | 27 | 63 | 5' - 20 bases |
| | | | | 3' - 6 bases |
| 34 | | 0 | 55 | 5' - 18 bases |
| | | | | Int - 10 bases |
| | | | | 3' - 6 bases |
| 35 | | 0 | 49 | 5' - 18 bases |
| | | | | Int - 16 bases |
| | | | | 3' - 6 bases |
| 36 | | 53 | 64 | 5' - 18 bases |
| | | | | 3' - 7 bases |
| 37 | | 56 | 63 | 5' - 18 bases |
| | | | | 3' - 8 bases |
| 38 | | 56 | 62 | 5' - 18 bases |
| | | | | 3' - 9 bases |
| 39 | | 53 | 61 | 5' - 18 bases |
| | | | | 3' - 10 bases |
| 40 | | 5 | 58 | 5' - 18 bases |
| | | | | Int - 3 bases |
| | | | | 3' - 10 bases |
| 41 | | 0 | 59 | 5' - 18 bases |
| | | | | Int - 6 bases |
| | | | | 3' - 6 bases |
| 42 | | 0 | 55 | 5' - 18 bases |
| | | | | Int - 6 bases |
| | | | | 3' - 10 bases |
| 43 | | 0 | 52 | 5' - 18 bases |
| | | | | Int - 9 bases |
| | | | | 3' - 10 bases |
| 44 | | 0 | 49 | 5' - 18 bases |
| | | | | 3' - 22 bases |
| 45 | | 0 | 52 | 5' - 18 bases |
| | | | | Int - 13 bases |
| | | | | 3' - 6 bases |
| 427 | | 4 | 64 | 5' - 18 bases |
| | | | | Int - 3 bases |
| | | | | 3' - 4 bases |

| | | | | |
|---|---|---|---|---|
| Oligonucleotide sequences are shown 5'-3'. Lowercase = RNA. Lengths of the RNA oligonucleotides are indicated (bases). The number of RNA residues removed in truncation studies at the 5'-end, 3'-end, and internal (int) are indicated. The relative functional activity of each species is indicated by the % cleavage in a T7EI heteroduplex assay. | | | | |

This example demonstrates that for purposes of gene editing in mammalian cells that the tracrRNA can tolerate significant deletions from both the 5'-end and 3'-end and retain full functionality. Deletion of 18 bases from the 5'-end was well tolerated. Deletion of 20 bases from the 5'-end led to reduced activity, possibly due to lower affinity of binding of the crRNA. It is possible that this reduced length or even shorter might be functional if Tm-enhancing modifications were employed to stabilize the short duplex forming region. Deletion of up to 10 bases from the 3'-end was well tolerated. Additional deletions resulted in loss of activity. Internal deletions that disrupted hairpin elements or spacing between hairpin elements were not functional.

In summary, this example demonstrates that truncation of the tracrRNA from the 89 base length of the wild-type (WT, SEQ ID No. 18) to a 67 base length (SEQ ID No. 2) or to a 62 base length (SEQ ID No. 38), or to a 61 base length (SEQ ID No. 39) retained high functional activity. Use of shortened tracrRNAs of this kind will be less costly and easier to manufacture by chemical methods than the WT 89 base RNA. Some of the truncated species (SEQ ID No. 2, SEQ ID No. 38, and SEQ ID No. 39) showed increased functional activity over the 89 base WT tracrRNA. Therefore in addition to being less costly and easier to manufacture by chemical methods, the shortened tracrRNAs of the present disclosure showed improved activity.

### EXAMPLE 3

Examples 1 and 2 demonstrated that crRNA:tracrRNA complexes shorter than the WT lengths of 42 and 89 bases, respectively, can show higher functional activity in mammalian gene editing. The present example shows further optimization of the lengths of these RNA species.

A series of crRNAs and tracrRNAs (Table 3) were synthesized having different lengths as indicated. Truncations were made at the 3'-end of the crRNA, the 5'-end of the tracrRNA, and/or the 3'-end of the tracrRNA. The crRNAs and tracrRNA were paired as indicated in Table 3. The crRNAs all employed a 20 base protospacer domain targeting *HPRT1* at the 5'-end and variable length 3'-ends (tracrRNA binding domains). An alignment of the crRNA and tracrRNA sequences studied in this example is shown in FIG. 4 to make clear the positions of truncations relative to each functional domain.

The paired crRNA:tracrRNA RNA oligonucleotides were transfected into the HEK-Cas9 cells and processed as described in Example 2. Relative gene editing activities were assessed by comparing cleavage rates in the *HPRT1* gene using the T7EI mismatch endonuclease cleavage assay with quantitative measurement of products done using the Fragment Analyzer. Results are shown in Table 3. The relative location of deletions are shown in sequence alignment format in FIG. 4.

**Table 3: Effect of length truncations in both the crRNA and tracrRNA on efficiency of gene editing in mammalian cells by Cas9 endonuclease.**

| **cr/tracr RNA pair** | **SEQ ID No.** | **crRNA Sequence** | **Length** | **Cleavage %** |
|---|---|---|---|---|
| | | **tracrRNA Sequence** | | |
| 42/89 | 46 | | 42 | 25 |
| | 18 | | 89 | |
| 39/89 | 47 | | 39 | 31 |
| | 18 | | 89 | |
| 36/89 | 48 | cuuauauccaacacuucgugguuuuagagcuaugcu | 36 | 38 |
| | 18 | | 89 | |
| 34/89 | 49 | cuuauauccaacacuucgugguuuuagagcuaug | 34 | 21 |
| | 18 | | 89 | |
| 42/74 | 46 | | 42 | 35 |
| | 50 | | 74 | |
| 39/74 | 47 | | 39 | 34 |
| | 50 | | 74 | |
| 36/74 | 48 | cuuauauccaacacuucgugguuuuagagcuaugcu | 36 | 26 |
| | 50 | | 74 | |
| 34/74 | 49 | cuuauauccaacacuucgugguuuuagagcuaug | 34 | 20 |
| | 50 | | 74 | |
| 42/70 | 46 | | 42 | 55 |
| | 51 | | 70 | |
| 39/70 | 47 | | 39 | 48 |
| | 51 | | 70 | |
| 36/70 | 48 | cuuauauccaacacuucgugguuuuagagcuaugcu | 36 | 32 |
| | 51 | | 70 | |
| 34/70 | 49 | cuuauauccaacacuucgugguuuuagagcuaug | 34 | 9 |
| | 51 | | 70 | |
| 42/67 | 46 | | 42 | 36 |
| | 2 | | 67 | |
| 39/67 | 47 | | 39 | 41 |
| | 2 | | 67 | |
| 36/67 | 48 | cuuauauccaacacuucgugguuuuagagcuaugcu | 36 | 57 |
| | 2 | | 67 | |
| 34/67 | 49 | cuuauauccaacacuucgugguuuuagagcuaug | 34 | 44 |
| | 2 | | 67 | |
| 42/65 | 46 | | 42 | 50 |
| | 52 | | 65 | |
| 39/65 | 47 | | 39 | 46 |
| | 52 | | 65 | |
| 36/65 | 48 | cuuauauccaacacuucgugguuuuagagcuaugcu | 36 | 47 |
| | 52 | | 65 | |
| 34/65 | 49 | cuuauauccaacacuucgugguuuuagagcuaug | 34 | 16 |
| | 52 | | 65 | |
| 42/63 | 46 | | 42 | 6 |
| | 53 | | 63 | |
| 39/63 | 47 | | 39 | 13 |
| | 53 | | 63 | |
| 36/63 | 48 | cuuauauccaacacuucgugguuuuagagcuaugcu | 36 | 28 |
| | 53 | | 63 | |
| 34/63 | 49 | cuuauauccaacacuucgugguuuuagagcuaug | 34 | 33 |
| | 53 | | 63 | |

| | | | | |
|---|---|---|---|---|
| Oligonucleotide sequences are shown 5'-3'. Lowercase = RNA. Lengths of the RNA oligonucleotides are indicated (bases). The relative functional activity of each crRNA:tracrRNA pair is indicated by the % cleavage in a T7EI heteroduplex assay. | | | | |

All of the compounds studied directed CRISPR/Cas editing at the HPRT1 locus in HEK-Cas9 cells. Efficiency varied widely from 6% to 57%. The most effective crRNA+tracrRNA combination was the 36 base crRNA (SEQ ID No. 48) with the 67mer tracrRNA (SEQ ID No. 2). A schematic representation of the truncated, optimized crRNA:tracrRNA complex is shown in FIG. 5. In this case the tracrRNA binding domain of the crRNA was truncated to 16 bases from the WT 22 base sequence (3'-end). This hybridizes to the crRNA binding domain at the 5'-end of the tracrRNA. The tracrRNA was truncated 18 bases at the 5'-end and 4 bases at the 3'-end to product the active 67 base product. For this pair, a blunt end is formed upon hybridization of the 3'-end of the crRNA with the 5'-end of the tracrRNA. Other versions also showed high activity, including the 42 base (WT) crRNA (SEQ ID No. 46) paired with the 70 base tracrRNA (SEQ ID No. 51).

The shortest crRNA tested was 34 bases in length (SEQ ID No. 49) and, in general, showed lower activity than the longer variants. The shorter duplex domain formed between this variant and the tracrRNA has reduced binding affinity (Tm) compared to the 36 base crRNA variant and that 34 base complex was less stable at 37°C. Use of chemical modification that increase binding affinity (Tm), such as 2'OMe RNA, 2'F RNA, or LNA residues, will increase stability of this short duplex domain and will lead to improved activity, permitting use of very short crRNAs of this design. Extensive use of Tm-enhancing modifications will permit use of even shorter tracrRNA binding domains in the crRNA, such as 13 base, or 12 base, or 11 base, or 10 base, or 9 base, or 8 base or shorter, depending on the kind and number of modified residues employed.

### EXAMPLE 4

Examples 1, 2, and 3 demonstrated that crRNA:tracrRNA complexes shorter than the WT lengths of 42 and 89 bases, respectively, can show higher functional activity in mammalian gene editing. In those examples, all truncations were made in the universal domains of the RNAs. The present example tests the effects that truncations have on the target-specific protospacer domain of the guide crRNA.

A series of crRNAs (Table 4) were synthesized having protospacer domain lengths of 20, 19, 18, or 17 bases as indicated. Truncations were made at the 5'-end of the crRNA, using a 16mer universal tracrRNA binding sequence at the 3'-end. The crRNAs were paired with an unmodified 67mer tracrRNA (SEQ ID No. 2). The crRNAs targeted 4 different sites in the same exon of the human *HPRT1* gene.

The paired crRNA:tracrRNA RNA oligonucleotides were transfected into the HEK-Cas9 cells and processed as described in Example 2. Relative gene editing activities were assessed by comparing cleavage rates in the *HPRT1* gene using the T7EI mismatch endonuclease cleavage assay with quantitative measurement of products done using the Fragment Analyzer. Results are shown in Table 4.

**Table 4: Effect of length truncations in the 5'-protospacer domain of the crRNA on efficiency of gene editing in mammalian cells by Cas9 endonuclease.**

| **SEQ ID No.** | **crRNA Sequence 5'-3**' | **Length Protospacer domain** | **Cleavage (%)** | ***HPRT1* site** |
|---|---|---|---|---|
| 48 | | 20 | 64 | 38285 |
| 1 | | 19 | 62 | |
| 54 | | 18 | 57 | |
| 55 | | 17 | 42 | |
| 56 | | 20 | 78 | 38087 |
| 57 | | 19 | 81 | |
| 58 | | 18 | 82 | |
| 59 | | 17 | 82 | |
| 60 | | 20 | 52 | 38358 |
| 61 | | 19 | 30 | |
| 62 | | 18 | 12 | |
| 63 | | 17 | 0 | |
| 64 | | 20 | 70 | 38094 |
| 65 | | 19 | 71 | |
| 66 | | 18 | 52 | |
| 67 | | 17 | 0 | |

| | | | | |
|---|---|---|---|---|
| Oligonucleotide sequences are shown 5'-3'. Lowercase = RNA. The target-specific protospacer domain is underlined and length is indicated (bases). The relative functional activity of each species is indicated by the % cleavage in a T7EI heteroduplex assay. | | | | |

Of the 4 sites studied, one (site 38087) showed high activity for all 4 crRNAs with no changes seen as the protospacer domain was shortened. Site 38285 similar efficacy for the 20 and 19 base protospacer crRNAs (SEQ ID Nos. 48 and 1), a slight decrease for the 18 base version (SEQ ID No. 54), and a large decrease for the 17 base version (SEQ ID No. 55). Site 38094 showed similar efficacy for the 20 and 19 base protospacer crRNAs (SEQ ID Nos. 64 and 65), a moderate decrease for the 18 base version (SEQ ID No. 66), and no activity for the 17 base version (SEQ ID No. 67). Site 38358 showed good activity for the 20 base version (SEQ ID No. 60), lower activity for the 19 base version (SEQ ID No. 61), even lower activity for the 18 base version (SEQ ID No. 62) and no activity for the 17 base version (SEQ ID No. 63).

The use of shortened 17 base protospacer guide domains can lower the occurrence of undesired off-target events compared to the wild-type 20 base domain (Fu et al., Nature Biotechnol., 32:279, 2014). We observe that on-target efficacy varies in a sequence-context specific fashion and that 20 base and 19 base protospacer guide domains are generally effective but that activity begins to decrease when 18 base protospacer domains are used and activity significantly decreases when 17 base protospacer domains are used. Therefore, to maintain desired on-target efficiency, use of 20 and 19 base target-specific protospacer guide domains are employed herein. Significant truncation of the protospacer guide domain in many cases lowers on-target cleavage of a DNA target by the Cas9 endonuclease. Use of chemical modifications that enhance Tm (increase binding affinity of the protospacer target-specific domain of the crRNA to the target DNA sequence) may permit use of shorter sequences such that a 17 base protospacer guide may show similar on-target efficacy as an unmodified 20 base protospacer guide domain.

### EXAMPLE 5

This example demonstrates that truncated crRNA:tracrRNA complex show improved gene editing activity at multiple sites. The prior examples studied efficacy of the truncated RNAs as triggers of CRISPR gene editing in mammalian cells at a single site in the human *HRPT1* gene. Site/sequence specific effects may exist. The present example demonstrates improved performance of the truncated species at 12 sites in an exon of the human *HPRT1* gene.

A series of crRNAs (Table 5) were synthesized having a protospacer domain lengths of 20 bases specific to 12 sites in the human HPRT1 gene with a 16mer universal tracrRNA binding sequence at the 3'-end. The crRNAs were paired with an unmodified 67mer tracrRNA (SEQ ID No. 2). The same 12 sites were studied using WT length crRNA:tracrRNA complexes wherein the crRNA comprised a 20 base protospacer guide paired with a 22mer universal tracrRNA binding sequence at the 3'-end complexed with the WT 89mer tracrRNA (SEQ ID No. 18).

The paired crRNA:tracrRNA RNA oligonucleotides were transfected into the HEK-Cas9 cells and processed as described in Example 2. Relative gene editing activities were assessed by comparing cleavage rates in the *HPRT1* gene using the T7EI mismatch endonuclease cleavage assay with quantitative measurement of products done using the Fragment Analyzer. Results are shown in Table 5.

**Table 5: Effect of length truncations in both the crRNA and tracrRNA on efficiency of gene editing in mammalian cells by Cas9 endonuclease.**

| **cr/tracr RNA pair** | **SEQ ID No.** | **crRNA Sequence** | **Length** | **Cleavage** % |
|---|---|---|---|---|
| | | **tracrRNA Sequence** | | |
| 38094 short | 64 | | 36 | 55 |
| | 2 | | 67 | |
| 38094 long | 68 | | 42 | 31 |
| | 18 | | 89 | |
| 38231 short | 69 | | 36 | 7 |
| | 2 | | 67 | |
| 38231 long | 70 | | 42 | 0 |
| | 18 | | 89 | |
| 38371 short | 71 | | 36 | 57 |
| | 2 | | 67 | |
| 38371 long | 72 | | 42 | 27 |
| | 18 | | 89 | |
| 38509 short | 73 | | 36 | 56 |
| | 2 | | 67 | |
| 38509 long | 74 | | 42 | 7 |
| | 18 | | 89 | |
| 38574 short | 75 | | 36 | 58 |
| | 2 | | 67 | |
| 38574 long | 76 | | 42 | 22 |
| | 18 | | 89 | |
| 38087 short | 56 | | 36 | 60 |
| | 2 | | 67 | |
| 38087 long | 77 | | 42 | 53 |
| | 18 | | 89 | |
| 38133 short | 78 | | 36 | 53 |
| | 2 | | 67 | |
| 38133 long | 79 | | 42 | 37 |
| | 18 | | 89 | |
| 38285 short | 48 | | 36 | 38 |
| | 2 | | 67 | |
| 38285 long | 46 | | 42 | 8 |
| | 18 | | 89 | |
| 38287 short | 80 | | 36 | 48 |
| | 2 | | 67 | |
| 38287 long | 81 | | 42 | 6 |
| | 18 | | 89 | |
| 38358 short | 60 | | 36 | 42 |
| | 2 | | 67 | |
| 38358 long | 82 | | 42 | 8 |
| | 18 | | 89 | |
| 38636 short | 83 | | 36 | 26 |
| | 2 | | 67 | |
| 38636 long | 84 | | 42 | 16 |
| | 18 | | 89 | |
| 38673 short | 85 | | 36 | 45 |
| | 2 | | 67 | |
| 38673 long | 86 | | 42 | 32 |
| | 18 | | 89 | |

| | | | | |
|---|---|---|---|---|
| Oligonucleotide sequences are shown 5'-3'. Lowercase = RNA. Lengths of the RNA oligonucleotides are indicated (bases). The relative functional activity of each crRNA:tracrRNA pair is indicated by the % cleavage in a T7EI heteroduplex assay. | | | | |

The relative efficiency of CRISPR mediated gene editing in the HEK-Cas9 cells varied with sequence context. However, in all cases the shorter optimized RNA guides (36mer crRNA and 67mer tracrRNA) showed higher efficiency than the WT RNAs (42mer crRNA and 89mer tracrRNA). Use of the shortened, optimized guide RNAs improve Cas9 cleavage of targeted DNAs relative to the WT RNAs, improving the gene editing rates.

### EXAMPLE 6

Example 1 described chemical modification patterns that functioned with Cas9 in an *in vitro* biochemical target DNA cleavage assay. This example demonstrates functioning of chemically modified tracrRNAs to direct genome editing by the Spy Cas9 nuclease in mammalian cells. Optimal modification patterns differ between *in vitro* and *in vivo* use.

A series of tracrRNAs (Table 6) were synthesized having a variety of chemical modifications, including: the ribose modifications 2'OMe RNA and LNA; the end-modifying groups propanediol spacer and napthyl-azo modifier (N,N-diethyl-4-(4-nitronaphthalen-1-ylazo)-phenylamine, or "ZEN"); and select internucleotide linkages with phosphorothioate modifications. See: Lennox et al., Molecular Therapy Nucleic Acids 2:e117 2013 for structure of the napthyl-azo modified and use of the napthyl-azo modifier and propanediol modifier for use as end-groups to block exonuclease attack. The tracrRNAs listed in Table 6 were complexed with unmodified truncated anti-*HPRT1* crRNA SEQ ID No. 1 (Table 1) which has a 19 base protospacer domain targeting *HPRT1* at the 5'-end and a 16 base tracrRNA binding domain at the 3'-end. The paired crRNA:tracrRNA RNA oligonucleotides were transfected into the HEK-Cas9 cells and processed as described above. Relative gene editing activities were assessed by comparing cleavage rates in the *HPRT1* gene using the T7EI mismatch endonuclease cleavage assay with quantitative measurement of products done using the Fragment Analyzer.

**Table 6: Optimization of tracrRNA oligonucleotide modification patterns in mammalian cells.**

| **SEQ ID No.** | **tracrRNA Sequence (5'-3')** | **Cleavage (%)** |
|---|---|---|
| 2 | | 65 |
| 4 | | 0 |
| 11 | | 56 |
| 17 | | 12 |
| 12 | | 20 |
| 13 | | 64 |
| 87 | | 61 |
| 88 | | 60 |
| 89 | | 64 |
| 90 | | 60 |
| 91 | | 61 |
| 92 | | 59 |
| 93 | | 57 |
| 94 | | 57 |
| 95 | | 62 |
| 96 | | 62 |
| 97 | | 53 |
| 98 | | 58 |
| 99 | | 20 |
| 100 | | 63 |
| 101 | | 55 |
| 102 | | 39 |
| 103 | | 54 |
| 104 | | 55 |
| 105 | | 23 |
| 106 | | 58 |
| 107 | | 8 |
| 108 | | 0 |
| 109 | | 11 |
| 110 | | 61 |
| 111 | | 61 |
| 112 | | 62 |
| 113 | | 62 |
| 114 | | 61 |
| 115 | | 14 |
| 116 | | 60 |
| 117 | | 60 |
| 118 | | 15 |
| 119 | | 0 |
| 120 | | 7 |
| 121 | | 14 |
| 122 | | 11 |
| 123 | | 0 |
| 124 | | 0 |
| 125 | | 0 |
| 126 | | 64 |
| 127 | | 0 |
| 128 | | 0 |
| 129 | | 57 |
| 130 | | |
| 131 | | 58 |
| 132 | | 59 |
| 133 | | 0 |
| 134 | | 58 |
| 135 | | 19 |
| 136 | | 54 |
| 137 | | 13 |
| 138 | | 0 |
| 139 | | 0 |
| 140 | | 0 |
| 141 | | 0 |
| 142 | | 4 |
| 143 | | 0 |
| 144 | | 52 |
| 145 | | 63 |
| 146 | | 0 |
| 147 | | 62 |
| 148 | | 57 |
| 149 | | 47 |
| 150 | | 61 |
| 151 | | 61 |
| 152 | | 61 |
| 153 | | 61 |
| 154 | | 50 |
| 155 | | 46 |
| 156 | | 59 |
| 157 | | 2 |
| 158 | | 18 |
| 159 | | 50 |
| 160 | | 58 |
| 161 | | 14 |
| 162 | | 8 |

| | | |
|---|---|---|
| Oligonucleotide sequences are shown 5'-3'. Uppercase = DNA; Lowercase = RNA; Underlined = 2'-O-methyl RNA; Italics = 2'-fluoro RNA; +a, +c, +t, +g = LNA; C3 = C3 spacer (propanediol modifier); * = phosphorothioate internucleotide linkage; ZEN - napthyl-azo modifier; Inv-dT = inverted-dT. The relative functional activity of each species is indicated by the % cleavage in a T7EI heteroduplex assay. | | |

Modification is usually necessary for synthetic nucleic acids to function well in an intracellular environment due to the presence of exonucleases and endonucleases that degrade unmodified oligonucleotides. A wide range of modifications have been described that confer nuclease resistance to oligonucleotides. The precise combination and order of modifications employed that works well for a given application can vary with sequence context and the nature of the protein interactions required for biological function. Extensive prior work has been done relating to chemical modification of antisense oligonucleotides (which interact with RNase HI) and siRNAs (which interact with DICER, AGO2, and other proteins). It is expected that chemical modification will improve function of the CRISPR crRNA:tracrRNA complex. However, it is not possible to predict what modifications and/or pattern of modifications will be compatible with functional complexation of the synthetic RNAs with Cas9. The present disclosure defines minimal, moderate, and extensive chemical modification patterns for the tracrRNA that retain high levels of function to direct Cas9 mediated gene editing in mammalian cells.

The results in Table 6 demonstrate that extensive modification is tolerated throughout the 5' and 3' end domains of the tracrRNA. Modification of the internal domains of the tracrRNA showed reduced activity, likely due to altered structure of the folded RNA and/or blocking of protein contact points with the 2'-OH of key RNA residues by the 2'OMe modification. For example, compound SEQ ID No. 100 has 39/67 residues modified with 2'OMe RNA (58%) and retains full activity compared with the unmodified sequence. SEQ ID No. 134 has 46/67 residues modified with 2'OMe RNA (69%) and retains near full activity compared with the unmodified sequence (FIG. 6). SEQ ID No. 134 is a truncated 67mer variant of the tracrRNA. Using SEQ ID No. 134 as a model, modification of 11 sequential residues in the 5'-domain with 2'OMe RNA was tolerated with no loss of activity. Modification of 35 sequential residues in the 3'-domain with 2'OMe RNA was tolerated with not loss of activity. Of note, the two hairpin structures present in the 3'-domain are necessary for function as deletion of either of these features results in loss of activity (Example 2, FIG. 3), yet both of these domains can be completely modified with 2'OMe RNA without compromising function. Note that both SEQ ID Nos. 134 and 100 also have phosphorothioate (PS) modified internucleotide linkages at the 5'- and 3'-ends, which provides additional protection against exonuclease attack.

Specific residues were identified that led to large reductions or complete loss of activity when modified. Using the 67 base tracrRNA (for example, SEQ ID No. 134) as reference, starting from the 5'-end of the sequence substitution of 2'OMe RNA for the natural RNA at residues U12, A15, G26, U27, G30, U31, and U32 led to substantial loss of activity (FIG. 6). Specific residues were also identified that led to smaller yet significant reductions in activity when modified. Using the 67 base tracrRNA (for example, SEQ ID No. 134) as reference, starting from the 5'-end of the sequence substitution of 2'OMe RNA for the natural RNA at residues U13, U18, C23, U24, and C28 led to reduced activity (FIG. 6). This study was performed using 2'OMe RNA. Use of other modifications, such as 2'F, LNA, DNA, etc. at these positions may be better tolerated. The central 21 residue domain of unmodified RNA in SEQ ID No. 134 was modified with 2'-F RNA either completely (SEQ ID No. 141) or partially (SEQ ID Nos. 142 and 143). These variants were not functional. The central 21 residue domain of unmodified RNA in SEQ ID No. 134 was modified with DNA either completely (SEQ ID No. 138) or partially (SEQ ID Nos. 139 and 140). These variants were not functional. Modification of isolated residues in this domain may work, however large continuous blocks of modification in this domain reduce activity of the tracrRNA.

To further investigate which individual residues can be modified using 2'OMe RNA within the central domain of the tracrRNA, a single base modification 2'OMe RNA 'walk' was done (SEQ ID Nos. 144-162). Within this series, modification as residues A14, A19, A20, G21, G22, A25, and C29 showed no loss of activity and are candidates for modification.

Antisense oligonucleotides are often made using complete PS modification, where every internucleotide linkage is phosphorothioate modified. This extensive level of modification is possible because the protein effector molecule RNase HI (which mediates ASO-directed mRNA degradation) tolerates the PS modification in the ASO when forming a functional substrate/enzyme complex. On the other hand, siRNAs do not tolerate full PS modification; extensive PS modification disrupts productive interaction with the effector protein AGO2 (which mediates siRNA-directed mRNA degradation). Extensive PS modification of the tracrRNA in the internal RNA loops disrupts functional interaction with Cas9 (Seq ID No. 133; 29 PS modifications). Limited PS end-modification can be done with no loss of activity (SEQ ID Nos. 98 and 101; 2-3 PP linkages on each end). Less extensive PS modification may be tolerated in the central domain. In particular, RNase cleavage mapping (where incubation of the tracrRNA in a series of serum or cell extract dilutions are used to find the sites that are most sensitive to RNase attack) may be used to identify critical sites where PS modification of only one or a few linkages may stabilize the RNA without disrupting function.

There are applications where the PS modification contributes to chemical toxicity. In this case use of other methods to block exonuclease attack are desirable. Options include end-modifiers such as inverted-dT or abasic groups such as dSpacer, C3 spacer (propanediol), ZEN (napthyl-azo modifier), and others. Placement of such end-modifying groups can eliminate the need for terminal PS internucleotide linkages.

### EXAMPLE 7

Example 1 described chemical modification patterns that functioned with Cas9 in an *in vitro* biochemical target DNA cleavage assay. This example demonstrates functioning of chemically modified crRNAs to direct genome editing by the Spy Cas9 nuclease in mammalian cells. Optimal modification patterns differ between *in vitro* and *in vivo* use.

A series of crRNAs (Table 7) were synthesized having a variety of chemical modifications, including: the ribose modifications 2'OMe RNA, 2'F, and LNA; the end-modifying groups propanediol spacer and napthyl-azo modifier (N,N-diethyl-4-(4-nitronaphthalen-1-ylazo)-phenylamine, or "ZEN"), and an inverted-dT residue; and select internucleotide linkages with phosphorothioate modifications. See: Lennox et al., Molecular Therapy Nucleic Acids 2:e117 2013 for structure of the napthyl-azo modified and use of the napthyl-azo modifier and propanediol modifier for use as end-groups to block exonuclease attack. The crRNAs had either a 19 base protospacer domain targeting *HPRT1* at the 5'-end (SEQ ID Nos. 1, 9 10, 14-16, 22-24, 163-173) or a 20 base protospacer domain targeting the same site (SEQ ID Nos. 48, 174-237) with a 16 base tracrRNA binding domain at the 3'-end. The crRNAs listed in Table 7 were complexed with unmodified truncated (67 base) tracrRNA SEQ ID No. 2 (Table 1) or chemically-modified truncated (67 base) tracrRNA SEQ ID No. 100 (Table 6). The use of two tracrRNAs enables determination if function of chemical modified crRNAs varies if paired with a modified tracrRNA. The paired crRNA:tracrRNA RNA oligonucleotides were transfected into the HEK-Cas9 cells and processed as described previously. Relative gene editing activities were assessed by comparing cleavage rates in the *HPRT1* gene using the T7EI mismatch endonuclease cleavage assay, with quantitative measurement of products done using the Fragment Analyzer.

**Table 7: Optimization of crRNA oligonucleotide modification patterns in mammalian cells.**

| **SEQ ID No.** | **crRNA Sequence (5'-3')** | **Cleavage % tracrRNA SEQ ID No 2** | **Cleavage % tracrRNA SEQ ID No. 100** |
|---|---|---|---|
| 1 | uuauauccaacacuucgugguuuuagagcuaugcu | 63 | 61 |
| 9 | uuauauccaacacuucgugguuuuagagcuaugcu | 1 | 0 |
| 10 | uuauauccaacacuucgugguuuuagagcuaugcu | 0 | 1 |
| 22 | uuauauccaacacuucgugguuuuagagcuaugcu | 1 | 1 |
| 23 | uu**a**u**a**ucc**aa**cacuuc**g**u**gg**uuuu**agag**cu**a**u**g**cu | 5 | ND |
| 24 | *uuauauccaacacuucgugguuuuagagcuaugcu* | 3 | 5 |
| 14 | uuauauccaacacuucgugguuuuagagcuaugcu | 63 | 26 |
| 15 | uuauauccaacacuucgugguuuuagagcuaugcu | 5 | 3 |
| 16 | uuauauccaacacuucgugguuuuagagcuaugcu | 5 | 5 |
| 163 | | 65 | 49 |
| 164 | | 65 | 65 |
| 165 | uuauauccaacacuucgugguuuuagagcuaugcu | 0 | 3 |
| 166 | uuauauccaacacuucgugguuuuagagcuaugcu | 54 | 42 |
| 167 | uuauauccaacacuucgugguuuuagagcuaugcu | 49 | 58 |
| 168 | uuauauccaacacuucgugguuuuagagcuaugcu | 64 | 60 |
| 169 | uuauauccaacacuucgugguuuuagagcuaugcu | 16 | 16 |
| 170 | uuauauccaacacuucgugguuuuagagcuaugcu | 3 | 3 |
| 171 | uuauauccaacacuucgugguuuuagagcuaugcu | 42 | 62 |
| 172 | uuauauccaacacuucgugguuuuagagcuaugcu | 4 | 13 |
| 173 | uuauauccaacacuucgugguuuuagagcuaugcu | 1 | 1 |
| 48 | | 61 | 60 |
| 174 | | 60 | 59 |
| 175 | | 62 | 60 |
| 176 | | 61 | 59 |
| 177 | | 60 | 59 |
| 178 | | 61 | 59 |
| 179 | | 61 | 58 |
| 180 | | 57 | 59 |
| 181 | | 62 | 59 |
| 182 | | 64 | 62 |
| 183 | | 62 | 60 |
| 184 | | 64 | 64 |
| 185 | | 60 | 63 |
| 186 | | 64 | 62 |
| 187 | | 65 | 65 |
| 188 | | 0 | 0 |
| 189 | | 63 | 64 |
| 190 | | 64 | 62 |
| 191 | | 64 | 63 |
| 192 | | 64 | 64 |
| 193 | | 64 | 65 |
| 194 | | 60 | 63 |
| 195 | | 63 | 62 |
| 196 | | 62 | 63 |
| 197 | | 61 | 64 |
| 198 | | 61 | 64 |
| 199 | | 63 | 68 |
| 200 | | 59 | 67 |
| 201 | | 63 | 67 |
| 202 | | 60 | 69 |
| 203 | | 53 | 67 |
| 204 | | 54 | 67 |
| 205 | | 59 | 62 |
| 206 | | 58 | 61 |
| 207 | | 50 | 60 |
| 208 | | 0 | 7 |
| 209 | | 0 | 0 |
| 210 | | 0 | 0 |
| 211 | | 0 | 0 |
| 212 | | 56 | 68 |
| 213 | | 41 | 64 |
| 214 | | 53 | 67 |
| 215 | | 0 | 2 |
| 216 | | 0 | 0 |
| 217 | | 0 | 0 |
| 218 | | 0 | 0 |
| 219 | | 0 | 0 |
| 220 | | 0 | 0 |
| 221 | | 58 | 61 |
| 222 | | 31 | 54 |
| 223 | | 6 | 60 |
| 224 | | 27 | 57 |
| 225 | | 0 | 2 |
| 226 | | 2 | 25 |
| 227 | | 3 | 31 |
| 228 | | 4 | 35 |
| 229 | | 0 | 0 |
| 230 | | 0 | 0 |
| 231 | | 0 | 1 |
| 232 | | 0 | 0 |
| 233 | | 33 | 67 |
| 234 | | 24 | 66 |
| 235 | | 56 | 65 |
| 236 | | 11 | 55 |
| 237 | | 62 | 65 |
| 238 | | 17 | 67 |
| 239 | | 39 | 66 |
| 240 | | 27 | 63 |
| 241 | | 14 | 46 |
| 242 | | 41 | 67 |
| 243 | | 23 | 24 |
| 244 | | ND | 60 |
| 245 | | ND | 65 |
| 246 | | ND | 64 |
| 247 | | ND | 64 |
| 248 | | ND | 63 |
| 249 | | ND | 53 |
| 250 | | 0 | 2 |
| 251 | | 0 | 0 |
| 252 | | 0 | 18 |
| 253 | | 0 | 3 |
| 254 | | 5 | 0 |
| 255 | | 0 | 0 |
| 256 | | 27 | 53 |
| 257 | | 10 | 50 |
| 258 | | 29 | 47 |
| 259 | | 7 | 45 |
| 260 | | 0 | 4 |
| 261 | | 0 | 0 |
| 262 | | 0 | 0 |
| 263 | | 0 | 0 |
| 264 | | 50 | 62 |
| 265 | | 45 | 59 |
| 266 | | 26 | 36 |
| 267 | | 20 | 34 |
| 268 | | 27 | 59 |
| 269 | | 45 | 60 |
| 270 | | 16 | 43 |
| 271 | | 22 | 45 |
| 272 | | 63 | 57 |
| 273 | | 59 | 60 |
| 274 | | 63 | 63 |
| 275 | | 64 | 62 |
| 276 | | 0 | 1 |
| 277 | | 5 | 16 |
| 278 | | 64 | 61 |
| 279 | | 64 | 63 |
| 280 | | 30 | 49 |
| 281 | | 56 | 60 |
| 282 | | 53 | 61 |
| 283 | | 0 | 3 |
| 284 | | 0 | 2 |
| 285 | | 2 | 8 |
| 286 | | 48 | 61 |
| 287 | | 0 | 0 |
| 288 | | 0 | 0 |
| 289 | | 2 | 14 |
| 290 | | 0 | 0 |

| | | | |
|---|---|---|---|
| Oligonucleotide sequences are shown 5'-3'. Uppercase = DNA; Lowercase = RNA; Underlined = 2'-O-methyl RNA; Italics = 2'-fluoro RNA; +a, +c, +t, +g = LNA; C3 = C3 spacer (propanediol modifier); * = phosphorothioate internucleotide linkage; ZEN = napthyl-azo modifier; InvdT = inverted-dT. The relative functional activity of each species is indicated by the % cleavage in a T7EI heteroduplex assay when the indicated crRNA is paired with the indicated tracrRNA. ND = not determined. | | | |

Some kind of chemical modification is usually necessary for synthetic nucleic acids to function well in an intracellular environment due to the presence of exonucleases and endonucleases that degrade unmodified oligonucleotides. A wide range of modifications have been described that confer nuclease resistance to oligonucleotides. The precise combination and order of modifications employed that works well for a given application can vary with sequence context and the nature of the protein interactions required for biological function. Extensive prior work has been done relating to chemical modification of antisense oligonucleotides (which interact with RNase H1) and siRNAs (which interact with DICER, AGO2, and other proteins). It is expected that chemical modification will improve function of the CRISPR crRNA:tracrRNA complex. However, it is not possible to predict what modifications and/or pattern of modifications will be compatible with association of the RNAs with Cas9 in a functional way. The present disclosure defines minimal, moderate, and extensive chemical modification patterns for the crRNA that retain high levels of function to direct Cas9 mediated gene editing in mammalian cells. The survey in Example 7 was performed targeting a single site in the human *HPRT1* gene. Note that modification patterns of the 20 base 5'-end protospacer guide domain of the crRNA that perform well may vary with sequence context. However, it is likely that modification patterns of the 3'-end tracrRNA binding domain that perform well as defined herein will be affected when the sequence of the adjacent protospacer domain changes when different sites are targeted, so the 3'-domain modification patterns shown here will be "universal".

The results in Table 7 demonstrate that extensive modification is tolerated throughout the 5' and 3' ends of the crRNA. Modification of certain select positions within internal domains of the crRNA lead to reduced activity or totally blocks activity, likely due to altered structure of the folded RNA and/or blocking of protein contact points with the 2'-OH of key RNA residues by the 2'OMe modification. For example, compound SEQ ID No. 204 has 21/36 residues modified with 2'OMe RNA (58%) and retains full activity compared with the unmodified sequence. Compound SEQ ID No. 239 has 30/36 residues modified with 2'OMe RNA (83%) and retains full activity compared with the unmodified sequence. Both of these compounds also have 3 phosphorothioate (PS) modified internucleotide linkages at the 5'- and 3'-ends, which provides additional protection against exonuclease attack. In contrast, SEQ ID No. 165 has only 4/36 residues modified with 2'OMe RNA (11%) yet has totally lost activity.

Large blocks of sequence were tolerant to 2'OMe modification at the 5'-end and 3'-end of the crRNA, however modification of certain residues in the central portion of the molecule led to inactivation. To further investigate which individual residues can be modified using 2'OMe RNA within the central domain of the crRNA, a single base modification 2'OMe RNA 'walk' was done (SEQ ID Nos. 272-286). Specific residues (positions within the crRNA) were identified that led to large reductions or complete loss of activity. Using the 36 base crRNA SEQ ID No. 239 as model and numbering from the 5'-end of the sequence, substitution of 2'OMe RNA for the natural RNA of residues U15 and U16 lead to substantial loss of activity and residue U19 led to a moderate loss of activity (FIG. 7). These 3 sites lie within the target-specific protospacer guide domain, so sequence varies with target (residues 15, 16, and 19, FIG. 7). It is possible that in certain sequence contexts that these sites will be tolerant to modification. Within the universal tracrRNA-binding domain (residues 21-36), substitution of 2'OMe RNA for the natural RNA of residues U22, U23, and U24 led to substantial loss of activity. Given that this domain does not change with sequence context, it is likely that these sites will not vary in modification tolerance as target sequence changes. Sequence-specific effects of modification in the 20-base target-specific protospacer guide domain are studies in greater detail in Example 10.

Antisense oligonucleotide are often made with complete PS modification, where every internucleotide linkage is phosphorothioate modified. This extensive level of modification is possible because the protein effector molecule RNase HI tolerates the PS modification in the ASO when forming a functional substrate/enzyme complex. On the other hand, siRNAs do not tolerate full PS modification; extensive PS modification disrupts productive interaction with the effector protein AGO2. Limited PS end modification of the crRNA can be done with no loss of activity (SEQ ID Nos. 177, 178, 239, etc., have 3 PS linkages on each end). End-modification is desirable as this adds additional protection from exonuclease attack. PS modification of select internal sites may also be tolerated and may provide additional protection from endonuclease attack. Using SEQ ID No. 264 as a base modification pattern, internal linkages were PS modified in the tracrRNA-binding domain (SEQ ID No. 265), in the 3'-end of the protospacer guide domain (seed region) (SEQ ID No. 266), or both regions (SEQ ID No. 267). Increasing level of PS modification led to reduced functional activity, with SEQ ID No. 267 having ∼50% the activity of the less modified SEQ ID No. 264 variant. SEQ ID No 267 has 21 out of 35 internucleotide linkages modified and will be stable to nuclease exposure. In cases where exposure to a high nuclease environment is needed (such as direct IV administration for research or therapeutic indications), this highly modified variant may actually show higher activity than the less modified variants, which will be degraded more quickly.

There are experimental settings where the PS modification contributes to chemical toxicity. In this case use of other methods to block exonuclease attack are desirable. The crRNA can have a C3 spacer (propanediol modifier) or a ZEN (napthyl-azo modifier) placed on either or both the 5'-end and 3'-end to block exonuclease attack, obviating the need the PS modification. This strategy can be employed to eliminate the PS-end block modification (See SEQ ID Nos. 179-186). This strategy can be used to reduce PS content of more highly modified crRNA variants. SEQ ID No. 271 has the internal protospacer domain and tracrRNA binding domain PS-modified in the same pattern as SEQ ID No. 267, yet employs only 15 PS internucleotide linkages (instead of 21) and shows improved activity. Therefore combination of non-base end-blocks with internal PS modification may be used to increase nuclease stability while maintaining high activity.

### EXAMPLE 8

The following example demonstrates improved potency of the modified CRISPR crRNAs and tracrRNAs of the present disclosure. Examples 2-7 employed transfection of crRNA:tracrRNA complexes into human HEK-Cas9 cells at 30 nM concentration. Experimental testing had previously shown that this dose represented the upper shoulder of the dose response curve such that using higher doses of RNA did not improve gene editing efficiency but use of lower doses resulted lower gene editing efficiency. Those measurements were done using unmodified RNAs. The present example re-examines the dose response of new optimized chemically modified RNAs of the present disclosure compared with unmodified RNAs and demonstrates that chemical modification (i.e., nuclease stabilization) results in more potent compounds which can be used at lower dose.

Example 5 demonstrated that the truncated guide RNAs performed superior to WT RNAs at 12 sites in the human *HPRT1* gene. Four of these sites (38087, 38231, 38133, and 38285) were chosen for comparison of unmodified vs. modified RNA in the present example. Unmodified crRNAs were paired with the unmodified tracrRNA (SEQ ID No. 2) at a 1:1 molar ratio. Unmodified crRNAs were paired with the modified tracrRNA (SEQ ID No. 100) at a 1:1 molar ratio. Modified crRNAs were paired with the modified tracrRNA (SEQ ID No. 100) at a 1:1 molar ratio. Sequences are shown in Table 8. RNAs were transfected into HEK-Cas9 cells as described previously at 30 nM, 10 nM, and 3 nM concentrations. Cells were incubated for 48 hours at 37°C, then were processed for DNA and studied for evidence of gene editing activity comparing cleavage rates at the *HPRT1* locus in the T7EI mismatch endonuclease assay, with quantitative measurement of products done using the Fragment Analyzer as previously described. Results are shown in Table 8.

**Table 8: Increased potency of modified vs. unmodified crRNA:tracrRNA complexes to direct Cas9-mediated gene editing in mammalian cells.**

| **cr/tracr RNA pair** | **SEQ ID No.** | **crRNA Sequence** | **30 nM Cleavage %** | **10 nM Cleavage %** | **3 nM Cleavage %** |
|---|---|---|---|---|---|
| | | **tracrRNA Sequence** | | | |
| 38087 Un-cr Un-tr | 56 | | 80 | 76 | 35 |
| | 2 | | | | |
| 38087 Un-cr Mod-tr | 56 | | 83 | 76 | 50 |
| | 100 | | | | |
| 38087 Mod-cr | 445 | | 77 | 77 | 54 |
| | 100 | | | | |
| 38231 Un-cr Un-tr | 69 | | 31 | 4 | 0 |
| | 2 | | | | |
| 38231 Un-cr Mod-tr | 69 | | 45 | 14 | 1 |
| | 100 | | | | |
| 38231 Mod-cr Mod-tr | 446 | | 48 | 25 | 4 |
| | 100 | | | | |
| 38133 Un-cr Un-tr | 78 | | 73 | 61 | 27 |
| | 2 | | | | |
| 38133 Un-cr Mod-tr | 78 | | 74 | 61 | 37 |
| | 100 | | | | |
| 38133 Mod-cr Mod-tr | 447 | | 75 | 66 | 55 |
| | 100 | | | | |
| 38285 Un-cr Un-tr | 48 | | 66 | 16 | 2 |
| | 2 | | | | |
| 38285 Un-cr Mod-tr | 48 | | 67 | 16 | 5 |
| | 100 | | | | |
| 38285 Mod-cr Mod-tr | 178 | | 62 | 60 | 26 |
| | 100 | | | | |

| | | | | | |
|---|---|---|---|---|---|
| Oligonucleotide sequences are shown 5'-3'. Lowercase = RNA; Underlined = 2'-O-methyl RNA; * = phosphorothioate internucleotide linkage. Unmodified crRNA = Un-cr. Unmodified tracrRNA = Un-tr. Modified crRNA = Mod-cr. Modified tracrRNA = Mod-tr. The relative functional activity of each species is indicated by the % cleavage in a T7EI heteroduplex assay for each dose studied. | | | | | |

In general, modification of the crRNA and tracrRNA had a small impact on gene editing efficiency when the RNAs were transfected at high dose where the RNAs are present in excess. At lower doses, the modified reagents showed improved potency and, in some cases, markedly improved potency. The degree of improvement varied with site. The very potent site 38087 showed highly efficiency gene editing at the 30 nM and 10 nM doses with all crRNA/tracrRNA variants tested, but at the 3 nM use of the modified tracrRNA (with either of the crRNAs) showed improved activity. A low potency site, such as 38231, showed improved gene editing efficiency even at the highest dose tested (30 nM) using the modified RNAs. Modification of the tracrRNA alone showed benefit, but the greatest benefit was realized when both the crRNA and tracrRNA were modified. FIG. 8 shows a schematic of one effective modified crRNA (SEQ ID No. 178) paired with modified tracrRNA (SEQ ID No. 100), specific for *HPRT1* site 38285. FIG. 9 shows a schematic of a more highly modified pair that is also highly functional, crRNA (SEQ ID No. 239) paired with modified tracrRNA (SEQ ID No. 134), also specific for *HPRT1* site 38285.

The present example employed transfection of the crRNA:tracrRNA complex into HEK-Cas9 cells, where Cas9 protein is constitutively expressed. Therefore transfected RNAs can bind Cas9 protein immediately, minimizing risk of degradation in the cytoplasm by nucleases. It is anticipated that the benefit of chemical modification of the crRNA and/or tracrRNA will be greater in cases where the transfected RNAs must survive exposure to cellular nucleases while Cas9 protein is being made, as occurs when using protocols where Cas9 mRNA or a Cas9 expression vector is co-transfected with the targeting RNAs, such that Cas9 is not already expressed in the cells. The benefits of using highly modified RNAs will be greatest for *in vivo* applications (such as medical therapeutics) where the RNAs may be exposed to both nucleases present in serum (following IV administration) and cellular cytoplasmic nucleases.

### EXAMPLE 9

Examples 2-8 demonstrate activity of truncated and/or chemically-modified CRISPR crRNAs and/or tracrRNAs to trigger Cas9-mediated genome editing in mammalian cells that constitutively express Cas9. The present example demonstrates that the truncated, modified RNA compositions can bind Cas9 protein and this complex can be transfected into human cells and further that transfection of the ribonuclear protein (RNP) complex is sufficient to trigger highly efficient genome editing.

Reagents specific for human *HPRT1* site 38285 were employed in the present example. Unmodified crRNA was paired with unmodified tracrRNA at a 1:1 molar ratio. Unmodified crRNA was paired with modified tracrRNA at a 1:1 molar ratio. Modified crRNA was paired with modified tracrRNA at a 1:1 molar ratio. Sequences are shown in Table 9. RNAs were transfected into unmodified HEK293 cells as described above except that a 1:1 complex of Cas9 protein (Caribou Biosciences) with crRNA:tracrRNA were employed at 10 nM concentration using increased amounts of RNAiMAX lipid transfection reagent (1.2 µL, increased over the 0.75 µL amount used per 100 µL transfection in 96 well format for the 30 nM RNA-alone transfections in HEK-Cas9 cells). Cells were incubated for 48 hours at 37°C, then were processed for DNA and studied for evidence of gene editing activity comparing cleavage rates at the *HPRT1* locus in the T7EI mismatch endonuclease assay, with quantitative measurement of products done using the Fragment Analyzer as previously described. Results are shown in Table 9.

**Table 9: Increased potency of modified vs. unmodified crRNA:tracrRNA complexes to direct Cas9-mediated gene editing in mammalian cells.**

| **cr/tracr RNA pair** | **SEQ ID No.** | **crRNA Sequence** | **10 nM Cleavage %** |
|---|---|---|---|
| | | **tracrRNA Sequence** | |
| 38285 Un-cr Un-tr | 48 | cuuauauccaacacuucgugguuuuagagcuaugcu | 42 |
| | 2 | | |
| 38285 Un-cr Mod-tr | 48 | cuuauauccaacacuucgugguuuuagagcuaugcu | 41 |
| | 100 | | |
| 38285 Mod-cr Mod-tr | 178 | c*u*u*auauccaacacuucgugguuuuagagcuau*g*c*u | 54 |
| | 100 | | |

| | | | |
|---|---|---|---|
| Oligonucleotide sequences are shown 5'-3'. Lowercase = RNA; Underlined = 2'-O-methyl RNA; * = phosphorothioate internucleotide linkage. Unmodified crRNA = Un-cr. Unmodified tracrRNA = Un-tr. Modified crRNA = Mod-cr. Modified tracrRNA = Mod-tr. The relative functional activity of each complex is indicated by the % cleavage in a T7EI heteroduplex assay for each dose studied. | | | |

All 3 CRISPR RNA complexes performed well in the RNP-transfection protocol for mammalian genome editing. The unmodified crRNA + unmodified tracrRNA pair (SEQ ID Nos. 48 and 2) and the unmodified crRNA + modified tracrRNA pair (SEQ ID Nos. 48 and 100) performed 2.5x better at 10 nM dose in the RNP protocol than in the HEK-Cas9 protocol, consistent with the less modified RNAs suffering degradation between transfection and eventual complexation with Cas9 protein in the cytoplasm or nucleus. Thus higher doses are needed for unmodified RNAs and in some settings it is likely that unmodified RNAs will fail to direct any genome editing activity. The modified crRNA + modified tracrRNA (SEQ ID NOs. 178 and 100), on the other hand, worked with high efficiency in both protocols.

The modified, truncated CRISPR RNAs work well with direct Cas9 RNP transfection methods.

### EXAMPLE 10

The chemical modification optimization studies performed in Examples 6 and 7 studied the activity of crRNAs having various modification patterns paired with a tracrRNA having various modification patterns. The tracrRNA is universal and the same sequence is employed at all target sites. It is expected that the performance of various modification patterns for the tracrRNA will be similar between different target sites. The crRNA, however, varies sequence between different target sites. In the optimized version tested in Examples 7 and 8, the 5'-20 bases of the crRNA are target-specific (i.e., the "protospacer domain") and the 3'-16 bases are universal (i.e., "the tracrRNA binding domain"). Like the tracrRNA, it is expected that the performance of various modification patterns in the universal 16 base 3'-domain of the crRNA will be similar at all target sites. However, it is possible that performance of different modification patterns may be influenced by the sequence context present in the 5'-20 base target-specific domain.

It is well established that effective modification patterns for small interfering RNAs (siRNAs) are affected by sequence context (Behlke, Oligonucleotides 18:305-320, 2008). For siRNAs, certain "limited modification" patterns can be applied to all sites, whereas for "heavy modification" it is not possible to predict which patterns will be functional for a given sequence and empiric testing is necessary. The present example studies the effect that sequence context has on the crRNA, testing different modification patterns within the 5'-20 base target-specific domain at different sites.

The modification studies in Examples 6 and 7 employed a single crRNA PAM site in the human *HPRT1* gene. The present study examines 12 sites in the human *HPRT1* gene, including the site previously examined, comparing functional performance of different modification patterns and establishes a single modification pattern that can be employed with good results at all sites. See Example 5 for other studies relating to these 12 sites.

A series of crRNAs (Table 10) were synthesized having a protospacer domain lengths of 20 bases specific to 12 sites in the human *HPRT1* gene with a 16mer universal tracrRNA binding sequence at the 3'-end. The crRNAs were made using a variety of chemical modifications, including: the ribose modifications 2'OMe RNA, the end-modifying groups propanediol spacer and napthyl-azo modifier (N,N-diethyl-4-(4-nitronaphthalen-1-ylazo)-phenylamine, or "ZEN"), an inverted-dT residue; and select internucleotide linkages with phosphorothioate modifications. A schematic representation of the different modification patterns employed is shown in FIG. 10.

The crRNAs were paired with a highly modified 67mer tracrRNA (SEQ ID No. 100). The paired crRNA:tracrRNA RNA oligonucleotides were transfected into the HEK-Cas9 cells and processed as described in Example 2. Relative gene editing activities were assessed by comparing cleavage rates in the *HPRT1* gene using the T7EI mismatch endonuclease cleavage assay with quantitative measurement of products done using the Fragment Analyzer. Results are shown in Table 10 and in FIG. 11.

**Table 10: Optimization of crRNA oligonucleotide modification patterns in mammalian cells across 12 target sites.**

| **HPRT1 Target site** | **SEQ ID No.** | **Mod Pattern** | **crRNA Sequence (5'-3')** | **Cleavage % tracrRNA SEQ ID No. 100** |
|---|---|---|---|---|
| 38094 | 64 | 1 | uccauuucauagucuuuccuguuuuagagcuaugcu | 62 |
| 38231 | 69 | 1 | uuuuguaauuaacagcuugcguuuuagagcuaugcu | 35 |
| 38371 | 71 | 1 | cuuagagaauauuuguagagguuuuagagcuaugcu | 66 |
| 38509 | 73 | 1 | uugacuauaaugaauacuucguuuuagagcuaugcu | 71 |
| 38574 | 75 | 1 | caaaacacgcauaaaaauuuguuuuagagcuaugcu | 52 |
| 38087 | 56 | 1 | aauuauggggauuacuaggaguuuuagagcuaugcu | 72 |
| 38133 | 78 | 1 | ggucacuuuuaacacacccaguuuuagagcuaugcu | 65 |
| 38285 | 48 | 1 | cuuauauccaacacuucgugguuuuagagcuaugcu | 62 |
| 38287 | 80 | 1 | ggcuuauauccaacacuucgguuuuagagcuaugcu | 47 |
| 38358 | 60 | 1 | auuucacauaaaacucuuuuguuuuagagcuaugcu | 59 |
| 38636 | 83 | 1 | ucaaauuaugaggugcuggaguuuuagagcuaugcu | 27 |
| 38673 | 85 | 1 | uacagcuuuaugugacuaauguuuuagagcuaugcu | 49 |
| 38094 | 291 | 2 | | 71 |
| 38231 | 292 | 2 | | 54 |
| 38371 | 293 | 2 | | 65 |
| 38509 | 294 | 2 | | 78 |
| 38574 | 295 | 2 | | 56 |
| 38087 | 296 | 2 | | 76 |
| 38133 | 297 | 2 | | 70 |
| 38285 | 178 | 2 | | 65 |
| 38287 | 298 | 2 | | 59 |
| 38358 | 299 | 2 | | 73 |
| 38636 | 300 | 2 | | 29 |
| 38673 | 301 | 2 | | 60 |
| 38094 | 302 | 3 | | 67 |
| 38231 | 303 | 3 | | 57 |
| 38371 | 304 | 3 | | 65 |
| 38509 | 305 | 3 | | 79 |
| 38574 | 306 | 3 | | 52 |
| 38087 | 307 | 3 | | 76 |
| 38133 | 308 | 3 | | 66 |
| 38285 | 309 | 3 | | 60 |
| 38287 | 310 | 3 | | 56 |
| 38358 | 311 | 3 | | 66 |
| 38636 | 312 | 3 | | 24 |
| 38673 | 313 | 3 | | 51 |
| 38094 | 314 | 4 | | 68 |
| 38231 | 315 | 4 | | 53 |
| 38371 | 316 | 4 | | 65 |
| 38509 | 317 | 4 | | 76 |
| 38574 | 318 | 4 | | 51 |
| 38087 | 319 | 4 | | 76 |
| 38133 | 320 | 4 | | 70 |
| 38285 | 321 | 4 | | 65 |
| 38287 | 322 | 4 | | 56 |
| 38358 | 323 | 4 | | 64 |
| 38636 | 324 | 4 | | 23 |
| 38673 | 325 | 4 | | 48 |
| 38094 | 326 | 5 | | 71 |
| 38231 | 327 | 5 | | 53 |
| 38371 | 328 | 5 | | 69 |
| 38509 | 329 | 5 | | 77 |
| 38574 | 330 | 5 | | 51 |
| 38087 | 331 | 5 | | 80 |
| 38133 | 332 | 5 | | 70 |
| 38285 | 333 | 5 | | 64 |
| 38287 | 334 | 5 | | 59 |
| 38358 | 335 | 5 | | 64 |
| 38636 | 336 | 5 | | 25 |
| 38673 | 337 | 5 | | 56 |
| 38094 | 338 | 6 | | 70 |
| 38231 | 339 | 6 | | 53 |
| 38371 | 340 | 6 | | 68 |
| 38509 | 341 | 6 | | 72 |
| 38574 | 342 | 6 | | 51 |
| 38087 | 343 | 6 | | 81 |
| 38133 | 344 | 6 | | 71 |
| 38285 | 345 | 6 | | 64 |
| 38287 | 346 | 6 | | 55 |
| 38358 | 347 | 6 | | 65 |
| 38636 | 348 | 6 | | 24 |
| 38673 | 349 | 6 | | 55 |
| 38094 | 350 | 7 | | 73 |
| 38231 | 351 | 7 | | 51 |
| 38371 | 352 | 7 | | 73 |
| 38509 | 353 | 7 | | 78 |
| 38574 | 354 | 7 | | 50 |
| 38087 | 355 | 7 | | 83 |
| 38133 | 356 | 7 | | 63 |
| 38285 | 357 | 7 | | 63 |
| 38287 | 358 | 7 | | 43 |
| 38358 | 359 | 7 | | 66 |
| 38636 | 360 | 7 | | 28 |
| 38673 | 361 | 7 | | 61 |
| 38094 | 362 | 8 | | 63 |
| 38231 | 363 | 8 | | 40 |
| 38371 | 364 | 8 | | 64 |
| 38509 | 365 | 8 | | 67 |
| 38574 | 366 | 8 | | 18 |
| 38087 | 367 | 8 | | 75 |
| 38133 | 368 | 8 | | 48 |
| 38285 | 369 | 8 | | 53 |
| 38287 | 370 | 8 | | 24 |
| 38358 | 371 | 8 | | 56 |
| 38636 | 372 | 8 | | 22 |
| 38673 | 373 | 8 | | 50 |
| 38094 | 374 | 9 | | 65 |
| 38231 | 375 | 9 | | 7 |
| 38371 | 376 | 9 | | 70 |
| 38509 | 377 | 9 | | 57 |
| 38574 | 378 | 9 | | 8 |
| 38087 | 379 | 9 | | 74 |
| 38133 | 380 | 9 | | 38 |
| 38285 | 222 | 9 | | 54 |
| 38287 | 381 | 9 | | 32 |
| 38358 | 382 | 9 | | 58 |
| 38636 | 383 | 9 | | 19 |
| 38673 | 384 | 9 | | 55 |
| 38094 | 385 | 10 | | 66 |
| 38231 | 386 | 10 | | 54 |
| 38371 | 387 | 10 | | 57 |
| 38509 | 388 | 10 | | 75 |
| 38574 | 389 | 10 | | 50 |
| 38087 | 390 | 10 | | 71 |
| 38133 | 391 | 10 | | 68 |
| 38285 | 181 | 10 | | 58 |
| 38287 | 392 | 10 | | 57 |
| 38358 | 393 | 10 | | 64 |
| 38636 | 394 | 10 | | 22 |
| 38673 | 395 | 10 | | 50 |
| 38094 | 396 | 11 | | 74 |
| 38231 | 397 | 11 | | 44 |
| 38371 | 398 | 11 | | 72 |
| 38509 | 399 | 11 | | 74 |
| 38574 | 400 | 11 | | 57 |
| 38087 | 401 | 11 | | 82 |
| 38133 | 402 | 11 | | 73 |
| 38285 | 184 | 11 | | 60 |
| 38287 | 403 | 11 | | 62 |
| 38358 | 404 | 11 | | 69 |
| 38636 | 405 | 11 | | 26 |
| 38673 | 406 | 11 | | 44 |

| | | | | |
|---|---|---|---|---|
| Oligonucleotide sequences are shown 5'-3'. Lowercase = RNA; Underlined = 2'-O-methyl RNA; C3 = C3 spacer (propanediol modifier); * = phosphorothioate internucleotide linkage; ZEN = napthyl-azo modifier. The relative functional activity of each species is indicated by the % cleavage in a T7EI heteroduplex assay when the indicated crRNA is paired with the indicated tracrRNA at each of 12 sites in human *HRPT1*. | | | | |

The modified crRNAs employed a fixed modification pattern in the 16-base 3'-end domain which is universal and binds the tracrRNA. Different modification pattern were tested/compared in the 5'-end domain that is target specific (i.e., sequence varies with target site). The test set comprised variants having 0, 3, 4, 6, 8, 10, 12, 13, or 14 contiguous 2'OMe RNA residues starting at the 5'-end and walking towards the 3'-end. The modification patterns avoided positions previously demonstrated to reduce functional performance of the crRNA (Example 7). Use of only non-base modifier end groups (C3 spacer or ZEN) were also tested (without additional modification). When functional activity is compared across all 12 sites in the survey, all sites tested showed full activity when 0-10 RNA residues at the 5'-end were replaced with 2'OMe RNA residues. Only 1/12 sites showed a slight reduction in activity with 12 residues modified, however 3/12 sites showed a reduction in activity when 13 residues were modified and 4/12 sites showed a reduction in activity when 14 residues were modified. The end-modifiers (C3, ZEN) showed full activity at all sites.

The highest level of crRNA modification that showed full activity at all sites tested included Mod Patterns 6 and 7 (FIG. 10). This represents 61% and 67% of the bases in the crRNA modified with 2'OMe RNA, respectively.

| | |
|---|---|
| **n*n*n*nnnnnnnnnnnnnnnnnguuuuagagcuau*g*c*u** | Mod Pattern 6 (SEQ ID NO.:434) |
| **n*n*n*nnnnnnnnnnnnnnnnnguuuuagagcuau*g*c*u** | Mod Pattern 7 (SEQ ID NO.:435) |

The data in the present example also demonstrates that individual sites can be modified at higher levels and retain potency. For example, 8 of the 12 sites studied showed full activity using Mod Pattern 8, which has 72% of the residues modified. Further, example 7 demonstrates that the crRNA targeting site 38285 in HPRT1 (SEQ ID No. 239) has full activity and has 30/36 residues modified (83%, with only 6 unmodified RNA residues remaining). A base modification pattern such as Mod Pattern 6 or Mod Pattern 7 can be used as a starting point for studies to empirically ascertain the extent that a particular sequence can be modified before activity is lost. FIG. 12 shows a schematic where a Mod Pattern 6 crRNA is paired with a highly modified tracrRNA, SEQ ID No. 134.

### EXAMPLE 11

The Examples herein employ the Cas9 endonuclease from *Streptococcus pyogenes.* The native amino acid sequence of *S.py.* Cas9 (SpyCas9) is shown below (SEQ ID No. 407).

The native Cas9 DNA sequence was codon optimized for expression in *E.coli* bacteria and had elements added for mammalian nuclear localization (nuclease localization signals) and aid protein purification (His-tag). The final amino-acid sequence of the recombinant protein is shown (SEQ ID No 408). The DNA sequence employed to express the recombinant protein in *E.coli* is shown (SEQ ID No. 409).

The native Cas9 DNA sequence was codon optimized for expression in human cells and had elements added for antibody recognition (V5 epitope) and mammalian nuclear localization (nuclease localization signals, NLS) added. The final amino-acid sequence is shown (SEQ ID No. 410) and DNA sequence follows (SEQ ID No 411).

The native *S.py* Cas9 DNA sequence codon was optimized for expression in human cells and assembled as a T7 RNA polymerase expression cassette (SEQ ID No. 412). The sequence contains a T7 RNA polymerase promoter, a V5 epitope tag, a nuclear localization signal, the codon optimized Cas9 sequence, a second nuclear localization signal, and the BGH (bovine growth hormone) gene 3'-UTR element with a polyadenylation signal. Sequence of mRNA made from this expression cassette is shown (SEQ ID No. 413).
*S.py.* Cas9 amino acid sequence (SEQ ID No. 407).
*S.py* Cas9 amino acid sequence expressed from DNA codon optimized for expression in E.coli containing 3 NLS sequences and a purification His-tag (SEQ ID No. 408).
*S.py* Cas9 DNA sequence codon optimized for expression in E.coli containing 3 NLS sequences and a purification His-tag (SEQ ID No. 409).
*S.py* Cas9 amino acid sequence expressed from DNA codon optimized for expression in human cells containing a V5 epitope tag and 2 NLS sequences (SEQ ID No. 410).
*S.py* Cas9 DNA sequence codon optimized for expression in human cells containing a V5 epitope tag and 2 NLS sequences (SEQ ID No. 411).
*S.py* Cas9 DNA sequence codon optimized for expression in human cells as a T7 RNA polymerase expression cassette (SEQ ID No. 412). The sequence contains a T7 RNA polymerase promoter, a V5 epitope tag, a nuclear localization signal, the codon optimized Cas9 sequence, a second nuclear localization signal, and the BGH (bovine growth hormone) gene 3'-UTR element with a polyadenylation signal.
*S.py* Cas9 mRNA (SEQ ID No. 413) as made from the expression cassette (SEQ ID No. 412). The sequence contains a V5 epitope tag, a nuclear localization signal, the codon optimized Cas9 sequence, a second nuclear localization signal, and the BGH (bovine growth hormone) gene 3'-UTR element and poly-A tail.

### EXAMPLE 12

The following example demonstrates reduced stimulation of the innate immune system in mammalian cells by the truncated chemically modified crRNA:tracrRNA complexes of the present disclosure when compared with unmodified IVT sgRNAs.

Mammalian cells possess a variety of receptors intended to identify and respond to foreign RNAs as part of anti-viral immunity. This includes receptors such as TLR-3, TLR-7, TLR8, RIG-I, MDA5, OAS, PKR, and others. In broad terms, RNAs that are short or contain chemical modifications present in mammalian cells (such as 2'OMe RNA) evade detection or are less stimulatory than are long, unmodified RNAs. The present example compares the level of stimulation of 2 immune response associated genes (*IFIT1* and *IFITM1)* when mammalian HEK293 cells are transfected with truncated unmodified or truncated modified crRNA:tracrRNA complexes with a commercial IVT sgRNA (Thermo Fisher Scientific, Waltham, MA).

CRISPR guide RNAs specific to human *HPRT1* site 38285 were employed. Sequences are shown in Table 11 below. The unmodified crRNA:tracrRNA complexes (SEQ ID Nos. 48 and 2), the modified crRNA:tracrRNA complexes (SEQ ID Nos. 178 and 100) and the sgRNA (SEQ ID No. 414) were transfected into HEK-Cas9 cells at 30 nM concentration as outlined in Example 2 above. RNA was prepared 24 hours after transfection using the SV96 Total RNA Isolation Kit (Promega, Madison, WI). cDNA was synthesized using 150 ng total RNA with SuperScript™-II Reverse Transcriptase (Invitrogen, Carlsbad, CA) per the manufacturer's instructions using both random hexamer and oligo-dT priming. Transfection experiments were all performed a minimum of three times.

Quantitative real-time PCR was performed using 10 ng cDNA per 10 µL reaction with Immolase™ DNA Polymerase (Bioline, Randolph, MA), 200 nM primers, and 200 nM probe. Cycling conditions employed were: 95°C for 10 minutes followed by 40 cycles of 2-step PCR with 95°C for 15 seconds and 60°C for 1 minute. PCR and fluorescence measurements were done using an ABI Prism™ 7900 Sequence Detector (Applied Biosystems Inc., Foster City, CA). All reactions were performed in triplicate using 2-color multiplexing. Expression data were normalized against an average of two internal control genes. Copy number standards were linearized cloned amplicons for all assays. Unknowns were extrapolated against standards to establish absolute quantitative measurements. Housekeeping internal control normalization assays were *HPRT1* (primers and probe SEQ ID Nos. 415-417) and *SFRS9* (primers and probe SEQ ID Nos. 418-420). Immune activation pathway assays were *IFITM1* (primers and probe SEQ ID Nos. 421-423) and *IFIT1* (primers and probe SEQ ID Nos. 424-426). The results were normalized using non-transfected cells as baseline and are shown in FIG. 13.

**Table 11. Nucleic acid reagents employed in immune activation experiments in Example 12.**

| **SEQ ID No.** | **Reagent** | **Sequence** |
|---|---|---|
| 48 | Unmodified crRNA | cuuauauccaacacuucgugguuuuagagcuaugcu |
| 2 | Unmodified tracrRNA | |
| 178 | Modified crRNA | **c*u*u*auauccaacacuucgugguuuuagagcuau*g*c*u** |
| 100 | Modified tracrRNA | |
| 414 | IVT sgRNA | |
| 415 | Hs HPRT F517 | GACTTTGCTTTCCTTGGTCAG |
| 416 | Hs HPRT R591 | GGCTTATATCCAACACTTCGTGGG |
| I¹ | Hs HPRT P554 | FAM-ATGGTCAAG(ZEN)GTCGCAAGCTTGCTGGT-ZEN |
| 418 | Hs SFRS9 F569 | TGTGCAGAAGGATGGAGT |
| 419 | Hs SFRS9 R712 | CTGGTGCTTCTCTCAGGATA |
| II² | Hs SFRS9 P644 | HEX-TGGAATATG(ZEN)CCCTGCGTAAACTGGA-ZEN |
| 421 | Hs IFITM1 For | CTCTTCTTGAACTGGTGCTGTCTG |
| 422 | Hs IFITM1 Rev | CAGGATGAATCCAATGGTCATGAGG |
| III³ | Hs IFITM1 Probe FAM | FAM-AAGTGCCTG(ZEN)AACATCTGGGCCCTGATT-ZEN |
| 424 | Hs IFIT1 For | CCATTGTCTGGATTTAAGCGG |
| 425 | Hs IFIT1 Rev | GCCACAAAAAATCACAAGCCA |
| IV⁴ | Hs IFIT1 Probe HEX | HEX-TTTCTTTGC(ZEN)TTCCCCTAAGGCAGGCTG-ZEN |

| | | |
|---|---|---|
| ¹ Compound I is an oligonucleotide having the formula SEQ ID NO: 417-(ZEN)-SEQ ID NO: 441. ² Compound II is an oligonucleotide having the formula SEQ ID NO: 420-(ZEN)-SEQ ID NO: 442. ³ Compound III is an oligonucleotide having the formula SEQ ID NO: 423-(ZEN)-SEQ ID NO: 443. ⁴ Compound IV is an oligonucleotide having the formula SEQ ID NO: 426-(ZEN)-SEQ ID NO: 444. Oligonucleotide sequences are shown 5'-3'. Uppercase = DNA; Lowercase = RNA; Underlined = 2'-O-methyl RNA; * = phosphorothioate internucleotide linkage; ppp = triphosphate; ZEN = napthyl-azo modifier, dark quencher; FAM = 6-carboxyfluorescein; HEX = hexachlorofluorescein. | | |

Treatment with the unmodified or chemically modified truncated crRNA:tracrRNA complex did not lead to detectable increases in IFIT1 or IFITM1 expression over baseline. In contrast, treatment with the longer IVT sgRNA led to a 45-fold induction of IFITM1 and a 220-fold induction of IFIT1. Thus, significant stimulation of the innate immune system occurred using the sgRNA that was absent using the short crRNA:tracrRNA complexes of the present disclosure.

### EXAMPLE 13

The following example combines modification patterns identified in Examples 6 and 7 as being particularly efficacious to demonstrate new highly modified crRNA and tracrRNA compositions that perform with high efficiency in mammalian CRISPR genome editing applications.

A series of crRNAs and tracrRNAs (Table 12) were synthesized having chemical modifications as indicated. The crRNAs employed a 20 base protospacer domain targeting the same site in the human *HPRT1* gene (38285) at the 5'- end with a 16 base tracrRNA binding domain at the 3'-end. The tracrRNAs were synthesized having chemical modifications as indicated, using the 67 nucleotide or 62 nucleotide truncated versions of the tracrRNA sequence. The crRNAs and tracrRNAs listed in Table 12 were paired as indicated and transfected into the HEK-Cas9 cells at 30 nM concentration and processed as described in previous Examples. Relative gene editing activities were assessed by comparing cleavage rates in the *HPRT1* gene using the T7EI mismatch endonuclease cleavage assay, with quantitative measurement of products done using the Fragment Analyzer.

**Table 12: Activity of highly modified crRNA:tracrRNA complexes to direct Cas9-mediated gene editing in mammalian cells.**

| **cr/tracr RNA pair** | **SEQ ID No.** | **crRNA Sequence** | **Cleavage %** |
|---|---|---|---|
| | | **tracrRNA Sequence** | |
| 1 | 448 | *c***u***u***auaucca***acacuucgugguuu***uagagcuau***g***c***u* | 57 |
| | 2 | | |
| 2 | 448 | | 58 |
| | 100 | | |
| 3 | 48 | cuuauauccaacacuucgugguuuuagagcuaugcu | 58 |
| | 449 | | |
| 4 | 48 | cuuauauccaacacuucgugguuuuagagcuaugcu | 57 |
| | 450 | | |
| 5 | 48 | cuuauauccaacacuucgugguuuuagagcuaugcu | 65 |
| | 451 | | |

Oligonucleotide sequences are shown 5'-3'. Lowercase = RNA; Underlined = 2'-O-methyl RNA; Lowercase italic = 2'F RNA; * = phosphorothioate internucleotide linkage. The relative functional activity of each complex is indicated by the % cleavage in a T7EI heteroduplex assay for each dose studied.

The crRNA:tracrRNA pairs #1 and #2 show that a highly 2'F RNA modified crRNA (SEQ ID No. 448, which has 22/36 residues modified, or 61%) is highly functional when paired with either an unmodified tracrRNA (SEQ ID No. 2) or a highly 2'OMe modified tracrRNA (SEQ ID No. 100). The crRNA:tracrRNA pairs #3 and #4 show that tracrRNA compositions having moderate (SEQ ID No. 450, with 19/67 residues modified, or 28%) or high (SEQ ID No. 449, with 46/67 residues modified, or 69%) levels of 2'F RNA modification are highly functional. Information derived from Example 6 (in particular, the 2'OMe "walk", SEQ ID Nos. 144-162) was used to identify specific residues that can be modified within the internal domain of the tracrRNA (see Fig. 6). The crRNA:tracrRNA pair #5 demonstrates that an extremely highly modified tracrRNA, which in this case was a truncated 62 nucleotide design (SEQ ID No. 451, having 51/62 residues modified with 2'OMe RNA, or 82%), has high potency in triggering CRISPR genome editing in mammalian cells. Therefore, the original 89 RNA nucleotide wild-type tracrRNA has been optimized herein to a form that has as little as 11 RNA residues remaining (11/62), thereby significantly reducing risk of RNA-based activation of the mammalian innate immune system and reducing the nuclease-susceptible RNA content of the tracrRNA to a minimal level.

The use of the terms "a" and "an" and "the" and similar referents in the context of describing the subject matter of the present disclosure (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the claimed subject matter and does not pose a limitation on the scope of the disclosure unless otherwise claimed. No language in the specification should be construed as indicating any non-claimed element as essential to the practice of the claimed subject matter.

## Claims

1. An isolated tracrRNA comprising a chemically-modified form of one of SEQ ID NOs.:2, 18, 30-33 and 36-39, wherein the isolated tracrRNA displays activity in a Clustered Regularly Interspaced Short Palindromic Repeats (CRISPR)-CRISPR associated (Cas) (CRISPR-Cas) endonuclease system, wherein the chemically-modified form of one of SEQ ID NOs.:2, 18, 30-33 and 36-39 comprises a chemically-modified nucleotide having an end-modifying group selected from a group consisting of a propanediol (C3) spacer, N,N-diethyl-4-(4-nitronaphthalen-1-ylazo)-phenylamine ("ZEN"), and an inverted-dT residue.

2. The isolated tracrRNA of claim 1, wherein the chemically-modified form of one of SEQ ID NOs.:2, 18, 30-33 and 36-39 further comprises a chemically-modified nucleotide having an additional modification selected from a group consisting of a ribose modification and an internucleotide modifying linkage.

3. The isolated tracrRNA of claim 2, wherein the chemically-modified nucleotide having an additional modification consists of a ribose modification selected from the group consisting of 2'OMe, 2'F, a bicyclic nucleic acid and a locked nucleic acid (LNA).

4. The isolated tracrRNA of claim 2, wherein the chemically-modified nucleotide having an additional modification consists of an internucleotide modifying linkage consisting of phosphorothioate modification.

5. The isolated tracrRNA of claim 2, wherein the isolated tracrRNA is selected from the group consisting of SEQ ID NOs.: 97, 99, 104, 105, 130, 131, and 132.

6. An isolated crRNA comprising a chemically-modified form of formula (I):
5'-X-Z-3' (I),
wherein X represents sequences comprising a target-specific protospacer domain comprising from 17 nucleotides to 20 nucleotides and Z represents sequences comprising a tracrRNA-binding domain comprising from 12 nucleotides to 19 nucleotides,
wherein the isolated crRNA displays activity in a Clustered Regularly Interspaced Short Palindromic Repeats (CRISPR)-CRISPR associated (Cas) (CRISPR-Cas) endonuclease system,
wherein the chemically-modified form of formula (I) comprises a chemically-modified nucleotide having an end-modifying group selected from the group consisting of a propanediol (C3) spacer, N,N-diethyl-4-(4-nitronaphthalen-1-ylazo)-phenylamine ("ZEN"), and an inverted-dT residue.

7. The isolated crRNA of claim 6, wherein the chemically-modified form of formula (I) further comprises a chemically-modified nucleotide having an additional modification selected from a group consisting of a ribose modification and an internucleotide modifying linkage.

8. The isolated crRNA of claim 7, wherein the chemically-modified nucleotide having the additional modification consists of a ribose modification selected from a group consisting of 2'OMe, 2'F, a bicyclic nucleic acid and locked nucleic acid (LNA).

9. The isolated crRNA of claim 7, wherein the chemically-modified nucleotide having the additional modification consists of an internucleotide modifying linkage consisting of phosphorothioate modification.

10. The isolated crRNA of claim 7, wherein the chemically-modified form of formula (I) is selected from SEQ ID NOs.: 163, 179-181, 235-237, 240-243, 256, 257, 268-271, 385-406, 438 and 439.

11. An isolated tracrRNA selected from the group consisting of SEQ ID NOs.:11-13, 17, 87-106, 110-114, 116, 117, 126, 129, 130, 131, 132, 134, 136, 144, 145, and 147-156, 159, 160, 449 and 451, wherein the isolated tracrRNA is active in a Clustered Regularly Interspaced Short Palindromic Repeats (CRISPR)/CRISPR-associated protein endonuclease system.

12. An isolated crRNA selected from the group consisting of SEQ ID NOs.: 14, 164, 166-169, 171, 174-178, 186, 187, 189-207, 212-214, 221-224, 226-228, 233-249, 252, 256-259, 264-271, 291-374, 376, 377, 379-384, 430-437 and 445-448, wherein the isolated crRNA is active in a Clustered Regularly Interspaced Short Palindromic Repeats (CRISPR)/CRISPR-associated protein endonuclease system.

## Patentansprüche

1. Eine isolierte tracrRNA umfassend eine chemisch modifizierte Form von einer der SEQUENZIDENTIFIKATIONSNRN.: 2, 18, 30-33 und 36-39, worin die isolierte tracrRNA Aktivität zeigt in einem Clustered Regularly Interspaced Short Palindromic Repeats (CRISPR)-CRISPR assoziierten (Cas) (CRISPR-Cas) Endonukleasesystem, worin die chemisch modifizierte Form von einer der SEQUENZIDENTIFIKATIONSNRN.: 2, 18, 30-33 und 36-39 ein chemisch modifiziertes Nukleotid umfasst, das eine endmodifizierende Gruppe hat, gewählt aus einer Gruppe bestehend aus einem Propandiol (C3) Spacer, N,N-Diethyl-4-(4-nitronaphthalen-l-ylazo)-phenylamin ("ZEN"), und einem inverted-dT-Rest.

2. Die isolierte tracrRNA gemäß Anspruch 1, worin die chemisch modifizierte Form von einer der SEQUENZIDENTIFIKATIONSNRN.: 2, 18, 30-33 und 36-39 weiter ein chemisch modifiziertes Nukleotid umfasst, das eine zusätzliche Modifikation gewählt aus einer Gruppe bestehend aus einer Ribosemodifikation und einer Internukleotid-modifizierenden Verknüpfung hat.

3. Die isolierte tracrRNA gemäß Anspruch 2, worin das chemisch modifizierte Nukleotid, das eine zusätzliche Modifikation hat, aus einer Ribosemodifikation besteht, gewählt aus der Gruppe bestehend aus 2'OMe, 2'F, einer bizyklischen Nukleinsäure und einer verbrückten Nukleinsäure (LNA).

4. Die isolierte tracrRNA gemäß Anspruch 2, worin das chemisch modifizierte Nukleotid, das eine zusätzliche Modifikation hat, aus einer Internukleotid-modifizierenden Verknüpfung bestehend aus einer Phosphorothioatmodifikation besteht.

5. Die isolierte tracrRNA gemäß Anspruch 2, worin die isolierte tracrRNA gewählt ist aus der Gruppe bestehend aus SEQUENZIDENTIFIKATIONSNRN.: 97, 99, 104, 105, 130, 131 und 132.

6. Eine isolierte crRNA umfassend eine chemisch modifizierte From der Formel (I):
5'-X-Z-3' (I)
worin X Sequenzen darstellt, die eine Ziel-spezifische Protospacer-Domäne umfassend von 17 Nukleotiden bis 20 Nukleotiden umfassen, und Z stellt Sequenzen umfassend eine tracrRNA-bindende Domäne umfassend von 12 Nukleotiden bis 19 Nukleotiden dar,
worin die isolierte crRNA Aktivität zeigt in einem Clustered Regularly Interspaced Short Palindromic Repeats (CRISPR)-CRISPR assoziierten (Cas) (CRISPR-Cas) Endonukleasesystem,
worin die chemisch modifizierte Form der Formel (I) ein chemisch modifiziertes Nukleotid umfasst, das eine endmodifizierende Gruppe gewählt aus der Gruppe bestehend aus einem Propandiol (C3) Spacer, N,N-Diethyl-4-(4-nitronaphthalen-1-ylazo)-phenylamin ("ZEN"), und einem inverted-dT-Rest hat.

7. Die isolierte crRNA gemäß Anspruch 6, worin die chemisch modifizierte Form der Formel (I) weiter ein chemisch modifiziertes Nukleotid umfasst, das eine zusätzliche Modifikation gewählt aus einer Gruppe bestehend aus einer Ribosemodifikation und einer Internukleotid-modifizierenden Verknüpfung hat.

8. Die isolierte crRNA gemäß Anspruch 7, worin das chemisch modifizierte Nukleotid, das die zusätzliche Modifikation hat, aus einer Ribosemodifikation besteht, gewählt aus einer Gruppe bestehend aus 2'Ome, 2'F, einer bizyklischen Nukleinsäure und einer verbrückten Nukleinsäure (LNA).

9. Die isolierte crRNA gemäß Anspruch 7, worin das chemisch modifizierte Nukleotid, das die zusätzliche Modifikation hat, aus einer Internukleotid-modifizierenden Verknüpfung bestehend aus einer Phosphorothioatmodifikation besteht.

10. Die isolierte crRNA gemäß Anspruch 7, worin die chemisch modifizierte Form der Formel (I) gewählt ist aus SEQUENZIDENTIFIKATIONSNRN.: 163, 179-181, 235-237, 240-243, 256, 257, 268-271, 385-406, 438 und 439.

11. Eine isolierte tracrRNA gewählt aus der Gruppe bestehend aus SEQUENZIDENTIFIKATIONSNRN.: 11-13, 17, 87-106, 110-114, 116, 117, 126, 129, 130, 131, 132, 134, 136, 144, 145 und 147-156, 159, 160, 449 und 451, worin die isolierte tracrRNA in einem Clustered Regularly Interspaced Short Palindromic Repeats (CRISPR)/CRISPRassoziierten Proteinendonukleasesystem aktiv ist.

12. Eine isolierte crRNA gewählt aus der Gruppe bestehend aus SEQUENZIDENTIFIKATIONSNRN.: 14, 164, 166-169, 171, 174-178, 186, 187, 189-207, 212-214, 221-224, 226-228, 233-249, 252, 256-259, 264-271, 291-374, 376, 377, 379-384, 430-437 und 445-448, worin die isolierte crRNA in einem Clustered Regularly Interspaced Short Palindromic Repeats (CRISPR)/CRISPR-assoziierten Proteinendonukleasesystem aktiv ist.

## Revendications

1. ARNtracr isolé, comprenant une forme chimiquement modifiée de l'une des Séquences N° 2, 18, 30 à 33 et 36 à 39, lequel ARNtracr isolé exerce une activité dans un système d'endonucléase CRISPR-Cas (Clustered Regularly Interspaced Short Palindromic Repeats (CRISPR, courtes répétitions palindromiques regroupées et régulièrement espacées), associée à CRISPR (Cas)), dans lequel la forme chimiquement modifiée de l'une des Séquences N° 2, 18, 30 à 33 et 36 à 39 comporte un nucléotide chimiquement modifié, qui présente un groupe modificateur d'extrémité choisi dans l'ensemble formé par un espaceur propanediol (C3), un groupe « ZEN » ou N,N-diéthyl-4-(4-nitro-napht-l-yl-azo)-phényl-amine, et un résidu dT inversé.

2. ARNtracr isolé, conforme à la revendication 1, dans lequel la forme chimiquement modifiée de l'une des Séquences N° 2, 18, 30 à 33 et 36 à 39 comporte en outre un nucléotide chimiquement modifié, qui présente une modification supplémentaire choisie dans l'ensemble formé par une modification du ribose et un raccord internucléotidique modificateur.

3. ARNtracr isolé, conforme à la revendication 2, dans lequel le nucléotide chimiquement modifié présentant une modification supplémentaire présente une modification du ribose choisie dans l'ensemble constitué par les modifications symbolisées par 2'-OMe et 2'-F et celles entraînant la présence d'un acide nucléique bicyclique ou d'un acide nucléique bloqué (LNA).

4. ARNtracr isolé, conforme à la revendication 2, dans lequel le nucléotide chimiquement modifié présentant une modification supplémentaire présente un raccord internucléotidique modificateur qui consiste en un raccord modifié phosphorothioate.

5. ARNtracr isolé, conforme à la revendication 2, lequel ARNtracr isolé est choisi dans l'ensemble formé par les Séquences N° 97, 99, 104, 105, 130, 131 et 132.

6. ARNcr isolé, comportant une forme chimiquement modifiée de formule (I) :
5'-X-Z-3' (I)
dans laquelle X représente des séquences comprenant un domaine proto-espaceur à cible spécifique, comportant de 17 nucléotides à 20 nucléotides, et Z représente des séquences comprenant un domaine de liaison à un ARNtracr, comportant de 12 nucléotides à 19 nucléotides,
lequel ARNcr isolé exerce une activité dans un système d'endonucléase CRISPR-Cas (Clustered Regularly Interspaced Short Palindromic Repeats (CRISPR, courtes répétitions palindromiques regroupées et régulièrement espacées), associée à CRISPR (Cas)),
et dans lequel la forme chimiquement modifiée de formule (I) comporte un nucléotide chimiquement modifié, qui comporte un groupe modificateur d'extrémité choisi dans l'ensemble formé par un espaceur propanediol (C3), un groupe « ZEN » ou N,N-diéthyl-4-(4-nitro-napht-1-yl-azo)-phényl-amine, et un résidu dT inversé.

7. ARNcr isolé, conforme à la revendication 6, dans lequel la forme chimiquement modifiée de formule (I) comporte en outre un nucléotide chimiquement modifié, qui présente une modification supplémentaire choisie dans l'ensemble formé par une modification du ribose et un raccord internucléotidique modificateur.

8. ARNcr isolé, conforme à la revendication 7, dans lequel le nucléotide chimiquement modifié présentant une modification supplémentaire présente une modification du ribose choisie dans l'ensemble constitué par les modifications symbolisées par 2'-OMe et 2'-F et celles entraînant la présence d'un acide nucléique bicyclique ou d'un acide nucléique bloqué (LNA).

9. ARNcr isolé, conforme à la revendication 7, dans lequel le nucléotide chimiquement modifié présentant une modification supplémentaire présente un raccord internucléotidique modificateur qui consiste en un raccord modifié phosphorothioate.

10. ARNcr isolé, conforme à la revendication 7, dans lequel la forme chimiquement modifiée de formule (I) est choisie parmi les Séquences N° 163, 179 à 181, 235 à 237, 240 à 243, 256, 257, 268 à 271, 385 à 406, 438 et 439.

11. ARNtracr isolé, choisi dans l'ensemble formé par les Séquences N° 11 à 13, 17, 87 à 106, 110 à 114, 116, 117, 126, 129, 130, 131, 132, 134, 136, 144, 145, 147 à 156, 159, 160, 449 et 451, lequel ARNtracr isolé est actif dans un système d'endonucléase CRISPR (Clustered Regularly Interspaced Short Palindromic Repeats)/protéine associée à CRISPR.

12. ARNcr isolé, choisi dans l'ensemble formé par les Séquences N° 14, 164, 166 à 169, 171, 174 à 178, 186, 187, 189 à 207, 212 à 214, 221 à 224, 226 à 228, 233 à 249, 252, 256 à 259, 264 à 271, 291 à 374, 376, 377, 379 à 384, 430 à 437, et 445 à 448, lequel ARNcr isolé est actif dans un système d'endonucléase CRISPR (Clustered Regularly Interspaced Short Palindromic Repeats)/protéine associée à CRISPR.


### SEQUENCE LISTING

<110> INTEGRATED DNA TECHNOLOGIES, INC.
<120> CRISPR-BASED COMPOSITIONS AND METHODS OF USE
<130> IDT01-008-PCT-EP
<140> EP 15828610.4
   <141> 2015-12-18
<150> PCT/US2015/066942
   <151> 2015-12-18
<150> 62/239,546
   <151> 2015-10-09
<150> 62/093,588
   <151> 2014-12-18
<160> 451
<170> PatentIn version 3.5
<210> 1
   <211> 35
   <212> RNA
   <213> Artificial Sequence
<220>
   <221> source
   <223> /note="Description of Artificial Sequence: Synthetic oligonucleotide"
<400> 1
   uuauauccaa cacuucgugg uuuuagagcu augcu 35
<210> 2
   <211> 67
   <212> RNA
   <213> Artificial Sequence
<220>
   <221> source
   <223> /note="Description of Artificial Sequence: Synthetic oligonucleotide"
<400> 2
   agcauagcaa guuaaaauaa ggcuaguccg uuaucaacuu gaaaaagugg caccgagucg 60
   gugcuuu 67
<210> 3
   <211> 89
   <212> RNA
   <213> Artificial Sequence
<220>
   <221> source
   <223> /note="Description of Artificial Sequence: Synthetic oligonucleotide"
<220>
   <221> modified_base
   <222> (1)..(89)
   <223> 2'-O-methyl nucleotide
<400> 3
   guuggaacca uucaaaacag cauagcaagu uaaaauaagg cuaguccguu aucaacuuga 60
   aaaaguggca ccgagucggu gcuuuuuuu 89
<210> 4
   <211> 67
   <212> RNA
   <213> Artificial Sequence
<220>
   <221> source
   <223> /note="Description of Artificial Sequence: Synthetic oligonucleotide"
<220>
   <221> modified_base
   <222> (1)..(67)
   <223> 2'-O-methyl nucleotide
<400> 4
   agcauagcaa guuaaaauaa ggcuaguccg uuaucaacuu gaaaaagugg caccgagucg 60
   gugcuuu 67
<210> 5
   <211> 41
   <212> RNA
   <213> Artificial Sequence
<220>
   <221> source
   <223> /note="Description of Artificial Sequence: Synthetic oligonucleotide"
<400> 5
   uuauauccaa cacuucgugg uuuuagagcu augcuguuuu g 41
<210> 6
   <211> 89
   <212> RNA
   <213> Artificial Sequence
<220>
   <221> source
   <223> /note="Description of Artificial Sequence: Synthetic oligonucleotide"
<220>
   <221> modified_base
   <222> (1)..(89)
   <223> 2'-O-methyl nucleotide
<400> 6
   guuggaacca uucaaaacag cauagcaagu uaaaauaagg cuaguccguu aucaacuuga 60
   aaaaguggca ccgagucggu gcuuuuuuu 89
<210> 7
   <211> 41
   <212> RNA
   <213> Artificial Sequence
<220>
   <221> source
   <223> /note="Description of Artificial Sequence: Synthetic oligonucleotide"
<220>
   <221> modified_base
   <222> (1)..(41)
   <223> 2'-O-methyl nucleotide
<400> 7
   uuauauccaa cacuucgugg uuuuagagcu augcuguuuu g 41
<210> 8
   <211> 41
   <212> RNA
   <213> Artificial Sequence
<220>
   <221> source
   <223> /note="Description of Artificial Sequence: Synthetic oligonucleotide"
<220>
   <221> modified_base
   <222> (20)..(41)
   <223> 2'-O-methyl nucleotide
<400> 8
   uuauauccaa cacuucgugg uuuuagagcu augcuguuuu g 41
<210> 9
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (1)..(35)
   <223> 2'-O-methyl nucleotide
<400> 9
   uuauauccaa cacuucgugg uuuuagagcu augcu 35
<210> 10
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (20)..(35)
   <223> 2'-O-methyl nucleotide
<400> 10
   uuauauccaa cacuucgugg uuuuagagcu augcu 35
<210> 11
   <211> 67
   <212> RNA
   <213> Artificial Sequence
<220>
   <221> source
   <223> /note="Description of Artificial Sequence: Synthetic oligonucleotide"
<220>
   <221> modified_base
   <222> (39)..(67)
   <223> 2'-O-methyl nucleotide
<400> 11
   agcauagcaa guuaaaauaa ggcuaguccg uuaucaacuu gaaaaagugg caccgagucg 60
   gugcuuu 67
<210> 12
   <211> 67
   <212> RNA
   <213> Artificial Sequence
<220>
   <221> source
   <223> /note="Description of Artificial Sequence: Synthetic oligonucleotide"
<220>
   <221> modified_base
   <222> (1)..(9)
   <223> 2'-O-methyl nucleotide
<220>
   <221> modified_base
   <222> (16)..(16)
   <223> 2'-O-methyl nucleotide
<220>
   <221> modified_base
   <222> (21)..(24)
   <223> 2'-O-methyl nucleotide
<220>
   <221> modified_base
   <222> (28)..(28)
   <223> 2'-O-methyl nucleotide
<220>
   <221> modified_base
   <222> (39)..(67)
   <223> 2'-O-methyl nucleotide
<400> 12
   agcauagcaa guuaaaauaa ggcuaguccg uuaucaacuu gaaaaagugg caccgagucg 60
   gugcuuu 67
<210> 13
   <211> 67
   <212> RNA
   <213> Artificial Sequence
<220>
   <221> source
   <223> /note="Description of Artificial Sequence: Synthetic oligonucleotide"
<220>
   <221> modified_base
   <222> (1)..(9)
   <223> 2'-O-methyl nucleotide
<220>
   <221> modified_base
   <222> (38)..(67)
   <223> 2'-O-methyl nucleotide
<400> 13
   agcauagcaa guuaaaauaa ggcuaguccg uuaucaacuu gaaaaagugg caccgagucg 60
   gugcuuu 67
<210> 14
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (25)..(35)
   <223> 2'-O-methyl nucleotide
**<400>** 14
   uuauauccaa cacuucgugg uuuuagagcu augcu 35
<210> 15
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (2)..(12)
   <223> 2'-O-methyl nucleotide
<400> 15
   uuauauccaa cacuucgugg uuuuagagcu augcu 35
<210> 16
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (2)..(12)
   <223> 2'-O-methyl nucleotide
<220>
   <221> modified_base
   <222> (25)..(35)
   <223> 2'-O-methyl nucleotide
<400> 16
   uuauauccaa cacuucgugg uuuuagagcu augcu 35
<210> 17
   <211> 67
   <212> RNA
   <213> Artificial Sequence
<220>
   <221> source
   <223> /note="Description of Artificial Sequence: Synthetic oligonucleotide"
<220>
   <221> modified_base
   <222> (39)..(39)
   <223> 2'-O-methyl nucleotide
<220>
   <221> modified_base
   <222> (41)..(41)
   <223> 2'-O-methyl nucleotide
<220>
   <221> modified_base
   <222> (43)..(43)
   <223> 2'-O-methyl nucleotide
<220>
   <221> modified_base
   <222> (45)..(45)
   <223> 2'-O-methyl nucleotide
<220>
   <221> modified_base
   <222> (47)..(47)
   <223> 2'-O-methyl nucleotide
<220>
   <221> modified_base
   <222> (49)..(49)
   <223> 2'-O-methyl nucleotide
<220>
   <221> modified_base
   <222> (51)..(51)
   <223> 2'-O-methyl nucleotide
<220>
   <221> modified_base
   <222> (53)..(53)
   <223> 2'-O-methyl nucleotide
<220>
   <221> modified_base
   <222> (55)..(55)
   <223> 2'-O-methyl nucleotide
<220>
   <221> modified_base
   <222> (57)..(57)
   <223> 2'-O-methyl nucleotide
<220>
   <221> modified_base
   <222> (59)..(59)
   <223> 2'-O-methyl nucleotide
<220>
   <221> modified_base
   <222> (61)..(61)
   <223> 2'-O-methyl nucleotide
<220>
   <221> modified_base
   <222> (63)..(63)
   <223> 2'-O-methyl nucleotide
<220>
   <221> modified_base
   <222> (65)..(65)
   <223> 2'-O-methyl nucleotide
<220>
   <221> modified_base
   <222> (67)..(67)
   <223> 2'-O-methyl nucleotide
<400> 17
   agcauagcaa guuaaaauaa ggcuaguccg uuaucaacuu gaaaaagugg caccgagucg 60
   gugcuuu 67
<210> 18
   <211> 89
   <212> RNA
   <213> Artificial Sequence
<220>
   <221> source
   <223> /note="Description of Artificial Sequence: Synthetic oligonucleotide"
<400> 18
   guuggaacca uucaaaacag cauagcaagu uaaaauaagg cuaguccguu aucaacuuga 60
   aaaaguggca ccgagucggu gcuuuuuuu 89
<210> 19
   <211> 89
   <212> RNA
   <213> Artificial Sequence
<220>
   <221> source
   <223> /note="Description of Artificial Sequence: Synthetic oligonucleotide"
<220>
   <221> modified_base
   <222> (57)..(89)
   <223> 2'-O-methyl nucleotide
<400> 19
   guuggaacca uucaaaacag cauagcaagu uaaaauaagg cuaguccguu aucaacuuga 60
   aaaaguggca ccgagucggu gcuuuuuuu 89
<210> 20
   <211> 89
   <212> RNA
   <213> Artificial Sequence
<220>
   <221> source
   <223> /note="Description of Artificial Sequence: Synthetic oligonucleotide"
<220>
   <221> modified_base
   <222> (57)..(57)
   <223> 2'-O-methyl nucleotide
<220>
   <221> modified_base
   <222> (59)..(59)
   <223> 2'-O-methyl nucleotide
<220>
   <221> modified_base
   <222> (61)..(61)
   <223> 2'-O-methyl nucleotide
<220>
   <221> modified_base
   <222> (63)..(63)
   <223> 2'-O-methyl nucleotide
<220>
   <221> modified_base
   <222> (65)..(65)
   <223> 2'-O-methyl nucleotide
<220>
   <221> modified_base
   <222> (67)..(67)
   <223> 2'-O-methyl nucleotide
<220>
   <221> modified_base
   <222> (69)..(69)
   <223> 2'-O-methyl nucleotide
<220>
   <221> modified_base
   <222> (71)..(71)
   <223> 2'-O-methyl nucleotide
<220>
   <221> modified_base
   <222> (73)..(73)
   <223> 2'-O-methyl nucleotide
<220>
   <221> modified_base
   <222> (75)..(75)
   <223> 2'-O-methyl nucleotide
<220>
   <221> modified_base
   <222> (77)..(77)
   <223> 2'-O-methyl nucleotide
<220>
   <221> modified_base
   <222> (79)..(79)
   <223> 2'-O-methyl nucleotide
<220>
   <221> modified_base
   <222> (81)..(81)
   <223> 2'-O-methyl nucleotide
<220>
   <221> modified_base
   <222> (83)..(83)
   <223> 2'-O-methyl nucleotide
<220>
   <221> modified_base
   <222> (85)..(85)
   <223> 2'-O-methyl nucleotide
<220>
   <221> modified_base
   <222> (87)..(87)
   <223> 2'-O-methyl nucleotide
<220>
   <221> modified_base
   <222> (89)..(89)
   <223> 2'-O-methyl nucleotide
<400> 20
   guuggaacca uucaaaacag cauagcaagu uaaaauaagg cuaguccguu aucaacuuga 60
   aaaaguggca ccgagucggu gcuuuuuuu 89
<210> 21
   <211> 89
   <212> RNA
   <213> Artificial Sequence
<220>
   <221> source
   <223> /note="Description of Artificial Sequence: Synthetic oligonucleotide"
<220>
   <221> modified_base
   <222> (1)..(22)
   <223> 2'-O-methyl nucleotide
<220>
   <221> modified_base
   <222> (34)..(34)
   <223> 2'-O-methyl nucleotide
<220>
   <221> modified_base
   <222> (39)..(42)
   <223> 2'-O-methyl nucleotide
<220>
   <221> modified_base
   <222> (46)..(46)
   <223> 2'-O-methyl nucleotide
<220>
   <221> modified_base
   <222> (56)..(89)
   <223> 2'-O-methyl nucleotide
<400> 21
   guuggaacca uucaaaacag cauagcaagu uaaaauaagg cuaguccguu aucaacuuga 60
   aaaaguggca ccgagucggu gcuuuuuuu 89
<210> 22
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (1)..(19)
   <223> 2'-O-methyl nucleotide
<400> 22
   uuauauccaa cacuucgugg uuuuagagcu augcu 35
<210> 23
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (1)..(2)
   <223> 2'-fluoro nucleotide
<220>
   <221> modified_base
   <222> (4)..(4)
   <223> 2'-fluoro nucleotide
<220>
   <221> modified_base
   <222> (6)..(8)
   <223> 2'-fluoro nucleotide
<220>
   <221> modified_base
   <222> (11)..(11)
   <223> 2'-fluoro nucleotide
<220>
   <221> modified_base
   <222> (13)..(16)
   <223> 2'-fluoro nucleotide
<220>
   <221> modified_base
   <222> (18)..(18)
   <223> 2'-fluoro nucleotide
<220>
   <221> modified_base
   <222> (21)..(24)
   <223> 2'-fluoro nucleotide
<220>
   <221> modified_base
   <222> (29)..(30)
   <223> 2'-fluoro nucleotide
<220>
   <221> modified_base
   <222> (32)..(32)
   <223> 2'-fluoro nucleotide
<220>
   <221> modified_base
   <222> (34)..(35)
   <223> 2'-fluoro nucleotide
<400> 23
   uuauauccaa cacuucgugg uuuuagagcu augcu 35
<210> 24
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (1)..(35)
   <223> 2'-fluoro nucleotide
<400> 24
   uuauauccaa cacuucgugg uuuuagagcu augcu 35
<210> 25
   <211> 41
   <212> RNA
   <213> Artificial Sequence
<220>
   <221> source
   <223> /note="Description of Artificial Sequence: Synthetic oligonucleotide"
<220>
   <221> modified_base
   <222> (1)..(19)
   <223> 2'-O-methyl nucleotide
<400> 25
   uuauauccaa cacuucgugg uuuuagagcu augcuguuuu g 41
<210> 26
   <211> 41
   <212> RNA
   <213> Artificial Sequence
<220>
   <221> source
   <223> /note="Description of Artificial Sequence: Synthetic oligonucleotide"
<220>
   <221> modified_base
   <222> (2)..(12)
   <223> 2'-O-methyl nucleotide
<220>
   <221> modified_base
   <222> (25)..(40)
   <223> 2'-O-methyl nucleotide
<400> 26
   uuauauccaa cacuucgugg uuuuagagcu augcuguuuu g 41
<210> 27
   <211> 938
   <212> DNA
   <213> Homo sapiens
<400> 27
   gaatgttgtg ataaaaggtg atgctcacct ctcccacacc cttttatagt ttagggattg 60
   tatttccaag gtttctagac tgagagccct tttcatcttt gctcattgac actctgtacc 120
   cattaatcct ccttattagc tccccttcaa tggacacatg ggtagtcagg gtgcaggtct 180
   cagaactgtc cttcaggttc caggtgatca accaagtgcc ttgtctgtag tgtcaactca 240
   ttgctgcccc ttcctagtaa tccccataat ttagctctcc atttcatagt ctttccttgg 300
   gtgtgttaaa agtgaccatg gtacactcag cacggatgaa atgaaacagt gtttagaaac 360
   gtcagtcttc tcttttgtaa tgccctgtag tctctctgta tgttatatgt cacattttgt 420
   aattaacagc ttgctggtga aaaggacccc acgaagtgtt ggatataagc cagactgtaa 480
   gtgaattact ttttttgtca atcatttaac catctttaac ctaaaagagt tttatgtgaa 540
   atggcttata attgcttaga gaatatttgt agagaggcac atttgccagt attagattta 600
   aaagtgatgt tttctttatc taaatgatga attatgattc tttttagttg ttggatttga 660
   aattccagac aagtttgttg taggatatgc ccttgactat aatgaatact tcagggattt 720
   gaatgtaagt aattgcttct ttttctcact catttttcaa aacacgcata aaaatttagg 780
   aaagagaatt gttttctcct tccagcacct cataatttga acagactgat ggttcccatt 840
   agtcacataa agctgtagtc tagtacagac gtccttagaa ctggaacctg gccaggctag 900
   ggtgacactt cttgttggct gaaatagttg aacagctt 938
<210> 28
   <211> 27
   <212> DNA
   <213> Artificial Sequence
<220>
   <221> source
   <223> /note="Description of Artificial Sequence: Synthetic primer"
<400> 28
   aagaatgttg tgataaaagg tgatgct 27
<210> 29
   <211> 24
   <212> DNA
   <213> Artificial Sequence
<220>
   <221> source
   <223> /note="Description of Artificial Sequence: Synthetic primer "
<400> 29
   acacatccat gggacttctg cctc 24
<210> 30
   <211> 74
   <212> RNA
   <213> Artificial Sequence
<220>
   <221> source
   <223> /note="Description of Artificial Sequence: Synthetic oligonucleotide"
<400> 30
   caaaacagca uagcaaguua aaauaaggcu aguccguuau caacuugaaa aaguggcacc 60
   gagucggugc uuuu 74
<210> 31
   <211> 70
   <212> RNA
   <213> Artificial Sequence
<220>
   <221> source
   <223> /note="Description of Artificial Sequence: Synthetic oligonucleotide"
<400> 31
   aacagcauag caaguuaaaa uaaggcuagu ccguuaucaa cuugaaaaag uggcaccgag 60
   ucggugcuuu 70
<210> 32
   <211> 65
   <212> RNA
   <213> Artificial Sequence
<220>
   <221> source
   <223> /note="Description of Artificial Sequence: Synthetic oligonucleotide"
<400> 32
   agcauagcaa guuaaaauaa ggcuaguccg uuaucaacuu gaaaaagugg caccgagucg 60
   gugcu 65
<210> 33
   <211> 63
   <212> RNA
   <213> Artificial Sequence
<220>
   <221> source
   <223> /note="Description of Artificial Sequence: Synthetic oligonucleotide"
<400> 33
   cauagcaagu uaaaauaagg cuaguccguu aucaacuuga aaaaguggca ccgagucggu 60
   gcu 63
<210> 34
   <211> 55
   <212> RNA
   <213> Artificial Sequence
<220>
   <221> source
   <223> /note="Description of Artificial Sequence: Synthetic oligonucleotide"
<400> 34
   agcauagcaa guuaaaauag uuaucaacuu gaaaaagugg caccgagucg gugcu 55
<210> 35
   <211> 49
   <212> RNA
   <213> Artificial Sequence
<220>
   <221> source
   <223> /note="Description of Artificial Sequence: Synthetic oligonucleotide"
<400> 35
   agcauagcaa guuaaaauaa acuugaaaaa guggcaccga gucggugcu 49
<210> 36
   <211> 64
   <212> RNA
   <213> Artificial Sequence
<220>
   <221> source
   <223> /note="Description of Artificial Sequence: Synthetic oligonucleotide"
<400> 36
   agcauagcaa guuaaaauaa ggcuaguccg uuaucaacuu gaaaaagugg caccgagucg 60
   gugc 64
<210> 37
   <211> 63
   <212> RNA
   <213> Artificial Sequence
<220>
   <221> source
   <223> /note="Description of Artificial Sequence: Synthetic oligonucleotide"
<400> 37
   agcauagcaa guuaaaauaa ggcuaguccg uuaucaacuu gaaaaagugg caccgagucg 60
   gug 63
<210> 38
   <211> 62
   <212> RNA
   <213> Artificial Sequence
<220>
   <221> source
   <223> /note="Description of Artificial Sequence: Synthetic oligonucleotide"
<400> 38
   agcauagcaa guuaaaauaa ggcuaguccg uuaucaacuu gaaaaagugg caccgagucg 60
   gu 62
<210> 39
   <211> 61
   <212> RNA
   <213> Artificial Sequence
<220>
   <221> source
   <223> /note="Description of Artificial Sequence: Synthetic oligonucleotide"
<400> 39
   agcauagcaa guuaaaauaa ggcuaguccg uuaucaacuu gaaaaagugg caccgagucg 60
   g 61
<210> 40
   <211> 58
   <212> RNA
   <213> Artificial Sequence
<220>
   <221> source
   <223> /note="Description of Artificial Sequence: Synthetic oligonucleotide"
<400> 40
   agcauagcaa guuaaaauaa ggcuaguccg uuaucaacuu gaaaaagugc cgagucgg 58
<210> 41
   <211> 59
   <212> RNA
   <213> Artificial Sequence
<220>
   <221> source
   <223> /note="Description of Artificial Sequence: Synthetic oligonucleotide"
<400> 41
   agcauagcaa guuaaaauaa ggcuagucca acuugaaaaa guggcaccga gucggugcu 59
<210> 42
   <211> 55
   <212> RNA
   <213> Artificial Sequence
<220>
   <221> source
   <223> /note="Description of Artificial Sequence: Synthetic oligonucleotide"
<400> 42
   agcauagcaa guuaaaauaa ggcuagucca acuugaaaaa guggcaccga gucgg 55
<210> 43
   <211> 52
   <212> RNA
   <213> Artificial Sequence
<220>
   <221> source
   <223> /note="Description of Artificial Sequence: Synthetic oligonucleotide"
<400> 43
   agcauagcaa guuaaaauaa ggcuagucca acuugaaaaa gugccgaguc gg 52
<210> 44
   <211> 49
   <212> RNA
   <213> Artificial Sequence
<220>
   <221> source
   <223> /note="Description of Artificial Sequence: Synthetic oligonucleotide"
<400> 44
   agcauagcaa guuaaaauaa ggcuaguccg uuaucaacuu gaaaaagug 49
<210> 45
   <211> 52
   <212> RNA
   <213> Artificial Sequence
<220>
   <221> source
   <223> /note="Description of Artificial Sequence: Synthetic oligonucleotide"
<400> 45
   agcauagcaa guuaaaauaa ggcuaguccg uuaucagcac cgagucggug cu 52
<210> 46
   <211> 42
   <212> RNA
   <213> Artificial Sequence
<220>
   <221> source
   <223> /note="Description of Artificial Sequence: Synthetic oligonucleotide"
<400> 46
   cuuauaucca acacuucgug guuuuagagc uaugcuguuu ug 42
<210> 47
   <211> 39
   <212> RNA
   <213> Artificial Sequence
<220>
   <221> source
   <223> /note="Description of Artificial Sequence: Synthetic oligonucleotide"
<400> 47
   cuuauaucca acacuucgug guuuuagagc uaugcuguu 39
<210> 48
   <211> 36
   <212> RNA
   <213> Artificial Sequence
<220>
   <221> source
   <223> /note="Description of Artificial Sequence: Synthetic oligonucleotide"
<400> 48
   cuuauaucca acacuucgug guuuuagagc uaugcu 36
<210> 49
   <211> 34
   <212> RNA
   <213> Artificial Sequence
<220>
   <221> source
   <223> /note="Description of Artificial Sequence: Synthetic oligonucleotide"
<400> 49
   cuuauaucca acacuucgug guuuuagagc uaug 34
<210> 50
   <211> 74
   <212> RNA
   <213> Artificial Sequence
<220>
   <221> source
   <223> /note="Description of Artificial Sequence: Synthetic oligonucleotide"
<400> 50
   caaaacagca uagcaaguua aaauaaggcu aguccguuau caacuugaaa aaguggcacc 60
   gagucggugc uuuu 74
<210> 51
   <211> 70
   <212> RNA
   <213> Artificial Sequence
<220>
   <221> source
   <223> /note="Description of Artificial Sequence: Synthetic oligonucleotide"
<400> 51
   aacagcauag caaguuaaaa uaaggcuagu ccguuaucaa cuugaaaaag uggcaccgag 60
   ucggugcuuu 70
<210> 52
   <211> 65
   <212> RNA
   <213> Artificial Sequence
<220>
   <221> source
   <223> /note="Description of Artificial Sequence: Synthetic oligonucleotide"
<400> 52
   agcauagcaa guuaaaauaa ggcuaguccg uuaucaacuu gaaaaagugg caccgagucg 60
   gugcu 65
<210> 53
   <211> 63
   <212> RNA
   <213> Artificial Sequence
<220>
   <221> source
   <223> /note="Description of Artificial Sequence: Synthetic oligonucleotide"
<400> 53
   cauagcaagu uaaaauaagg cuaguccguu aucaacuuga aaaaguggca ccgagucggu 60
   gcu 63
<210> 54
   <211> 34
   <212> RNA
   <213> Artificial Sequence
<220>
   <221> source
   <223> /note="Description of Artificial Sequence: Synthetic oligonucleotide"
<400> 54
   uauauccaac acuucguggu uuuagagcua ugcu 34
<210> 55
   <211> 33
   <212> RNA
   <213> Artificial Sequence
<220>
   <221> source
   <223> /note="Description of Artificial Sequence: Synthetic oligonucleotide"
<400> 55
   auauccaaca cuucgugguu uuagagcuau gcu 33
<210> 56
   <211> 36
   <212> RNA
   <213> Artificial Sequence
<220>
   <221> source
   <223> /note="Description of Artificial Sequence: Synthetic oligonucleotide"
<400> 56
   aauuaugggg auuacuagga guuuuagagc uaugcu 36
<210> 57
   <211> 35
   <212> RNA
   <213> Artificial Sequence
<220>
   <221> source
   <223> /note="Description of Artificial Sequence: Synthetic oligonucleotide"
<400> 57
   auuaugggga uuacuaggag uuuuagagcu augcu 35
<210> 58
   <211> 34
   <212> RNA
   <213> Artificial Sequence
<220>
   <221> source
   <223> /note="Description of Artificial Sequence: Synthetic oligonucleotide"
<400> 58
   uuauggggau uacuaggagu uuuagagcua ugcu 34
<210> 59
   <211> 33
   <212> RNA
   <213> Artificial Sequence
<220>
   <221> source
   <223> /note="Description of Artificial Sequence: Synthetic oligonucleotide"
<400> 59
   uauggggauu acuaggaguu uuagagcuau gcu 33
<210> 60
   <211> 36
   <212> RNA
   <213> Artificial Sequence
<220>
   <221> source
   <223> /note="Description of Artificial Sequence: Synthetic oligonucleotide"
<400> 60
   auuucacaua aaacucuuuu guuuuagagc uaugcu 36
<210> 61
   <211> 35
   <212> RNA
   <213> Artificial Sequence
<220>
   <221> source
   <223> /note="Description of Artificial Sequence: Synthetic oligonucleotide"
<400> 61
   uuucacauaa aacucuuuug uuuuagagcu augcu 35
<210> 62
   <211> 34
   <212> RNA
   <213> Artificial Sequence
<220>
   <221> source
   <223> /note="Description of Artificial Sequence: Synthetic oligonucleotide"
<400> 62
   uucacauaaa acucuuuugu uuuagagcua ugcu 34
<210> 63
   <211> 33
   <212> RNA
   <213> Artificial Sequence
<220>
   <221> source
   <223> /note="Description of Artificial Sequence: Synthetic oligonucleotide"
<400> 63
   ucacauaaaa cucuuuuguu uuagagcuau gcu 33
<210> 64
   <211> 36
   <212> RNA
   <213> Artificial Sequence
<220>
   <221> source
   <223> /note="Description of Artificial Sequence: Synthetic oligonucleotide"
<400> 64
   uccauuucau agucuuuccu guuuuagagc uaugcu 36
<210> 65
   <211> 35
   <212> RNA
   <213> Artificial Sequence
<220>
   <221> source
   <223> /note="Description of Artificial Sequence: Synthetic oligonucleotide"
<400> 65
   ccauuucaua gucuuuccug uuuuagagcu augcu 35
<210> 66
   <211> 34
   <212> RNA
   <213> Artificial Sequence
<220>
   <221> source
   <223> /note="Description of Artificial Sequence: Synthetic oligonucleotide"
<400> 66
   cauuucauag ucuuuccugu uuuagagcua ugcu 34
<210> 67
   <211> 33
   <212> RNA
   <213> Artificial Sequence
<220>
   <221> source
   <223> /note="Description of Artificial Sequence: Synthetic oligonucleotide"
<400> 67
   auuucauagu cuuuccuguu uuagagcuau gcu 33
<210> 68
   <211> 42
   <212> RNA
   <213> Artificial Sequence
<220>
   <221> source
   <223> /note="Description of Artificial Sequence: Synthetic oligonucleotide"
<400> 68
   uccauuucau agucuuuccu guuuuagagc uaugcuguuu ug 42
<210> 69
   <211> 36
   <212> RNA
   <213> Artificial Sequence
<220>
   <221> source
   <223> /note="Description of Artificial Sequence: Synthetic oligonucleotide"
<400> 69
   uuuuguaauu aacagcuugc guuuuagagc uaugcu 36
<210> 70
   <211> 42
   <212> RNA
   <213> Artificial Sequence
<220>
   <221> source
   <223> /note="Description of Artificial Sequence: Synthetic oligonucleotide"
<400> 70
   uuuuguaauu aacagcuugc guuuuagagc uaugcuguuu ug 42
<210> 71
   <211> 36
   <212> RNA
   <213> Artificial Sequence
<220>
   <221> source
   <223> /note="Description of Artificial Sequence: Synthetic oligonucleotide"
<400> 71
   cuuagagaau auuuguagag guuuuagagc uaugcu 36
<210> 72
   <211> 42
   <212> RNA
   <213> Artificial Sequence
<220>
   <221> source
   <223> /note="Description of Artificial Sequence: Synthetic oligonucleotide"
<400> 72
   cuuagagaau auuuguagag guuuuagagc uaugcuguuu ug 42
<210> 73
   <211> 36
   <212> RNA
   <213> Artificial Sequence
<220>
   <221> source
   <223> /note="Description of Artificial Sequence: Synthetic oligonucleotide"
<400> 73
   uugacuauaa ugaauacuuc guuuuagagc uaugcu 36
<210> 74
   <211> 42
   <212> RNA
   <213> Artificial Sequence
<220>
   <221> source
   <223> /note="Description of Artificial Sequence: Synthetic oligonucleotide"
<400> 74
   uugacuauaa ugaauacuuc guuuuagagc uaugcuguuu ug 42
<210> 75
   <211> 36
   <212> RNA
   <213> Artificial Sequence
<220>
   <221> source
   <223> /note="Description of Artificial Sequence: Synthetic oligonucleotide"
<400> 75
   caaaacacgc auaaaaauuu guuuuagagc uaugcu 36
<210> 76
   <211> 42
   <212> RNA
   <213> Artificial Sequence
<220>
   <221> source
   <223> /note="Description of Artificial Sequence: Synthetic oligonucleotide"
<400> 76
   caaaacacgc auaaaaauuu guuuuagagc uaugcuguuu ug 42
<210> 77
   <211> 42
   <212> RNA
   <213> Artificial Sequence
<220>
   <221> source
   <223> /note="Description of Artificial Sequence: Synthetic oligonucleotide"
<400> 77
   aauuaugggg auuacuagga guuuuagagc uaugcuguuu ug 42
<210> 78
   <211> 36
   <212> RNA
   <213> Artificial Sequence
<220>
   <221> source
   <223> /note="Description of Artificial Sequence: Synthetic oligonucleotide"
<400> 78
   ggucacuuuu aacacaccca guuuuagagc uaugcu 36
<210> 79
   <211> 42
   <212> RNA
   <213> Artificial Sequence
<220>
   <221> source
   <223> /note="Description of Artificial Sequence: Synthetic oligonucleotide"
<400> 79
   ggucacuuuu aacacaccca guuuuagagc uaugcuguuu ug 42
<210> 80
   <211> 36
   <212> RNA
   <213> Artificial Sequence
<220>
   <221> source
   <223> /note="Description of Artificial Sequence: Synthetic oligonucleotide"
<400> 80
   ggcuuauauc caacacuucg guuuuagagc uaugcu 36
<210> 81
   <211> 42
   <212> RNA
   <213> Artificial Sequence
<220>
   <221> source
   <223> /note="Description of Artificial Sequence: Synthetic oligonucleotide"
<400> 81
   ggcuuauauc caacacuucg guuuuagagc uaugcuguuu ug 42
<210> 82
   <211> 42
   <212> RNA
   <213> Artificial Sequence
<220>
   <221> source
   <223> /note="Description of Artificial Sequence: Synthetic oligonucleotide"
<400> 82
   auuucacaua aaacucuuuu guuuuagagc uaugcuguuu ug 42
<210> 83
   <211> 36
   <212> RNA
   <213> Artificial Sequence
<220>
   <221> source
   <223> /note="Description of Artificial Sequence: Synthetic oligonucleotide"
<400> 83
   ucaaauuaug aggugcugga guuuuagagc uaugcu 36
<210> 84
   <211> 42
   <212> RNA
   <213> Artificial Sequence
<220>
   <221> source
   <223> /note="Description of Artificial Sequence: Synthetic oligonucleotide"
<400> 84
   ucaaauuaug aggugcugga guuuuagagc uaugcuguuu ug 42
<210> 85
   <211> 36
   <212> RNA
   <213> Artificial Sequence
<220>
   <221> source
   <223> /note="Description of Artificial Sequence: Synthetic oligonucleotide"
<400> 85
   uacagcuuua ugugacuaau guuuuagagc uaugcu 36
<210> 86
   <211> 42
   <212> RNA
   <213> Artificial Sequence
<220>
   <221> source
   <223> /note="Description of Artificial Sequence: Synthetic oligonucleotide"
<400> 86
   uacagcuuua ugugacuaau guuuuagagc uaugcuguuu ug 42
<210> 87
   <211> 67
   <212> RNA
   <213> Artificial Sequence
<220>
   <221> source
   <223> /note="Description of Artificial Sequence: Synthetic oligonucleotide"
<220>
   <221> modified_base
   <222> (1) .. (5)
   <223> 2'-O-methyl nucleotide
<400> 87
   agcauagcaa guuaaaauaa ggcuaguccg uuaucaacuu gaaaaagugg caccgagucg 60
   gugcuuu 67
<210> 88
   <211> 67
   <212> RNA
   <213> Artificial Sequence
<220>
   <221> source
   <223> /note="Description of Artificial Sequence: Synthetic oligonucleotide"
<220>
   <221> modified_base
   <222> (65) .. (67)
   <223> 2'-O-methyl nucleotide
<400> 88
   agcauagcaa guuaaaauaa ggcuaguccg uuaucaacuu gaaaaagugg caccgagucg 60
   gugcuuu 67
<210> 89
   <211> 67
   <212> RNA
   <213> Artificial Sequence
<220>
   <221> source
   <223> /note="Description of Artificial Sequence: Synthetic oligonucleotide"
<220>
   <221> modified_base
   <222> (1) .. (5)
   <223> 2'-O-methyl nucleotide
<220>
   <221> modified_base
   <222> (65) .. (67)
   <223> 2'-O-methyl nucleotide
<400> 89
   agcauagcaa guuaaaauaa ggcuaguccg uuaucaacuu gaaaaagugg caccgagucg 60
   gugcuuu 67
<210> 90
   <211> 65
   <212> RNA
   <213> Artificial Sequence
<220>
   <221> source
   <223> /note="Description of Artificial Sequence: Synthetic oligonucleotide"
<220>
   <221> modified_base
   <222> (1) .. (7)
   <223> 2'-O-methyl nucleotide
<400> 90
   agcauagcaa guuaaaauaa ggcuaguccg uuaucaacuu gaaaaagugg caccgagucg 60
   gugcu 65
<210> 91
   <211> 65
   <212> RNA
   <213> Artificial Sequence
<220>
   <221> source
   <223> /note="Description of Artificial Sequence: Synthetic oligonucleotide"
<220>
   <221> modified_base
   <222> (59) .. (64)
   <223> 2'-O-methyl nucleotide
<400> 91
   agcauagcaa guuaaaauaa ggcuaguccg uuaucaacuu gaaaaagugg caccgagucg 60
   gugcu 65
<210> 92
   <211> 65
   <212> RNA
   <213> Artificial Sequence
<220>
   <221> source
   <223> /note="Description of Artificial Sequence: Synthetic oligonucleotide"
<220>
   <221> modified_base
   <222> (45) .. (54)
   <223> 2'-O-methyl nucleotide
<400> 92
   agcauagcaa guuaaaauaa ggcuaguccg uuaucaacuu gaaaaagugg caccgagucg 60
   gugcu 65
<210> 93
   <211> 65
   <212> RNA
   <213> Artificial Sequence
<220>
   <221> source
   <223> /note="Description of Artificial Sequence: Synthetic oligonucleotide"
<220>
   <221> modified_base
   <222> (38) .. (40)
   <223> 2'-O-methyl nucleotide
<220>
   <221> modified_base
   <222> (45) .. (54)
   <223> 2'-O-methyl nucleotide
<400> 93
   agcauagcaa guuaaaauaa ggcuaguccg uuaucaacuu gaaaaagugg caccgagucg 60
   gugcu 65
<210> 94
   <211> 65
   <212> RNA
   <213> Artificial Sequence
<220>
   <221> source
   <223> /note="Description of Artificial Sequence: Synthetic oligonucleotide"
<220>
   <221> modified_base
   <222> (38) .. (40)
   <223> 2'-O-methyl nucleotide
<220>
   <221> modified_base
   <222> (45) .. (54)
   <223> 2'-O-methyl nucleotide
<220>
   <221> modified_base
   <222> (59) .. (64)
   <223> 2'-O-methyl nucleotide
<400> 94
   agcauagcaa guuaaaauaa ggcuaguccg uuaucaacuu gaaaaagugg caccgagucg 60
   gugcu 65
<210> 95
   <211> 65
   <212> RNA
   <213> Artificial Sequence
<220>
   <221> source
   <223> /note="Description of Artificial Sequence: Synthetic oligonucleotide"
<220>
   <221> modified_base
   <222> (1) .. (8)
   <223> 2'-O-methyl nucleotide
<220>
   <221> modified_base
   <222> (38) .. (40)
   <223> 2'-O-methyl nucleotide
<220>
   <221> modified_base
   <222> (45) .. (54)
   <223> 2'-O-methyl nucleotide
<220>
   <221> modified_base
   <222> (59) .. (64)
   <223> 2'-O-methyl nucleotide
<400> 95
   agcauagcaa guuaaaauaa ggcuaguccg uuaucaacuu gaaaaagugg caccgagucg 60
   gugcu 65
<210> 96
   <211> 65
   <212> RNA
   <213> Artificial Sequence
<220>
   <221> source
   <223> /note="Description of Artificial Sequence: Synthetic oligonucleotide"
<220>
   <221> modified_base
   <222> (41) .. (44)
   <223> 2'-O-methyl nucleotide
<220>
   <221> modified_base
   <222> (55) .. (58)
   <223> 2'-O-methyl nucleotide
<400> 96
   agcauagcaa guuaaaauaa ggcuaguccg uuaucaacuu gaaaaagugg caccgagucg 60
   gugcu 65
<210> 97
   <211> 67
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 97
   agcauagcaa guuaaaauaa ggcuaguccg uuaucaacuu gaaaaagugg caccgagucg 60
   gugcuuu 67
<210> 98
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> misc_feature
   <222> (65) .. (67)
   <223> /note="Phosphorothioate linkage"
<400> 98
   agcauagcaa guuaaaauaa ggcuaguccg uuaucaacuu gaaaaagugg caccgagucg 60
   gugcuuu 67
<210> 99
   <211> 67
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> modified_base
   <222> (1) .. (9)
   <223> 2'-O-methyl nucleotide
<220>
   <221> modified_base
   <222> (38) .. (67)
   <223> 2'-O-methyl nucleotide
<220>
   <221> source
   <223> /note="3' C3 spacer"
<400> 99
   agcauagcaa guuaaaauaa ggcuaguccg uuaucaacuu gaaaaagugg caccgagucg 60
   gugcuuu 67
<210> 100
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 100
   agcauagcaa guuaaaauaa ggcuaguccg uuaucaacuu gaaaaagugg caccgagucg 60
   gugcuuu 67
<210> 101
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (4)
   <223> /note="Phosphorothioate linkage"
<220>
   <221> misc_feature
   <222> (64) .. (67)
   <223> /note="Phosphorothioate linkage"
<400> 101
   agcauagcaa guuaaaauaa ggcuaguccg uuaucaacuu gaaaaagugg caccgagucg 60
   gugcuuu 67
<210> 102
   <211> 67
   <212> RNA
   <213> Artificial Sequence
<220>
   <221> source
   <223> /note="Description of Artificial Sequence: Synthetic oligonucleotide"
<220>
   <221> modified_base
   <222> (1) .. (3)
   <223> 2'-O-methyl nucleotide
<220>
   <221> modified_base
   <222> (65) .. (67)
   <223> 2'-O-methyl nucleotide
<400> 102
   agcauagcaa guuaaaauaa ggcuaguccg uuaucaacuu gaaaaagugg caccgagucg 60
   gugcuuu 67
<210> 103
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (4)
   <223> /note="Phosphorothioate linkage"
<220>
   <221> modified_base
   <222> (1) .. (3)
   <223> 2'-O-methyl nucleotide
<220>
   <221> misc_feature
   <222> (64) .. (67)
   <223> /note="Phosphorothioate linkage"
<220>
   <221> modified_base
   <222> (65) .. (67)
   <223> 2'-O-methyl nucleotide
<400> 103
   agcauagcaa guuaaaauaa ggcuaguccg uuaucaacuu gaaaaagugg caccgagucg 60
   gugcuuu 67
<210> 104
   <211> 67
   <212> RNA
   <213> Artificial Sequence
<220>
   <221> source
   <223> /note="Description of Artificial Sequence: Synthetic oligonucleotide"
<220>
   <221> source
   <223> /note="3' ZEN modifier"
<400> 104
   agcauagcaa guuaaaauaa ggcuaguccg uuaucaacuu gaaaaagugg caccgagucg 60
   gugcuuu 67
<210> 105
   <211> 67
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 105
   agcauagcaa guuaaaauaa ggcuaguccg uuaucaacuu gaaaaagugg caccgagucg 60
   gugcuuu 67
<210> 106
   <211> 62
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (38) .. (62)
   <223> 2'-O-methyl nucleotide
<220>
   <221> misc_feature
   <222> (60) .. (62)
   <223> /note="Phosphorothioate linkage"
<400> 106
   agcauagcaa guuaaaauaa ggcuaguccg uuaucaacuu gaaaaagugg caccgagucg 60
   gu 62
<210> 107
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (31) .. (62)
   <223> 2'-O-methyl nucleotide
<220>
   <221> misc_feature
   <222> (60) .. (62)
   <223> /note="Phosphorothioate linkage"
<400> 107
   agcauagcaa guuaaaauaa ggcuaguccg uuaucaacuu gaaaaagugg caccgagucg 60
   gugcuuu 67
<210> 108
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (20)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 108
   agcauagcaa guuaaaauaa ggcuaguccg uuaucaacuu gaaaaagugg caccgagucg 60
   gugcuuu 67
<210> 109
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (15) .. (20)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 109
   agcauagcaa guuaaaauaa ggcuaguccg uuaucaacuu gaaaaagugg caccgagucg 60
   gugcuuu 67
<210> 110
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (37) .. (67)
   <223> 2'-O-methyl nucleotide
<220>
   <221> misc_feature
   <222> (65) .. (67)
   <223> /note="Phosphorothioate linkage"
<400> 110
   agcauagcaa guuaaaauaa ggcuaguccg uuaucaacuu gaaaaagugg caccgagucg 60
   gugcuuu 67
<210> 111
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (36) .. (67)
   <223> 2'-O-methyl nucleotide
<220>
   <221> misc_feature
   <222> (65) .. (67)
   <223> /note="Phosphorothioate linkage"
<400> 111
   agcauagcaa guuaaaauaa ggcuaguccg uuaucaacuu gaaaaagugg caccgagucg 60
   gugcuuu 67
<210> 112
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (35) .. (67)
   <223> 2'-O-methyl nucleotide
<220>
   <221> misc_feature
   <222> (65) .. (67)
   <223> /note="Phosphorothioate linkage"
<400> 112
   agcauagcaa guuaaaauaa ggcuaguccg uuaucaacuu gaaaaagugg caccgagucg 60
   gugcuuu 67
<210> 113
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (34) .. (67)
   <223> 2'-O-methyl nucleotide
<220>
   <221> misc_feature
   <222> (65) .. (67)
   <223> /note="Phosphorothioate linkage"
<400> 113
   agcauagcaa guuaaaauaa ggcuaguccg uuaucaacuu gaaaaagugg caccgagucg 60
   gugcuuu 67
<210> 114
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 114
   agcauagcaa guuaaaauaa ggcuaguccg uuaucaacuu gaaaaagugg caccgagucg 60
   gugcuuu 67
<210> 115
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (32) .. (67)
   <223> 2'-O-methyl nucleotide
<220>
   <221> misc_feature
   <222> (65) .. (67)
   <223> /note="Phosphorothioate linkage"
<400> 115
   agcauagcaa guuaaaauaa ggcuaguccg uuaucaacuu gaaaaagugg caccgagucg 60
   gugcuuu 67
<210> 116
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (10)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 116
   agcauagcaa guuaaaauaa ggcuaguccg uuaucaacuu gaaaaagugg caccgagucg 60
   gugcuuu 67
<210> 117
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 117
   agcauagcaa guuaaaauaa ggcuaguccg uuaucaacuu gaaaaagugg caccgagucg 60
   gugcuuu 67
<210> 118
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (12)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 118
   agcauagcaa guuaaaauaa ggcuaguccg uuaucaacuu gaaaaagugg caccgagucg 60
   gugcuuu 67
<210> 119
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (13)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 119
   agcauagcaa guuaaaauaa ggcuaguccg uuaucaacuu gaaaaagugg caccgagucg 60
   gugcuuu 67
<210> 120
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (14)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 120
   agcauagcaa guuaaaauaa ggcuaguccg uuaucaacuu gaaaaagugg caccgagucg 60
   gugcuuu 67
<210> 121
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (14) .. (20)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 121
   agcauagcaa guuaaaauaa ggcuaguccg uuaucaacuu gaaaaagugg caccgagucg 60
   gugcuuu 67
<210> 122
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (13) .. (20)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 122
   agcauagcaa guuaaaauaa ggcuaguccg uuaucaacuu gaaaaagugg caccgagucg 60
   gugcuuu 67
<210> 123
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (12) .. (20)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 123
   agcauagcaa guuaaaauaa ggcuaguccg uuaucaacuu gaaaaagugg caccgagucg 60
   gugcuuu 67
<210> 124
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (11) .. (20)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 124
   agcauagcaa guuaaaauaa ggcuaguccg uuaucaacuu gaaaaagugg caccgagucg 60
   gugcuuu 67
<210> 125
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (20)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 125
   agcauagcaa guuaaaauaa ggcuaguccg uuaucaacuu gaaaaagugg caccgagucg 60
   gugcuuu 67
<210> 126
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (21) .. (23)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 126
   agcauagcaa guuaaaauaa ggcuaguccg uuaucaacuu gaaaaagugg caccgagucg 60
   gugcuuu 67
<210> 127
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (26) .. (29)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 127
   agcauagcaa guuaaaauaa ggcuaguccg uuaucaacuu gaaaaagugg caccgagucg 60
   gugcuuu 67
<210> 128
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (9)
   <223> 2'-O-methyl nucleotide
<220>
   <221> modified_base
   <222> (21) .. (29)
   <223> 2'-O-methyl nucleotide
<220>
   <221> modified_base
   <222> (38) .. (67)
   <223> 2'-O-methyl nucleotide
<220>
   <221> misc_feature
   <222> (65) .. (67)
   <223> /note="Phosphorothioate linkage"
<400> 128
   agcauagcaa guuaaaauaa ggcuaguccg uuaucaacuu gaaaaagugg caccgagucg 60
   gugcuuu 67
<210> 129
   <211> 67
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<220>
   <221> misc_feature
   <222> (1) .. (3)
   <223> /note="Phosphorothioate linkage"
<220>
   <221> modified_base
   <222> (1) .. (2)
   <223> LNA nucleotide
<220>
   <221> misc_feature
   <222> (65) .. (67)
   <223> /note="Phosphorothioate linkage"
<220>
   <221> modified_base
   <222> (66) .. (67)
   <223> LNA nucleotide
<400> 129
   agcauagcaa guuaaaauaa ggcuaguccg uuaucaacuu gaaaaagugg caccgagucg 60
   gugcutt 67
<210> 130
   <211> 68
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<220>
   <221> source
   <223> /note="5' C3 spacer"
<220>
   <221> modified_base
   <222> (1) .. (9)
   <223> 2'-O-methyl nucleotide
<220>
   <221> modified_base
   <222> (38) .. (67)
   <223> 2'-O-methyl nucleotide
<220>
   <221> modified_base
   <222> (68) .. (68)
   <223> Inverted-dT
<400> 130
   agcauagcaa guuaaaauaa ggcuaguccg uuaucaacuu gaaaaagugg caccgagucg 60
   gugcuuut 68
<210> 131
   <211> 62
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> modified_base
   <222> (1) .. (9)
   <223> 2'-O-methyl nucleotide
<220>
   <221> modified_base
   <222> (38) .. (62)
   <223> 2'-O-methyl nucleotide
<220>
   <221> source
   <223> /note="3' C3 spacer"
<400> 131
   agcauagcaa guuaaaauaa ggcuaguccg uuaucaacuu gaaaaagugg caccgagucg 60
   gu 62
<210> 132
   <211> 63
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<220>
   <221> source
   <223> /note="5' C3 spacer"
<220>
   <221> modified_base
   <222> (1) .. (9)
   <223> 2'-O-methyl nucleotide
<220>
   <221> modified_base
   <222> (38) .. (62)
   <223> 2'-O-methyl nucleotide
<220>
   <221> modified_base
   <222> (63) .. (63)
   <223> Inverted-dT
<400> 132
   agcauagcaa guuaaaauaa ggcuaguccg uuaucaacuu gaaaaagugg caccgagucg 60
   gut 63
<210> 133
   <211> 67
   <212> RNA
   <213> Artificial Sequence
<220>
   <221> source
   <223> /note="Description of Artificial Sequence: Synthetic oligonucleotide"
<220>
   <221> modified_base
   <222> (1) .. (9)
   <223> 2'-O-methyl nucleotide
<220>
   <221> misc_feature
   <222> (9) .. (38)
   <223> /note="Phosphorothioate linkage"
<220>
   <221> modified_base
   <222> (38) .. (67)
   <223> 2'-O-methyl nucleotide
<400> 133
   agcauagcaa guuaaaauaa ggcuaguccg uuaucaacuu gaaaaagugg caccgagucg 60
   gugcuuu 67
<210> 134
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 134
   agcauagcaa guuaaaauaa ggcuaguccg uuaucaacuu gaaaaagugg caccgagucg 60
   gugcuuu 67
<210> 135
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (32) .. (67)
   <223> 2'-O-methyl nucleotide
<220>
   <221> misc_feature
   <222> (65) .. (67)
   <223> /note="Phosphorothioate linkage"
<400> 135
   agcauagcaa guuaaaauaa ggcuaguccg uuaucaacuu gaaaaagugg caccgagucg 60
   gugcuuu 67
<210> 136
   <211> 62
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (33) .. (62)
   <223> 2'-O-methyl nucleotide
<220>
   <221> misc_feature
   <222> (60) .. (62)
   <223> /note="Phosphorothioate linkage"
<400> 136
   agcauagcaa guuaaaauaa ggcuaguccg uuaucaacuu gaaaaagugg caccgagucg 60
   gu 62
<210> 137
   <211> 62
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (32) .. (62)
   <223> 2'-O-methyl nucleotide
<220>
   <221> misc_feature
   <222> (60) .. (62)
   <223> /note="Phosphorothioate linkage"
<400> 137
   agcauagcaa guuaaaauaa ggcuaguccg uuaucaacuu gaaaaagugg caccgagucg 60
   gu 62
<210> 138
   <211> 67
   <212> RNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (12) .. (32)
   <223> DNA nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 138
   agcauagcaa gttaaaataa ggctagtccg ttaucaacuu gaaaaagugg caccgagucg 60
   gugcuuu 67
<210> 139
   <211> 67
   <212> RNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (12) .. (21)
   <223> DNA nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 139
   agcauagcaa gttaaaataa ggcuaguccg uuaucaacuu gaaaaagugg caccgagucg 60
   gugcuuu 67
<210> 140
   <211> 67
   <212> RNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (22) .. (32)
   <223> DNA nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 140
   agcauagcaa guuaaaauaa ggctagtccg ttaucaacuu gaaaaagugg caccgagucg 60
   gugcuuu 67
<210> 141
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (12) .. (32)
   <223> 2'-fluoro nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 141
   agcauagcaa guuaaaauaa ggcuaguccg uuaucaacuu gaaaaagugg caccgagucg 60
   gugcuuu 67
<210> 142
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (12) .. (21)
   <223> 2'-fluoro nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 142
   agcauagcaa guuaaaauaa ggcuaguccg uuaucaacuu gaaaaagugg caccgagucg 60
   gugcuuu 67
<210> 143
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (22) .. (32)
   <223> 2'-fluoro nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 143
   agcauagcaa guuaaaauaa ggcuaguccg uuaucaacuu gaaaaagugg caccgagucg 60
   gugcuuu 67
<210> 144
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (13) .. (13)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 144
   agcauagcaa guuaaaauaa ggcuaguccg uuaucaacuu gaaaaagugg caccgagucg 60
   gugcuuu 67
<210> 145
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (14) .. (14)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 145
   agcauagcaa guuaaaauaa ggcuaguccg uuaucaacuu gaaaaagugg caccgagucg 60
   gugcuuu 67
<210> 146
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (15) .. (15)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 146
   agcauagcaa guuaaaauaa ggcuaguccg uuaucaacuu gaaaaagugg caccgagucg 60
   gugcuuu 67
<210> 147
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (16) .. (16)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 147
   agcauagcaa guuaaaauaa ggcuaguccg uuaucaacuu gaaaaagugg caccgagucg 60
   gugcuuu 67
<210> 148
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (17) .. (17)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 148
   agcauagcaa guuaaaauaa ggcuaguccg uuaucaacuu gaaaaagugg caccgagucg 60
   gugcuuu 67
<210> 149
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (18) .. (18)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 149
   agcauagcaa guuaaaauaa ggcuaguccg uuaucaacuu gaaaaagugg caccgagucg 60
   gugcuuu 67
<210> 150
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (19) .. (19)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 150
   agcauagcaa guuaaaauaa ggcuaguccg uuaucaacuu gaaaaagugg caccgagucg 60
   gugcuuu 67
<210> 151
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (20) .. (20)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 151
   agcauagcaa guuaaaauaa ggcuaguccg uuaucaacuu gaaaaagugg caccgagucg 60
   gugcuuu 67
<210> 152
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (21) .. (21)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 152
   agcauagcaa guuaaaauaa ggcuaguccg uuaucaacuu gaaaaagugg caccgagucg 60
   gugcuuu 67
<210> 153
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (22) .. (22)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 153
   agcauagcaa guuaaaauaa ggcuaguccg uuaucaacuu gaaaaagugg caccgagucg 60
   gugcuuu 67
<210> 154
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (23) .. (23)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 154
   agcauagcaa guuaaaauaa ggcuaguccg uuaucaacuu gaaaaagugg caccgagucg 60
   gugcuuu 67
<210> 155
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1) .. (11)
   <223> 2'-O-methyl nucleotide
<220>
   <221> modified_base
   <222> (24) .. (24)
   <223> 2'-O-methyl nucleotide
<220>
   <221> modified_base
   <222> (33) .. (67)
   <223> 2'-O-methyl nucleotide
<220>
   <221> misc_feature
   <222> (65) .. (67)
   <223> /note="Phosphorothioate linkage"
<400> 155
   agcauagcaa guuaaaauaa ggcuaguccg uuaucaacuu gaaaaagugg caccgagucg 60
   gugcuuu 67
<210> 156
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1) .. (3)
   <223> /note="Phosphorothioate linkage"
<220>
   <221> modified_base
   <222> (1)..(11)
   <223> 2'-O-methyl nucleotide
<220>
   <221> modified_base
   <222> (25) .. (25)
   <223> 2'-O-methyl nucleotide
<220>
   <221> modified_base
   <222> (33)..(67)
   <223> 2'-O-methyl nucleotide
<220>
   <221> misc_feature
   <222> (65)..(67)
   <223> /note="Phosphorothioate linkage"
<400> 156
   agcauagcaa guuaaaauaa ggcuaguccg uuaucaacuu gaaaaagugg caccgagucg 60
   gugcuuu 67
<210> 157
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1)..(3)
   <223> /note="Phosphorothioate linkage"
<220>
   <221> modified_base
   <222> (1)..(11)
   <223> 2'-O-methyl nucleotide
<220>
   <221> modified_base
   <222> (26)..(26)
   <223> 2'-O-methyl nucleotide
<220>
   <221> modified_base
   <222> (33)..(67)
   <223> 2'-O-methyl nucleotide
<220>
   <221> misc_feature
   <222> (65)..(67)
   <223> /note="Phosphorothioate linkage"
<400> 157
   agcauagcaa guuaaaauaa ggcuaguccg uuaucaacuu gaaaaagugg caccgagucg 60
   gugcuuu 67
<210> 158
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1)..(3)
   <223> /note="Phosphorothioate linkage"
<220>
   <221> modified_base
   <222> (1)..(11)
   <223> 2'-O-methyl nucleotide
<220>
   <221> modified_base
   <222> (27)..(27)
   <223> 2'-O-methyl nucleotide
<220>
   <221> modified_base
   <222> (33)..(67)
   <223> 2'-O-methyl nucleotide
<220>
   <221> misc_feature
   <222> (65)..(67)
   <223> /note="Phosphorothioate linkage"
<400> 158
   agcauagcaa guuaaaauaa ggcuaguccg uuaucaacuu gaaaaagugg caccgagucg 60
   gugcuuu 67
<210> 159
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1)..(3)
   <223> /note="Phosphorothioate linkage"
<220>
   <221> modified_base
   <222> (1)..(11)
   <223> 2'-O-methyl nucleotide
<220>
   <221> modified_base
   <222> (28)..(28)
   <223> 2'-O-methyl nucleotide
<220>
   <221> modified_base
   <222> (33)..(67)
   <223> 2'-O-methyl nucleotide
<220>
   <221> misc_feature
   <222> (65)..(67)
   <223> /note="Phosphorothioate linkage"
<400> 159
   agcauagcaa guuaaaauaa ggcuaguccg uuaucaacuu gaaaaagugg caccgagucg 60
   gugcuuu 67
<210> 160
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1)..(3)
   <223> /note="Phosphorothioate linkage"
<220>
   <221> modified_base
   <222> (1)..(11)
   <223> 2'-O-methyl nucleotide
<220>
   <221> modified_base
   <222> (29)..(29)
   <223> 2'-O-methyl nucleotide
<220>
   <221> modified_base
   <222> (33)..(67)
   <223> 2'-O-methyl nucleotide
<220>
   <221> misc_feature
   <222> (65)..(67)
   <223> /note="Phosphorothioate linkage"
<400> 160
   agcauagcaa guuaaaauaa ggcuaguccg uuaucaacuu gaaaaagugg caccgagucg 60
   gugcuuu 67
<210> 161
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1)..(3)
   <223> /note="Phosphorothioate linkage"
<220>
   <221> modified_base
   <222> (1)..(11)
   <223> 2'-O-methyl nucleotide
<220>
   <221> modified_base
   <222> (30)..(30)
   <223> 2'-O-methyl nucleotide
<220>
   <221> modified_base
   <222> (33)..(67)
   <223> 2'-O-methyl nucleotide
<220>
   <221> misc_feature
   <222> (65)..(67)
   <223> /note="Phosphorothioate linkage"
<400> 161
   agcauagcaa guuaaaauaa ggcuaguccg uuaucaacuu gaaaaagugg caccgagucg 60
   gugcuuu 67
<210> 162
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1)..(3)
   <223> /note="Phosphorothioate linkage"
<220>
   <221> modified_base
   <222> (1)..(11)
   <223> 2'-O-methyl nucleotide
<220>
   <221> modified_base
   <222> (31)..(31)
   <223> 2'-O-methyl nucleotide
<220>
   <221> modified_base
   <222> (33)..(67)
   <223> 2'-O-methyl nucleotide
<220>
   <221> misc_feature
   <222> (65)..(67)
   <223> /note="Phosphorothioate linkage"
<400> 162
   agcauagcaa guuaaaauaa ggcuaguccg uuaucaacuu gaaaaagugg caccgagucg 60
   gugcuuu 67
<210> 163
   <211> 35
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 163
   uuauauccaa cacuucgugg uuuuagagcu augcu 35
<210> 164
   <211> 35
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> misc_feature
   <222> (32)..(35)
   <223> /note="Phosphorothioate linkage"
<400> 164
   uuauauccaa cacuucgugg uuuuagagcu augcu 35
<210> 165
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (21)..(24)
   <223> 2'-O-methyl nucleotide
<400> 165
   uuauauccaa cacuucgugg uuuuagagcu augcu 35
<210> 166
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (3)..(6)
   <223> 2'-O-methyl nucleotide
<400> 166
   uuauauccaa cacuucgugg uuuuagagcu augcu 35
<210> 167
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (29)..(34)
   <223> 2'-O-methyl nucleotide
<400> 167
   uuauauccaa cacuucgugg uuuuagagcu augcu 35
<210> 168
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (28)..(28)
   <223> 2'-O-methyl nucleotide
<220>
   <221> modified_base
   <222> (30)..(30)
   <223> 2'-O-methyl nucleotide
<220>
   <221> modified_base
   <222> (32)..(32)
   <223> 2'-O-methyl nucleotide
<220>
   <221> modified_base
   <222> (34)..(34)
   <223> 2'-0-methyl nucleotide
<400> 168
   uuauauccaa cacuucgugg uuuuagagcu augcu 35
<210> 169
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (27)..(27)
   <223> 2'-O-methyl nucleotide
<220>
   <221> modified_base
   <222> (29)..(29)
   <223> 2'-O-methyl nucleotide
<220>
   <221> modified_base
   <222> (31)..(31)
   <223> 2'-O-methyl nucleotide
<220>
   <221> modified_base
   <222> (33)..(33)
   <223> 2'-O-methyl nucleotide
<220>
   <221> modified_base
   <222> (35)..(35)
   <223> 2'-O-methyl nucleotide
<400> 169
   uuauauccaa cacuucgugg uuuuagagcu augcu 35
<210> 170
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (15)..(17)
   <223> 2'-O-methyl nucleotide
<400> 170
   uuauauccaa cacuucgugg uuuuagagcu augcu 35
<210> 171
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (3)..(8)
   <223> 2'-O-methyl nucleotide
<400> 171
   uuauauccaa cacuucgugg uuuuagagcu augcu 35
<210> 172
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (2)..(2)
   <223> 2'-0-methyl nucleotide
<220>
   <221> modified_base
   <222> (4)..(4)
   <223> 2'-0-methyl nucleotide
<220>
   <221> modified_base
   <222> (6)..(6)
   <223> 2'-0-methyl nucleotide
<220>
   <221> modified_base
   <222> (8)..(8)
   <223> 2'-O-methyl nucleotide
<220>
   <221> modified_base
   <222> (10)..(10)
   <223> 2'-0-methyl nucleotide
<220>
   <221> modified_base
   <222> (12)..(12)
   <223> 2'-0-methyl nucleotide
<400> 172
   uuauauccaa cacuucgugg uuuuagagcu augcu 35
<210> 173
   <211> 35
   <212> RNA
   <213> Artificial Sequence
<220>
   <221> source
   <223> /note="Description of Artificial Sequence: Synthetic oligonucleotide"
<220>
   <221> modified_base
   <222> (20)..(27)
   <223> 2'-0-methyl nucleotide
<400> 173
   uuauauccaa cacuucgugg uuuuagagcu augcu 35
<210> 174
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (1)..(3)
   <223> 2'-O-methyl nucleotide
<400> 174
   cuuauaucca acacuucgug guuuuagagc uaugcu 36
<210> 175
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (34)..(36)
   <223> 2'-O-methyl nucleotide
<400> 175
   cuuauaucca acacuucgug guuuuagagc uaugcu 36
<210> 176
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (1)..(3)
   <223> 2'-O-methyl nucleotide
<220>
   <221> modified_base
   <222> (34)..(36)
   <223> 2'-O-methyl nucleotide
<400> 176
   cuuauaucca acacuucgug guuuuagagc uaugcu 36
<210> 177
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> misc_feature
   <222> (33)..(36)
   <223> /note="Phosphorothioate linkage"
<400> 177
   cuuauaucca acacuucgug guuuuagagc uaugcu 36
<210> 178
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> misc_feature
   <222> (33)..(36)
   <223> /note="Phosphorothioate linkage"
<400> 178
   cuuauaucca acacuucgug guuuuagagc uaugcu 36
<210> 179
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<400> 179
   cuuauaucca acacuucgug guuuuagagc uaugcu 36
<210> 180
   <211> 36
   <212> RNA
   <213> Artificial Sequence
<220>
   <221> source
   <223> /note="Description of Artificial Sequence: Synthetic oligonucleotide"
<220>
   <221> source
   <223> /note="3' C3 spacer"
<400> 180
   cuuauaucca acacuucgug guuuuagagc uaugcu 36
<210> 181
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 181
   cuuauaucca acacuucgug guuuuagagc uaugcu 36
<210> 182
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<400> 182
   cuuauaucca acacuucgug guuuuagagc uaugcu 36
<210> 183
   <211> 36
   <212> RNA
   <213> Artificial Sequence
<220>
   <221> source
   <223> /note="Description of Artificial Sequence: Synthetic oligonucleotide"
<220>
   <221> source
   <223> /note="3' ZEN modifier"
<400> 183
   cuuauaucca acacuucgug guuuuagagc uaugcu 36
<210> 184
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 184
   cuuauaucca acacuucgug guuuuagagc uaugcu 36
<210> 185
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> modified_base
   <222> (1)..(3)
   <223> 2'-O-methyl nucleotide
<220>
   <221> modified_base
   <222> (34)..(36)
   <223> 2'-O-methyl nucleotide
<220>
   <221> source
   <223> /note="3' ZEN modifier"
<400> 185
   cuuauaucca acacuucgug guuuuagagc uaugcu 36
<210> 186
   <211> 35
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 186
   uuauauccaa cacuucgugg uuuuagagcu augcu 35
<210> 187
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-0-methyl nucleotide
<220>
   <221> modified_base
   <222> (11)..(11)
   <223> 2'-O-methyl nucleotide
<220>
   <221> modified_base
   <222> (13)..(14)
   <223> 2'-O-methyl nucleotide
<220>
   <221> modified_base
   <222> (17)..(18)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 187
   cuuauaucca acacuucgug guuuuagagc uaugcu 36
<210> 188
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (22)..(25)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 188
   cuuauaucca acacuucgug guuuuagagc uaugcu 36
<210> 189
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (33)..(36)
   <223> 2'-O-methyl nucleotide
<400> 189
   cuuauaucca acacuucgug guuuuagagc uaugcu 36
<210> 190
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (32)..(36)
   <223> 2'-O-methyl nucleotide
<220>
   <221> misc_feature
   <222> (33)..(36)
   <223> /note="Phosphorothioate linkage"
<400> 190
   cuuauaucca acacuucgug guuuuagagc uaugcu 36
<210> 191
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (31)..(36)
   <223> 2'-O-methyl nucleotide
<220>
   <221> misc_feature
   <222> (33)..(36)
   <223> /note="Phosphorothioate linkage"
<400> 191
   cuuauaucca acacuucgug guuuuagagc uaugcu 36
<210> 192
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (30)..(36)
   <223> 2'-O-methyl nucleotide
<220>
   <221> misc_feature
   <222> (33)..(36)
   <223> /note="Phosphorothioate linkage"
<400> 192
   cuuauaucca acacuucgug guuuuagagc uaugcu 36
<210> 193
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (29)..(36)
   <223> 2'-O-methyl nucleotide
<220>
   <221> misc_feature
   <222> (33)..(36)
   <223> /note="Phosphorothioate linkage"
<400> 193
   cuuauaucca acacuucgug guuuuagagc uaugcu 36
<210> 194
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (28)..(36)
   <223> 2'-O-methyl nucleotide
<220>
   <221> misc_feature
   <222> (33)..(36)
   <223> /note="Phosphorothioate linkage"
<400> 194
   cuuauaucca acacuucgug guuuuagagc uaugcu 36
<210> 195
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (27)..(36)
   <223> 2'-O-methyl nucleotide
<220>
   <221> misc_feature
   <222> (33)..(36)
   <223> /note="Phosphorothioate linkage"
<400> 195
   cuuauaucca acacuucgug guuuuagagc uaugcu 36
<210> 196
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 196
   cuuauaucca acacuucgug guuuuagagc uaugcu 36
<210> 197
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(5)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 197
   cuuauaucca acacuucgug guuuuagagc uaugcu 36
<210> 198
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 198
   cuuauaucca acacuucgug guuuuagagc uaugcu 36
<210> 199
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(7)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 199
   cuuauaucca acacuucgug guuuuagagc uaugcu 36
<210> 200
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 200
   cuuauaucca acacuucgug guuuuagagc uaugcu 36
<210> 201
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(9)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 201
   cuuauaucca acacuucgug guuuuagagc uaugcu 36
<210> 202
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 202
   cuuauaucca acacuucgug guuuuagagc uaugcu 36
<210> 203
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(11)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 203
   cuuauaucca acacuucgug guuuuagagc uaugcu 36
<210> 204
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(11)
   <223> 2'-O-methyl nucleotide
<220>
   <221> modified_base
   <222> (27)..(36)
   <223> 2'-O-methyl nucleotide
<220>
   <221> misc_feature
   <222> (33)..(36)
   <223> /note="Phosphorothioate linkage"
<400> 204
   cuuauaucca acacuucgug guuuuagagc uaugcu 36
<210> 205
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (17)..(18)
   <223> 2'-O-methyl nucleotide
<220>
   <221> modified_base
   <222> (27)..(36)
   <223> 2'-O-methyl nucleotide
<220>
   <221> misc_feature
   <222> (33)..(36)
   <223> /note="Phosphorothioate linkage"
<400> 205
   cuuauaucca acacuucgug guuuuagagc uaugcu 36
<210> 206
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (26)..(36)
   <223> 2'-O-methyl nucleotide
<220>
   <221> misc_feature
   <222> (33)..(36)
   <223> /note="Phosphorothioate linkage"
<400> 206
   cuuauaucca acacuucgug guuuuagagc uaugcu 36
<210> 207
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (25)..(36)
   <223> 2'-O-methyl nucleotide
<220>
   ²<221> misc_feature
<222> (33)..(36)
   <223> /note="Phosphorothioate linkage"
<400> 207
   cuuauaucca acacuucgug guuuuagagc uaugcu 36
<210> 208
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (24)..(36)
   <223> 2'-O-methyl nucleotide
<220>
   <221> misc_feature
   <222> (33)..(36)
   <223> /note="Phosphorothioate linkage"
<400> 208
   cuuauaucca acacuucgug guuuuagagc uaugcu 36
<210> 209
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (23)..(36)
   <223> 2'-O-methyl nucleotide
<220>
   <221> misc_feature
   <222> (33)..(36)
   <223> /note="Phosphorothioate linkage"
<400> 209
   cuuauaucca acacuucgug guuuuagagc uaugcu 36
<210> 210
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (22)..(36)
   <223> 2'-O-methyl nucleotide
<220>
   <221> misc_feature
   <222> (33)..(36)
   <223> /note="Phosphorothioate linkage"
<400> 210
   cuuauaucca acacuucgug guuuuagagc uaugcu 36
<210> 211
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (21)..(36)
   <223> 2'-O-methyl nucleotide
<220>
   <221> misc_feature
   <222> (33)..(36)
   <223> /note="Phosphorothioate linkage"
<400> 211
   cuuauaucca acacuucgug guuuuagagc uaugcu 36
<210> 212
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 212
   cuuauaucca acacuucgug guuuuagagc uaugcu 36
<210> 213
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 213
   cuuauaucca acacuucgug guuuuagagc uaugcu 36
<210> 214
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 214
   cuuauaucca acacuucgug guuuuagagc uaugcu 36
<210> 215
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(15)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 215
   cuuauaucca acacuucgug guuuuagagc uaugcu 36
<210> 216
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(16)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 216
   cuuauaucca acacuucgug guuuuagagc uaugcu 36
<210> 217
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(17)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 217
   cuuauaucca acacuucgug guuuuagagc uaugcu 36
<210> 218
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(18)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 218
   cuuauaucca acacuucgug guuuuagagc uaugcu 36
<210> 219
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(19)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 219
   cuuauaucca acacuucgug guuuuagagc uaugcu 36
<210> 220
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(20)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 220
   cuuauaucca acacuucgug guuuuagagc uaugcu 36
<210> 221
   <211> 36
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<220>
   <221> modified_base
   <222> (1)..(2)
   <223> LNA nucleotide
<220>
   <221> misc_feature
   <222> (1)..(3)
   <223> /note="Phosphorothioate linkage"
<220>
   <221> misc_feature
   <222> (34)..(36)
   <223> /note="Phosphorothioate linkage"
<220>
   <221> modified_base
   <222> (35)..(36)
   <223> LNA nucleotide
<400> 221
   ctuauaucca acacuucgug guuuuagagc uaugct 36
<210> 222
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 222
   cuuauaucca acacuucgug guuuuagagc uaugcu 36
<210> 223
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (26)..(36)
   <223> 2'-O-methyl nucleotide
<220>
   <221> misc_feature
   <222> (33)..(36)
   <223> /note="Phosphorothioate linkage"
<400> 223
   cuuauaucca acacuucgug guuuuagagc uaugcu 36
<210> 224
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (17)..(18)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 224
   cuuauaucca acacuucgug guuuuagagc uaugcu 36
<210> 225
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (16)..(18)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 225
   cuuauaucca acacuucgug guuuuagagc uaugcu 36
<210> 226
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (17)..(19)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 226
   cuuauaucca acacuucgug guuuuagagc uaugcu 36
<210> 227
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (17)..(20)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 227
   cuuauaucca acacuucgug guuuuagagc uaugcu 36
<210> 228
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (17)..(21)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 228
   cuuauaucca acacuucgug guuuuagagc uaugcu 36
<210> 229
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (17)..(22)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 229
   cuuauaucca acacuucgug guuuuagagc uaugcu 36
<210> 230
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (17)..(23)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 230
   cuuauaucca acacuucgug guuuuagagc uaugcu 36
<210> 231
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (23)..(23)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 231
   cuuauaucca acacuucgug guuuuagagc uaugcu 36
<210> 232
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (22)..(22)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 232
   cuuauaucca acacuucgug guuuuagagc uaugcu 36
<210> 233
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (21)..(21)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 233
   cuuauaucca acacuucgug guuuuagagc uaugcu 36
<210> 234
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (20)..(20)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 234
   cuuauaucca acacuucgug guuuuagagc uaugcu 36
<210> 235
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> modified_base
   <222> (1)..(3)
   <223> 2'-O-methyl nucleotide
<220>
   <221> modified_base
   <222> (20)..(20)
   <223> 2'-O-methyl nucleotide
<220>
   <221> modified_base
   <222> (34)..(36)
   <223> 2'-O-methyl nucleotide
<220>
   <221> source
   <223> /note="3' C3 spacer"
<400> 235
   cuuauaucca acacuucgug guuuuagagc uaugcu 36
<210> 236
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> modified_base
   <222> (1)..(13)
   <223> 2'-O-methyl nucleotide
<220>
   <221> modified_base
   <222> (26)..(36)
   <223> 2'-O-methyl nucleotide
<220>
   <221> source
   <223> /note="3' C3 spacer"
<400> 236
   cuuauaucca acacuucgug guuuuagagc uaugcu 36
<210> 237
   <211> 37
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<220>
   <221> source
   <223> /note="5' C3 spacer"
<220>
   <221> modified_base
   <222> (1)..(3)
   <223> 2'-O-methyl nucleotide
<220>
   <221> modified_base
   <222> (20)..(20)
   <223> 2'-O-methyl nucleotide
<220>
   <221> modified_base
   <222> (34)..(36)
   <223> 2'-O-methyl nucleotide
<220>
   <221> modified_base
   <222> (37)..(37)
   <223> Inverted-dT
<220>
   <221> source
   <223> /note="3' C3 spacer"
<400> 237
   cuuauaucca acacuucgug guuuuagagc uaugcut 37
<210> 238
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (20)..(21)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 238
   cuuauaucca acacuucgug guuuuagagc uaugcu 36
<210> 239
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (17)..(18)
   <223> 2'-O-methyl nucleotide
<220>
   <221> modified_base
   <222> (20)..(21)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 239
   cuuauaucca acacuucgug guuuuagagc uaugcu 36
<210> 240
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> modified_base
   <222> (1)..(14)
   <223> 2'-O-methyl nucleotide
<220>
   <221> modified_base
   <222> (20)..(21)
   <223> 2'-O-methyl nucleotide
<220>
   <221> modified_base
   <222> (25)..(36)
   <223> 2'-O-methyl nucleotide
<220>
   <221> source
   <223> /note="3' C3 spacer"
<400> 240
   cuuauaucca acacuucgug guuuuagagc uaugcu 36
<210> 241
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> modified_base
   <222> (1)..(14)
   <223> 2'-O-methyl nucleotide
<220>
   <221> modified_base
   <222> (17)..(18)
   <223> 2'-O-methyl nucleotide
<220>
   <221> modified_base
   <222> (20)..(21)
   <223> 2'-O-methyl nucleotide
<220>
   <221> modified_base
   <222> (25)..(36)
   <223> 2'-O-methyl nucleotide
<220>
   <221> source
   <223> /note="3' C3 spacer"
<400> 241
   cuuauaucca acacuucgug guuuuagagc uaugcu 36
<210> 242
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> modified_base
   <222> (1)..(14)
   <223> 2'-O-methyl nucleotide
<220>
   <221> modified_base
   <222> (20)..(21)
   <223> 2'-O-methyl nucleotide
<220>
   <221> modified_base
   <222> (25)..(36)
   <223> 2'-O-methyl nucleotide
<220>
   <221> source
   <223> /note="3' ZEN modifier"
<400> 242
   cuuauaucca acacuucgug guuuuagagc uaugcu 36
<210> 243
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> modified_base
   <222> (1)..(14)
   <223> 2'-O-methyl nucleotide
<220>
   <221> modified_base
   <222> (17)..(18)
   <223> 2'-O-methyl nucleotide
<220>
   <221> modified_base
   <222> (20)..(21)
   <223> 2'-O-methyl nucleotide
<220>
   <221> modified_base
   <222> (25)..(36)
   <223> 2'-O-methyl nucleotide
<220>
   <221> source
   <223> /note="3' ZEN modifier"
<400> 243
   cuuauaucca acacuucgug guuuuagagc uaugcu 36
<210> 244
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 244
   cuuauaucca acacuucgug guuuuagagc uaugcu 36
<210> 245
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 245
   cuuauaucca acacuucgug guuuuagagc uaugcu 36
<210> 246
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 246
   cuuauaucca acacuucgug guuuuagagc uaugcu 36
<210> 247
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 247
   cuuauaucca acacuucgug guuuuagagc uaugcu 36
<210> 248
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 248
   cuuauaucca acacuucgug guuuuagagc uaugcu 36
<210> 249
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 249
   cuuauaucca acacuucgug guuuuagagc uaugcu 36
<210> 250
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 250
   cuuauaucca acacuucgug guuuuagagc uaugcu 36
<210> 251
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 251
   cuuauaucca acacuucgug guuuuagagc uaugcu 36
<210> 252
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 252
   cuuauaucca acacuucgug guuuuagagc uaugcu 36
<210> 253
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (15)..(24)
   <223> 2'-fluoro nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 253
   cuuauaucca acacuucgug guuuuagagc uaugcu 36
<210> 254
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (15)..(20)
   <223> 2'-fluoro nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 254
   cuuauaucca acacuucgug guuuuagagc uaugcu 36
<210> 255
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(14)
   <223> 2'-O-methyl nucleotide
<220>
   <221> modified_base
   <222> (21)..(24)
   <223> 2'-fluoro nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 255
   cuuauaucca acacuucgug guuuuagagc uaugcu 36
<210> 256
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> modified_base
   <222> (1)..(3)
   <223> 2'-O-methyl nucleotide
<220>
   <221> modified_base
   <222> (25)..(36)
   <223> 2'-O-methyl nucleotide
<220>
   <221> source
   <223> /note="3' C3 spacer"
<400> 256
   cuuauaucca acacuucgug guuuuagagc uaugcu 36
<210> 257
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> source
   <223> /note="3' C3 spacer"
<400> 257
   cuuauaucca acacuucgug guuuuagagc uaugcu 36
<210> 258
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(11)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 258
   cuuauaucca acacuucgug guuuuagagc uaugcu 36
<210> 259
   <211> 35
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(35)
   <223> 2'-O-methyl nucleotide
<220>
   <221> misc_feature
   <222> (32)..(35)
   <223> /note="Phosphorothioate linkage"
<400> 259
   cuuauaucca acacuucgug guuuuagagc uaugc 35
<210> 260
   <211> 34
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(34)
   <223> 2'-O-methyl nucleotide
<220>
   <221> misc_feature
   <222> (31)..(34)
   <223> /note="Phosphorothioate linkage"
<400> 260
   cuuauaucca acacuucgug guuuuagagc uaug 34
<210> 261
   <211> 33
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(33)
   <223> 2'-O-methyl nucleotide
<220>
   <221> misc_feature
   <222> (30)..(33)
   <223> /note="Phosphorothioate linkage"
<400> 261
   cuuauaucca acacuucgug guuuuagagc uau 33
<210> 262
   <211> 32
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(32)
   <223> 2'-O-methyl nucleotide
<220>
   <221> misc_feature
   <222> (29)..(32)
   <223> /note="Phosphorothioate linkage"
<400> 262
   cuuauaucca acacuucgug guuuuagagc ua 32
<210> 263
   <211> 31
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(31)
   <223> 2'-O-methyl nucleotide
<220>
   <221> misc_feature
   <222> (28)..(31)
   <223> /note="Phosphorothioate linkage"
<400> 263
   cuuauaucca acacuucgug guuuuagagc u 31
<210> 264
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (21)..(21)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 264
   cuuauaucca acacuucgug guuuuagagc uaugcu 36
<210> 265
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> misc_feature
   <222> (21)..(25)
   <223> /note="Phosphorothioate linkage"
<220>
   <221> modified_base
   <222> (21)..(21)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 265
   cuuauaucca acacuucgug guuuuagagc uaugcu 36
<210> 266
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> misc_feature
   <222> (10)..(21)
   <223> /note="Phosphorothioate linkage"
<220>
   <221> modified_base
   <222> (21)..(21)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 266
   cuuauaucca acacuucgug guuuuagagc uaugcu 36
<210> 267
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> misc_feature
   <222> (10)..(21)
   <223> /note="Phosphorothioate linkage"
<220>
   <221> modified_base
   <222> (21)..(21)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 267
   cuuauaucca acacuucgug guuuuagagc uaugcu 36
<210> 268
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> modified_base
   <222> (1)..(10)
   <223> 2'-O-methyl nucleotide
<220>
   <221> modified_base
   <222> (21)..(21)
   <223> 2'-O-methyl nucleotide
<220>
   <221> modified_base
   <222> (25)..(36)
   <223> 2'-O-methyl nucleotide
<220>
   <221> source
   <223> /note="3' C3 spacer"
<400> 268
   cuuauaucca acacuucgug guuuuagagc uaugcu 36
<210> 269
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> modified_base
   <222> (1)..(10)
   <223> 2'-O-methyl nucleotide
<220>
   <221> misc_feature
   <222> (21)..(25)
   <223> /note="Phosphorothioate linkage"
<220>
   <221> modified_base
   <222> (21)..(21)
   <223> 2'-O-methyl nucleotide
<220>
   <221> modified_base
   <222> (25)..(36)
   <223> 2'-O-methyl nucleotide
<220>
   <221> source
   <223> /note="3' C3 spacer"
<400> 269
   cuuauaucca acacuucgug guuuuagagc uaugcu 36
<210> 270
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> modified_base
   <222> (1)..(10)
   <223> 2'-O-methyl nucleotide
<220>
   <221> misc_feature
   <222> (10)..(21)
   <223> /note="Phosphorothioate linkage"
<220>
   <221> modified_base
   <222> (21)..(21)
   <223> 2'-O-methyl nucleotide
<220>
   <221> modified_base
   <222> (25)..(36)
   <223> 2'-O-methyl nucleotide
<220>
   <221> source
   <223> /note="3' C3 spacer"
<400> 270
   cuuauaucca acacuucgug guuuuagagc uaugcu 36
<210> 271
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> modified_base
   <222> (1)..(10)
   <223> 2'-O-methyl nucleotide
<220>
   <221> misc_feature
   <222> (10)..(25)
   <223> /note="Phosphorothioate linkage"
<220>
   <221> modified_base
   <222> (21)..(21)
   <223> 2'-O-methyl nucleotide
<220>
   <221> modified_base
   <222> (25)..(36)
   <223> 2'-O-methyl nucleotide
<220>
   <221> source
   <223> /note="3' C3 spacer"
<400> 271
   cuuauaucca acacuucgug guuuuagagc uaugcu 36
<210> 272
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (11)..(11)
   <223> 2'-O-methyl nucleotide
<400> 272
   cuuauaucca acacuucgug guuuuagagc uaugcu 36
<210> 273
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (12)..(12)
   <223> 2'-O-methyl nucleotide
<400> 273
   cuuauaucca acacuucgug guuuuagagc uaugcu 36
<210> 274
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (13)..(13)
   <223> 2'-O-methyl nucleotide
<400> 274
   cuuauaucca acacuucgug guuuuagagc uaugcu 36
<210> 275
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (14)..(14)
   <223> 2'-O-methyl nucleotide
<400> 275
   cuuauaucca acacuucgug guuuuagagc uaugcu 36
<210> 276
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (15)..(15)
   <223> 2'-O-methyl nucleotide
<400> 276
   cuuauaucca acacuucgug guuuuagagc uaugcu 36
<210> 277
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (16)..(16)
   <223> 2'-O-methyl nucleotide
<400> 277
   cuuauaucca acacuucgug guuuuagagc uaugcu 36
<210> 278
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (17)..(17)
   <223> 2'-O-methyl nucleotide
<400> 278
   cuuauaucca acacuucgug guuuuagagc uaugcu 36
<210> 279
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (18)..(18)
   <223> 2'-O-methyl nucleotide
<400> 279
   cuuauaucca acacuucgug guuuuagagc uaugcu 36
<210> 280
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (19)..(19)
   <223> 2'-O-methyl nucleotide
<400> 280
   cuuauaucca acacuucgug guuuuagagc uaugcu 36
<210> 281
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (20)..(20)
   <223> 2'-O-methyl nucleotide
<400> 281
   cuuauaucca acacuucgug guuuuagagc uaugcu 36
<210> 282
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (21)..(21)
   <223> 2'-O-methyl nucleotide
<400> 282
   cuuauaucca acacuucgug guuuuagagc uaugcu 36
<210> 283
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (22)..(22)
   <223> 2'-O-methyl nucleotide
<400> 283
   cuuauaucca acacuucgug guuuuagagc uaugcu 36
<210> 284
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (23)..(23)
   <223> 2'-O-methyl nucleotide
<400> 284
   cuuauaucca acacuucgug guuuuagagc uaugcu 36
<210> 285
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (24)..(24)
   <223> 2'-O-methyl nucleotide
<400> 285
   cuuauaucca acacuucgug guuuuagagc uaugcu 36
<210> 286
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (25)..(25)
   <223> 2'-O-methyl nucleotide
<400> 286
   cuuauaucca acacuucgug guuuuagagc uaugcu 36
<210> 287
   <211> 33
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(7)
   <223> 2'-O-methyl nucleotide
<220>
   <221> modified_base
   <222> (22)..(33)
   <223> 2'-O-methyl nucleotide
<220>
   <221> misc_feature
   <222> (30)..(33)
   <223> /note="Phosphorothioate linkage"
<400> 287
   auauccaaca cuucgugguu uuagagcuau gcu 33
<210> 288
   <211> 32
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (21)..(33)
   <223> 2'-O-methyl nucleotide
<220>
   <221> misc_feature
   <222> (30)..(33)
   <223> /note="Phosphorothioate linkage"
<400> 288
   uauccaacac uucgugguuu uagagcuaug cu 32
<210> 289
   <211> 33
   **<212>** DNA
   **<213>** Artificial Sequence
**<220>**
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<220>
   <221> misc_feature
   <222> (1)..(4)
   <223> /note="Phosphorothioate linkage"
<220>
   <221> modified_base
   <222> (1)..(2)
   <223> LNA nucleotide
<220>
   <221> modified_base
   <222> (3)..(7)
   <223> 2'-O-methyl nucleotide
<220>
   <221> modified_base
   <222> (22)..(33)
   <223> 2'-O-methyl nucleotide
<220>
   <221> misc_feature
   <222> (30)..(33)
   <223> /note="Phosphorothioate linkage"
<400> 289
   atauccaaca cuucgugguu uuagagcuau gcu 33
<210> 290
   <211> 32
   <212> DNA
   <213> Artificial Sequence
<220>
   <221> source
   <223> /note="Description of Artificial Sequence: Synthetic oligonucleotide"
<220>
   <221> source
   <223> /note="Description of Combined DNA/RNA Molecule: Synthetic oligonucleotide"
<220>
   <221> misc_feature
   <222> (1)..(4)
   <223> /note="Phosphorothioate linkage"
<220>
   <221> modified_base
   <222> (1)..(2)
   <223> LNA nucleotide
<220>
   <221> modified_base
   <222> (3)..(6)
   <223> 2'-O-methyl nucleotide
<220>
   <221> modified_base
   <222> (21)..(32)
   <223> 2'-O-methyl nucleotide
<220>
   <221> misc_feature
   <222> (29)..(32)
   <223> /note="Phosphorothioate linkage"
<400> 290
   tauccaacac uucgugguuu uagagcuaug cu 32
<210> 291
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 291
   uccauuucau agucuuuccu guuuuagagc uaugcu 36
<210> 292
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 292
   uuuuguaauu aacagcuugc guuuuagagc uaugcu 36
<210> 293
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 293
   cuuagagaau auuuguagag guuuuagagc uaugcu 36
<210> 294
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 294
   uugacuauaa ugaauacuuc guuuuagagc uaugcu 36
<210> 295
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 295
   caaaacacgc auaaaaauuu guuuuagagc uaugcu 36
<210> 296
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 296
   aauuaugggg auuacuagga guuuuagagc uaugcu 36
<210> 297
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 297
   ggucacuuuu aacacaccca guuuuagagc uaugcu 36
<210> 298
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 298
   ggcuuauauc caacacuucg guuuuagagc uaugcu 36
<210> 299
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 299
   auuucacaua aaacucuuuu guuuuagagc uaugcu 36
<210> 300
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 300
   ucaaauuaug aggugcugga guuuuagagc uaugcu 36
<210> 301
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 301
   uacagcuuua ugugacuaau guuuuagagc uaugcu 36
<210> 302
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 302
   uccauuucau agucuuuccu guuuuagagc uaugcu 36
<210> 303
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 303
   uuuuguaauu aacagcuugc guuuuagagc uaugcu 36
<210> 304
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 304
   cuuagagaau auuuguagag guuuuagagc uaugcu 36
<210> 305
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 305
   uugacuauaa ugaauacuuc guuuuagagc uaugcu 36
<210> 306
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 306
   caaaacacgc auaaaaauuu guuuuagagc uaugcu 36
<210> 307
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 307
   aauuaugggg auuacuagga guuuuagagc uaugcu 36
<210> 308
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 308
   ggucacuuuu aacacaccca guuuuagagc uaugcu 36
<210> 309
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 309
   cuuauaucca acacuucgug guuuuagagc uaugcu 36
<210> 310
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 310
   ggcuuauauc caacacuucg guuuuagagc uaugcu 36
<210> 311
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 311
   auuucacaua aaacucuuuu guuuuagagc uaugcu 36
<210> 312
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 312
   ucaaauuaug aggugcugga guuuuagagc uaugcu 36
<210> 313
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 313
   uacagcuuua ugugacuaau guuuuagagc uaugcu 36
<210> 314
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 314
   uccauuucau agucuuuccu guuuuagagc uaugcu 36
<210> 315
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 315
   uuuuguaauu aacagcuugc guuuuagagc uaugcu 36
<210> 316
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 316
   cuuagagaau auuuguagag guuuuagagc uaugcu 36
<210> 317
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 317
   uugacuauaa ugaauacuuc guuuuagagc uaugcu 36
<210> 318
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 318
   caaaacacgc auaaaaauuu guuuuagagc uaugcu 36
<210> 319
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 319
   aauuaugggg auuacuagga guuuuagagc uaugcu 36
<210> 320
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 320
   ggucacuuuu aacacaccca guuuuagagc uaugcu 36
<210> 321
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 321
   cuuauaucca acacuucgug guuuuagagc uaugcu 36
<210> 322
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 322
   ggcuuauauc caacacuucg guuuuagagc uaugcu 36
<210> 323
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 323
   auuucacaua aaacucuuuu guuuuagagc uaugcu 36
<210> 324
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 324
   ucaaauuaug aggugcugga guuuuagagc uaugcu 36
<210> 325
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 325
   uacagcuuua ugugacuaau guuuuagagc uaugcu 36
<210> 326
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 326
   uccauuucau agucuuuccu guuuuagagc uaugcu 36
<210> 327
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 327
   uuuuguaauu aacagcuugc guuuuagagc uaugcu 36
<210> 328
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 328
   cuuagagaau auuuguagag guuuuagagc uaugcu 36
<210> 329
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 329
   uugacuauaa ugaauacuuc guuuuagagc uaugcu 36
<210> 330
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 330
   caaaacacgc auaaaaauuu guuuuagagc uaugcu 36
<210> 331
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 331
   aauuaugggg auuacuagga guuuuagagc uaugcu 36
<210> 332
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 332
   ggucacuuuu aacacaccca guuuuagagc uaugcu 36
<210> 333
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 333
   cuuauaucca acacuucgug guuuuagagc uaugcu 36
<210> 334
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 334
   ggcuuauauc caacacuucg guuuuagagc uaugcu 36
<210> 335
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 335
   auuucacaua aaacucuuuu guuuuagagc uaugcu 36
<210> 336
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 336
   ucaaauuaug aggugcugga guuuuagagc uaugcu 36
<210> 337
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 337
   uacagcuuua ugugacuaau guuuuagagc uaugcu 36
<210> 338
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 338
   uccauuucau agucuuuccu guuuuagagc uaugcu 36
<210> 339
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 339
   uuuuguaauu aacagcuugc guuuuagagc uaugcu 36
<210> 340
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 340
   cuuagagaau auuuguagag guuuuagagc uaugcu 36
<210> 341
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 341
   uugacuauaa ugaauacuuc guuuuagagc uaugcu 36
<210> 342
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 342
   caaaacacgc auaaaaauuu guuuuagagc uaugcu 36
<210> 343
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 343
   aauuaugggg auuacuagga guuuuagagc uaugcu 36
<210> 344
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 344
   ggucacuuuu aacacaccca guuuuagagc uaugcu 36
<210> 345
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 345
   cuuauaucca acacuucgug guuuuagagc uaugcu 36
<210> 346
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 346
   ggcuuauauc caacacuucg guuuuagagc uaugcu 36
<210> 347
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 347
   auuucacaua aaacucuuuu guuuuagagc uaugcu 36
<210> 348
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 348
   ucaaauuaug aggugcugga guuuuagagc uaugcu 36
<210> 349
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 349
   uacagcuuua ugugacuaau guuuuagagc uaugcu 36
<210> 350
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 350
   uccauuucau agucuuuccu guuuuagagc uaugcu 36
<210> 351
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 351
   uuuuguaauu aacagcuugc guuuuagagc uaugcu 36
<210> 352
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 352
   cuuagagaau auuuguagag guuuuagagc uaugcu 36
<210> 353
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 353
   uugacuauaa ugaauacuuc guuuuagagc uaugcu 36
<210> 354
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 354
   caaaacacgc auaaaaauuu guuuuagagc uaugcu 36
<210> 355
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 355
   aauuaugggg auuacuagga guuuuagagc uaugcu 36
<210> 356
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 356
   ggucacuuuu aacacaccca guuuuagagc uaugcu 36
<210> 357
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 357
   cuuauaucca acacuucgug guuuuagagc uaugcu 36
<210> 358
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 358
   ggcuuauauc caacacuucg guuuuagagc uaugcu 36
<210> 359
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 359
   auuucacaua aaacucuuuu guuuuagagc uaugcu 36
<210> 360
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 360
   ucaaauuaug aggugcugga guuuuagagc uaugcu 36
<210> 361
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 361
   uacagcuuua ugugacuaau guuuuagagc uaugcu 36
<210> 362
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 362
   uccauuucau agucuuuccu guuuuagagc uaugcu 36
<210> 363
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 363
   uuuuguaauu aacagcuugc guuuuagagc uaugcu 36
<210> 364
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 364
   cuuagagaau auuuguagag guuuuagagc uaugcu 36
<210> 365
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 365
   uugacuauaa ugaauacuuc guuuuagagc uaugcu 36
<210> 366
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 366
   caaaacacgc auaaaaauuu guuuuagagc uaugcu 36
<210> 367
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 367
   aauuaugggg auuacuagga guuuuagagc uaugcu 36
<210> 368
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 368
   ggucacuuuu aacacaccca guuuuagagc uaugcu 36
<210> 369
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 369
   cuuauaucca acacuucgug guuuuagagc uaugcu 36
<210> 370
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 370
   ggcuuauauc caacacuucg guuuuagagc uaugcu 36
<210> 371
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 371
   auuucacaua aaacucuuuu guuuuagagc uaugcu 36
<210> 372
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 372
   ucaaauuaug aggugcugga guuuuagagc uaugcu 36
<210> 373
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 373
   uacagcuuua ugugacuaau guuuuagagc uaugcu 36
<210> 374
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 374
   uccauuucau agucuuuccu guuuuagagc uaugcu 36
<210> 375
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 375
   uuuuguaauu aacagcuugc guuuuagagc uaugcu 36
<210> 376
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 376
   cuuagagaau auuuguagag guuuuagagc uaugcu 36
<210> 377
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 377
   uugacuauaa ugaauacuuc guuuuagagc uaugcu 36
<210> 378
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 378
   caaaacacgc auaaaaauuu guuuuagagc uaugcu 36
<210> 379
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 379
   aauuaugggg auuacuagga guuuuagagc uaugcu 36
<210> 380
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 380
   ggucacuuuu aacacaccca guuuuagagc uaugcu 36
<210> 381
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 381
   ggcuuauauc caacacuucg guuuuagagc uaugcu 36
<210> 382
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 382
   auuucacaua aaacucuuuu guuuuagagc uaugcu 36
<210> 383
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 383
   ucaaauuaug aggugcugga guuuuagagc uaugcu 36
<210> 384
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 384
   uacagcuuua ugugacuaau guuuuagagc uaugcu 36
<210> 385
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 385
   uccauuucau agucuuuccu guuuuagagc uaugcu 36
<210> 386
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 386
   uuuuguaauu aacagcuugc guuuuagagc uaugcu 36
<210> 387
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 387
   cuuagagaau auuuguagag guuuuagagc uaugcu 36
<210> 388
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 388
   uugacuauaa ugaauacuuc guuuuagagc uaugcu 36
<210> 389
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 389
   caaaacacgc auaaaaauuu guuuuagagc uaugcu 36
<210> 390
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 390
   aauuaugggg auuacuagga guuuuagagc uaugcu 36
<210> 391
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 391
   ggucacuuuu aacacaccca guuuuagagc uaugcu 36
<210> 392
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 392
   ggcuuauauc caacacuucg guuuuagagc uaugcu 36
<210> 393
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 393
   auuucacaua aaacucuuuu guuuuagagc uaugcu 36
<210> 394
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 394
   ucaaauuaug aggugcugga guuuuagagc uaugcu 36
<210> 395
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> source
   <223> /note="3' C3 spacer"
<400> 395
   uacagcuuua ugugacuaau guuuuagagc uaugcu 36
<210> 396
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 396
   uccauuucau agucuuuccu guuuuagagc uaugcu 36
<210> 397
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 397
   uuuuguaauu aacagcuugc guuuuagagc uaugcu 36
<210> 398
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 398
   cuuagagaau auuuguagag guuuuagagc uaugcu 36
<210> 399
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 399
   uugacuauaa ugaauacuuc guuuuagagc uaugcu 36
<210> 400
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 400
   caaaacacgc auaaaaauuu guuuuagagc uaugcu 36
<210> 401
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 401
   aauuaugggg auuacuagga guuuuagagc uaugcu 36
<210> 402
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 402
   ggucacuuuu aacacaccca guuuuagagc uaugcu 36
<210> 403
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 403
   ggcuuauauc caacacuucg guuuuagagc uaugcu 36
<210> 404
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 404
   auuucacaua aaacucuuuu guuuuagagc uaugcu 36
<210> 405
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 405
   ucaaauuaug aggugcugga guuuuagagc uaugcu 36
<210> 406
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 406
   uacagcuuua ugugacuaau guuuuagagc uaugcu 36
<210> 407
   <211> 1367
   <212> PRT
   <213> Streptococcus pyogenes
<400> 407
   Met Asp Lys Lys Tyr Ser Ile Gly Leu Asp Ile Gly Thr Asn Ser Val
   1               5                   10                  15
   Gly Trp Ala Val Ile Thr Asp Glu Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Gly Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Thr Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Val Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ile Leu Arg Arg Gln Glu Asp Phe Tyr Pro Phe Leu
               420                 425                 430
   Lys Asp Asn Arg Glu Lys Ile Glu Lys Ile Leu Thr Phe Arg Ile Pro
           435                 440                 445
   Tyr Tyr Val Gly Pro Leu Ala Arg Gly Asn Ser Arg Phe Ala Trp Met
       450                 455                 460
   Thr Arg Lys Ser Glu Glu Thr Ile Thr Pro Trp Asn Phe Glu Glu Val
   465                 470                 475                 480
   Val Asp Lys Gly Ala Ser Ala Gln Ser Phe Ile Glu Arg Met Thr Asn
                   485                 490                 495
   Phe Asp Lys Asn Leu Pro Asn Glu Lys Val Leu Pro Lys His Ser Leu
               500                 505                 510
   Leu Tyr Glu Tyr Phe Thr Val Tyr Asn Glu Leu Thr Lys Val Lys Tyr
           515                 520                 525
   Val Thr Glu Gly Met Arg Lys Pro Ala Phe Leu Ser Gly Glu Gln Lys
       530                 535                 540
   Lys Ala Ile Val Asp Leu Leu Phe Lys Thr Asn Arg Lys Val Thr Val
   545                 550                 555                 560
   Lys Gln Leu Lys Glu Asp Tyr Phe Lys Lys Ile Glu Cys Phe Asp Ser
                   565                 570                 575
   Val Glu Ile Ser Gly Val Glu Asp Arg Phe Asn Ala Ser Leu Gly Thr
               580                 585                 590
   Tyr His Asp Leu Leu Lys Ile Ile Lys Asp Lys Asp Phe Leu Asp Asn
           595                 600                 605
   Glu Glu Asn Glu Asp Ile Leu Glu Asp Ile Val Leu Thr Leu Thr Leu
       610                 615                 620
   Phe Glu Asp Arg Glu Met Ile Glu Glu Arg Leu Lys Thr Tyr Ala His
   625                 630                 635                 640
   Leu Phe Asp Asp Lys Val Met Lys Gln Leu Lys Arg Arg Arg Tyr Thr
                   645                 650                 655
   Gly Trp Gly Arg Leu Ser Arg Lys Leu Ile Asn Gly Ile Arg Asp Lys
               660                 665                 670
   Gln Ser Gly Lys Thr Ile Leu Asp Phe Leu Lys Ser Asp Gly Phe Ala
           675                 680                 685
   Asn Arg Asn Phe Met Gln Leu Ile His Asp Asp Ser Leu Thr Phe Lys
       690                 695                 700
   Glu Asp Ile Gln Lys Ala Gln Val Ser Gly Gln Gly Asp Ser Leu His
   705                 710                 715                 720
   Glu His Ile Ala Asn Leu Ala Gly Ser Pro Ala Ile Lys Lys Gly Ile
                   725                 730                 735
   Leu Gln Thr Val Lys Val Val Asp Glu Leu Val Lys Val Met Gly Arg
               740                 745                 750
   His Lys Pro Glu Asn Ile Val Ile Glu Met Ala Arg Glu Asn Gln Thr
           755                 760                 765
   Thr Gln Lys Gly Gln Lys Asn Ser Arg Glu Arg Met Lys Arg Ile Glu
   770 775 780
   Glu Gly Ile Lys Glu Leu Gly Ser Gln Ile Leu Lys Glu His Pro Val
   785 790 795 800
   Glu Asn Thr Gln Leu Gln Asn Glu Lys Leu Tyr Leu Tyr Tyr Leu Gln
   805 810 815
   Asn Gly Arg Asp Met Tyr Val Asp Gln Glu Leu Asp Ile Asn Arg Leu
   820 825 830
   Ser Asp Tyr Asp Val Asp His Ile Val Pro Gln Ser Phe Leu Lys Asp
   835 840 845
   Asp Ser Ile Asp Asn Lys Val Leu Thr Arg Ser Asp Lys Asn Arg Gly
   850 855 860
   Lys Ser Asp Asn Val Pro Ser Glu Glu Val Val Lys Lys Met Lys Asn
   865 870 875 880
   Tyr Trp Arg Gln Leu Leu Asn Ala Lys Leu Ile Thr Gln Arg Lys Phe
   885 890 895
   Asp Asn Leu Thr Lys Ala Glu Arg Gly Gly Leu Ser Glu Leu Asp Lys
   900 905 910
   Ala Gly Phe Ile Lys Arg Gln Leu Val Glu Thr Arg Gln Ile Thr Lys
   915 920 925
   His Val Ala Gln Ile Leu Asp Ser Arg Met Asn Thr Lys Tyr Asp Glu
   930 935 940
   Asn Asp Lys Leu Ile Arg Glu Val Lys Val Ile Thr Leu Lys Ser Lys
   945 950 955 960
   Leu Val Ser Asp Phe Arg Lys Asp Phe Gln Phe Tyr Lys Val Arg Glu
   965 970 975
   Ile Asn Asn Tyr His His Ala His Asp Ala Tyr Leu Asn Ala Val Val
   980 985 990
   Gly Thr Ala Leu Ile Lys Lys Tyr Pro Lys Leu Glu Ser Glu Phe Val
   995 1000 1005
   Tyr Gly Asp Tyr Lys Val Tyr Asp Val Arg Lys Met Ile Ala Lys
   1010 1015 1020
   Ser Glu Gln Glu Ile Gly Lys Ala Thr Ala Lys Tyr Phe Phe Tyr
   1025 1030 1035
   Ser Asn Ile Met Asn Phe Phe Lys Thr Glu Ile Thr Leu Ala Asn
   1040 1045 1050
   Gly Glu Ile Arg Lys Arg Pro Leu Ile Glu Thr Asn Gly Glu Thr
   1055 1060 1065
   Gly Glu Ile Val Trp Asp Lys Gly Arg Asp Phe Ala Thr Val Arg
   1070 1075 1080
   Lys Val Leu Ser Met Pro Gln Val Asn Ile Val Lys Lys Thr Glu
   1085 1090 1095
   Val Gln Thr Gly Gly Phe Ser Lys Glu Ser Ile Leu Pro Lys Arg
   1100 1105 1110
   Asn Ser Asp Lys Leu Ile Ala Arg Lys Lys Asp Trp Asp Pro Lys
   1115 1120 1125
   Lys Tyr Gly Gly Phe Asp Ser Pro Thr Val Ala Tyr Ser Val Leu
   1130 1135 1140
   Val Val Ala Lys Val Glu Lys Gly Lys Ser Lys Lys Leu Lys Ser
   1145 1150 1155
   Val Lys Glu Leu Leu Gly Ile Thr Ile Met Glu Arg Ser Ser Phe
   1160 1165 1170
   Glu Lys Asn Pro Ile Asp Phe Leu Glu Ala Lys Gly Tyr Lys Glu
   1175 1180 1185
   Val Lys Lys Asp Leu Ile Ile Lys Leu Pro Lys Tyr Ser Leu Phe
   1190 1195 1200
   Glu Leu Glu Asn Gly Arg Lys Arg Met Leu Ala Ser Ala Gly Glu
   1205 1210 1215
   Leu Gln Lys Gly Asn Glu Leu Ala Leu Pro Ser Lys Tyr Val Asn
   1220 1225 1230
   Phe Leu Tyr Leu Ala Ser His Tyr Glu Lys Leu Lys Gly Ser Pro
   1235 1240 1245
   Glu Asp Asn Glu Gln Lys Gln Leu Phe Val Glu Gln His Lys His
   1250 1255 1260
   Tyr Leu Asp Glu Ile Ile Glu Gln Ile Ser Glu Phe Ser Lys Arg
   1265 1270 1275
   Val Ile Leu Ala Asp Ala Asn Leu Asp Lys Val Leu Ser Ala Tyr
   1280 1285 1290
   Asn Lys His Arg Asp Lys Pro Ile Arg Glu Gln Ala Glu Asn Ile
   1295 1300 1305
   Ile His Leu Phe Thr Leu Thr Asn Leu Gly Ala Pro Ala Ala Phe
   1310 1315 1320
   Lys Tyr Phe Asp Thr Thr Ile Asp Arg Lys Arg Tyr Thr Ser Thr
   1325 1330 1335
   Lys Glu Val Leu Asp Ala Thr Leu Ile His Gln Ser Ile Thr Gly
   1340 1345 1350
   Leu Tyr Glu Thr Arg Ile Asp Leu Ser Gln Leu Gly Gly Asp
   1355 1360 1365
<210> 408
   <211> 1416
   <212> PRT
   <213> Artificial Sequence
<220>
   <221> source
   <223> /note="Description of Artificial Sequence: Synthetic polypeptide"
<400> 408
   Met Gly Ser Ser Ala Pro Lys Lys Lys Arg Lys Val Gly Ile His Gly
   1 5 10 15
   Val Pro Ala Ala Met Asp Lys Lys Tyr Ser Ile Gly Leu Asp Ile Gly
   20 25 30
   Thr Asn Ser Val Gly Trp Ala Val Ile Thr Asp Glu Tyr Lys Val Pro
   35 40 45
   Ser Lys Lys Phe Lys Val Leu Gly Asn Thr Asp Arg His Ser Ile Lys
   50 55 60
   Lys Asn Leu Ile Gly Ala Leu Leu Phe Asp Ser Gly Glu Thr Ala Glu
   65 70 75 80
   Ala Thr Arg Leu Lys Arg Thr Ala Arg Arg Arg Tyr Thr Arg Arg Lys
                   85                  90                  95
   Asn Arg Ile Cys Tyr Leu Gln Glu Ile Phe Ser Asn Glu Met Ala Lys
               100                 105                 110
   Val Asp Asp Ser Phe Phe His Arg Leu Glu Glu Ser Phe Leu Val Glu
           115                 120                 125
   Glu Asp Lys Lys His Glu Arg His Pro Ile Phe Gly Asn Ile Val Asp
       130                 135                 140
   Glu Val Ala Tyr His Glu Lys Tyr Pro Thr Ile Tyr His Leu Arg Lys
   145                 150                 155                 160
   Lys Leu Val Asp Ser Thr Asp Lys Ala Asp Leu Arg Leu Ile Tyr Leu
                   165                 170                 175
   Ala Leu Ala His Met Ile Lys Phe Arg Gly His Phe Leu Ile Glu Gly
               180                 185                 190
   Asp Leu Asn Pro Asp Asn Ser Asp Val Asp Lys Leu Phe Ile Gln Leu
           195                 200                 205
   Val Gln Thr Tyr Asn Gln Leu Phe Glu Glu Asn Pro Ile Asn Ala Ser
       210                 215                 220
   Gly Val Asp Ala Lys Ala Ile Leu Ser Ala Arg Leu Ser Lys Ser Arg
   225                 230                 235                 240
   Arg Leu Glu Asn Leu Ile Ala Gln Leu Pro Gly Glu Lys Lys Asn Gly
                   245                 250                 255
   Leu Phe Gly Asn Leu Ile Ala Leu Ser Leu Gly Leu Thr Pro Asn Phe
               260                 265                 270
   Lys Ser Asn Phe Asp Leu Ala Glu Asp Ala Lys Leu Gln Leu Ser Lys
           275                 280                 285
   Asp Thr Tyr Asp Asp Asp Leu Asp Asn Leu Leu Ala Gln Ile Gly Asp
       290                 295                 300
   Gln Tyr Ala Asp Leu Phe Leu Ala Ala Lys Asn Leu Ser Asp Ala Ile
   305                 310                 315                 320
   Leu Leu Ser Asp Ile Leu Arg Val Asn Thr Glu Ile Thr Lys Ala Pro
                   325                 330                 335
   Leu Ser Ala Ser Met Ile Lys Arg Tyr Asp Glu His His Gln Asp Leu
               340                 345                 350
   Thr Leu Leu Lys Ala Leu Val Arg Gln Gln Leu Pro Glu Lys Tyr Lys
           355                 360                 365
   Glu Ile Phe Phe Asp Gln Ser Lys Asn Gly Tyr Ala Gly Tyr Ile Asp
       370                 375                 380
   Gly Gly Ala Ser Gln Glu Glu Phe Tyr Lys Phe Ile Lys Pro Ile Leu
   385                 390                 395                 400
   Glu Lys Met Asp Gly Thr Glu Glu Leu Leu Val Lys Leu Asn Arg Glu
                   405                 410                 415
   Asp Leu Leu Arg Lys Gln Arg Thr Phe Asp Asn Gly Ser Ile Pro His
               420                 425                 430
   Gln Ile His Leu Gly Glu Leu His Ala Ile Leu Arg Arg Gln Glu Asp
           435                 440                 445
   Phe Tyr Pro Phe Leu Lys Asp Asn Arg Glu Lys Ile Glu Lys Ile Leu
       450                 455                 460
   Thr Phe Arg Ile Pro Tyr Tyr Val Gly Pro Leu Ala Arg Gly Asn Ser
   465                 470                 475                 480
   Arg Phe Ala Trp Met Thr Arg Lys Ser Glu Glu Thr Ile Thr Pro Trp
                   485                 490                 495
   Asn Phe Glu Glu Val Val Asp Lys Gly Ala Ser Ala Gln Ser Phe Ile
               500                 505                 510
   Glu Arg Met Thr Asn Phe Asp Lys Asn Leu Pro Asn Glu Lys Val Leu
           515                 520                 525
   Pro Lys His Ser Leu Leu Tyr Glu Tyr Phe Thr Val Tyr Asn Glu Leu
       530                 535                 540
   Thr Lys Val Lys Tyr Val Thr Glu Gly Met Arg Lys Pro Ala Phe Leu
   545                 550                 555                 560
   Ser Gly Glu Gln Lys Lys Ala Ile Val Asp Leu Leu Phe Lys Thr Asn
                   565                 570                 575
   Arg Lys Val Thr Val Lys Gln Leu Lys Glu Asp Tyr Phe Lys Lys Ile
               580                 585                 590
   Glu Cys Phe Asp Ser Val Glu Ile Ser Gly Val Glu Asp Arg Phe Asn
   595 600 605
   Ala Ser Leu Gly Thr Tyr His Asp Leu Leu Lys Ile Ile Lys Asp Lys
   610 615 620
   Asp Phe Leu Asp Asn Glu Glu Asn Glu Asp Ile Leu Glu Asp Ile Val
   625 630 635 640
   Leu Thr Leu Thr Leu Phe Glu Asp Arg Glu Met Ile Glu Glu Arg Leu
   645 650 655
   Lys Thr Tyr Ala His Leu Phe Asp Asp Lys Val Met Lys Gln Leu Lys
   660 665 670
   Arg Arg Arg Tyr Thr Gly Trp Gly Arg Leu Ser Arg Lys Leu Ile Asn
   675 680 685
   Gly Ile Arg Asp Lys Gln Ser Gly Lys Thr Ile Leu Asp Phe Leu Lys
   690 695 700
   Ser Asp Gly Phe Ala Asn Arg Asn Phe Met Gln Leu Ile His Asp Asp
   705 710 715 720
   Ser Leu Thr Phe Lys Glu Asp Ile Gln Lys Ala Gln Val Ser Gly Gln
   725 730 735
   Gly Asp Ser Leu His Glu His Ile Ala Asn Leu Ala Gly Ser Pro Ala
   740 745 750
   Ile Lys Lys Gly Ile Leu Gln Thr Val Lys Val Val Asp Glu Leu Val
   755 760 765
   Lys Val Met Gly Arg His Lys Pro Glu Asn Ile Val Ile Glu Met Ala
   770 775 780
   Arg Glu Asn Gln Thr Thr Gln Lys Gly Gln Lys Asn Ser Arg Glu Arg
   785 790 795 800
   Met Lys Arg Ile Glu Glu Gly Ile Lys Glu Leu Gly Ser Gln Ile Leu
   805 810 815
   Lys Glu His Pro Val Glu Asn Thr Gln Leu Gln Asn Glu Lys Leu Tyr
   820 825 830
   Leu Tyr Tyr Leu Gln Asn Gly Arg Asp Met Tyr Val Asp Gln Glu Leu
   835 840 845
   Asp Ile Asn Arg Leu Ser Asp Tyr Asp Val Asp His Ile Val Pro Gln
   850 855 860
   Ser Phe Leu Lys Asp Asp Ser Ile Asp Asn Lys Val Leu Thr Arg Ser
   865 870 875 880
   Asp Lys Asn Arg Gly Lys Ser Asp Asn Val Pro Ser Glu Glu Val Val
   885 890 895
   Lys Lys Met Lys Asn Tyr Trp Arg Gln Leu Leu Asn Ala Lys Leu Ile
   900 905 910
   Thr Gln Arg Lys Phe Asp Asn Leu Thr Lys Ala Glu Arg Gly Gly Leu
   915 920 925
   Ser Glu Leu Asp Lys Ala Gly Phe Ile Lys Arg Gln Leu Val Glu Thr
   930 935 940
   Arg Gln Ile Thr Lys His Val Ala Gln Ile Leu Asp Ser Arg Met Asn
   945 950 955 960
   Thr Lys Tyr Asp Glu Asn Asp Lys Leu Ile Arg Glu Val Lys Val Ile
   965 970 975
   Thr Leu Lys Ser Lys Leu Val Ser Asp Phe Arg Lys Asp Phe Gln Phe
   980 985 990
   Tyr Lys Val Arg Glu Ile Asn Asn Tyr His His Ala His Asp Ala Tyr
   995 1000 1005
   Leu Asn Ala Val Val Gly Thr Ala Leu Ile Lys Lys Tyr Pro Lys
   1010 1015 1020
   Leu Glu Ser Glu Phe Val Tyr Gly Asp Tyr Lys Val Tyr Asp Val
   1025 1030 1035
   Arg Lys Met Ile Ala Lys Ser Glu Gln Glu Ile Gly Lys Ala Thr
   1040 1045 1050
   Ala Lys Tyr Phe Phe Tyr Ser Asn Ile Met Asn Phe Phe Lys Thr
   1055 1060 1065
   Glu Ile Thr Leu Ala Asn Gly Glu Ile Arg Lys Arg Pro Leu Ile
   1070 1075 1080
   Glu Thr Asn Gly Glu Thr Gly Glu Ile Val Trp Asp Lys Gly Arg
   1085 1090 1095
   Asp Phe Ala Thr Val Arg Lys Val Leu Ser Met Pro Gln Val Asn
   1100 1105 1110
   Ile Val Lys Lys Thr Glu Val Gln Thr Gly Gly Phe Ser Lys Glu
   1115 1120 1125
   Ser Ile Leu Pro Lys Arg Asn Ser Asp Lys Leu Ile Ala Arg Lys
   1130 1135 1140
   Lys Asp Trp Asp Pro Lys Lys Tyr Gly Gly Phe Asp Ser Pro Thr
   1145 1150 1155
   Val Ala Tyr Ser Val Leu Val Val Ala Lys Val Glu Lys Gly Lys
   1160 1165 1170
   Ser Lys Lys Leu Lys Ser Val Lys Glu Leu Leu Gly Ile Thr Ile
   1175 1180 1185
   Met Glu Arg Ser Ser Phe Glu Lys Asn Pro Ile Asp Phe Leu Glu
   1190 1195 1200
   Ala Lys Gly Tyr Lys Glu Val Lys Lys Asp Leu Ile Ile Lys Leu
   1205 1210 1215
   Pro Lys Tyr Ser Leu Phe Glu Leu Glu Asn Gly Arg Lys Arg Met
   1220 1225 1230
   Leu Ala Ser Ala Gly Glu Leu Gln Lys Gly Asn Glu Leu Ala Leu
   1235 1240 1245
   Pro Ser Lys Tyr Val Asn Phe Leu Tyr Leu Ala Ser His Tyr Glu
   1250 1255 1260
   Lys Leu Lys Gly Ser Pro Glu Asp Asn Glu Gln Lys Gln Leu Phe
   1265 1270 1275
   Val Glu Gln His Lys His Tyr Leu Asp Glu Ile Ile Glu Gln Ile
   1280 1285 1290
   Ser Glu Phe Ser Lys Arg Val Ile Leu Ala Asp Ala Asn Leu Asp
   1295 1300 1305
   Lys Val Leu Ser Ala Tyr Asn Lys His Arg Asp Lys Pro Ile Arg
   1310 1315 1320
   Glu Gln  Ala Glu Asn Ile Ile  His Leu Phe Thr Leu  Thr Asn Leu
       1325                 1330                 1335
   Gly Ala  Pro Ala Ala Phe Lys  Tyr Phe Asp Thr Thr  Ile Asp Arg
       1340                 1345                 1350
   Lys Arg  Tyr Thr Ser Thr Lys  Glu Val Leu Asp Ala  Thr Leu Ile
       1355                 1360                 1365
   His Gln  Ser Ile Thr Gly Leu  Tyr Glu Thr Arg Ile  Asp Leu Ser
       1370                 1375                 1380
   Gln Leu  Gly Gly Asp Ala Ala  Pro Lys Lys Lys Arg  Lys Val Asp
       1385                 1390                 1395
   Pro Lys  Lys Lys Arg Lys Val  Ala Ala Ala Leu Glu  His His His
       1400                 1405                 1410
   His His  His
       1415
<210> 409
   <211> 4251
   <212> DNA
   <213> Artificial Sequence
<220>
   <221> source
   <223> /note="Description of Artificial Sequence: Synthetic polynucleotide"
<400> 409
   atgggcagca gcgccccaaa gaagaagcgg aaggtcggta tccacggagt cccagcagcc 60
   atggacaaaa agtactctat tggcctggat atcgggacca acagcgtcgg gtgggctgtt 120
   atcaccgacg agtataaagt accttcgaaa aagttcaaag tgctgggcaa caccgatcgc 180
   cattcaatca aaaagaactt gattggtgcg ctgttgtttg actccgggga aaccgccgag 240
   gcgactcgcc ttaaacgtac agcacgtcgc cggtacactc ggcgtaagaa tcgcatttgc 300
   tatttgcagg aaatctttag caacgagatg gcaaaagtcg atgactcgtt tttccaccgc 360
   ctcgaggaaa gctttctggt ggaggaagac aaaaagcatg agcgtcaccc gatcttcggc 420
   aacattgtcg atgaagtagc gtatcatgaa aaatacccaa ccatttacca cttacgcaaa 480
   aagctggtgg acagcactga caaagctgat ttgcgcctta tctatttagc cctggcacat 540
   atgattaagt ttcgtggtca cttcctgatc gaaggagact taaatcccga caacagtgat 600
   gttgataaat tgtttattca gcttgtccaa acttacaatc aactgttcga ggaaaacccg 660
   atcaatgcct ccggtgtgga tgcaaaagcc attttaagtg cacgccttag caagtcccgt 720
   cgcttagaaa accttatcgc gcagctgccc ggcgagaaaa agaatggttt gtttgggaac 780
   cttattgcct tgagcttagg cctcaccccg aatttcaaaa gtaatttcga tcttgcagaa 840
   gacgccaaat tacaactgtc gaaggatact tatgatgacg atctcgataa tctgttagcg 900
   cagattggtg accaatacgc cgatcttttt ctggcggcta aaaatctgag cgacgccatc 960
   ttgctttcgg atattctccg cgttaacacc gaaatcacga aagcgcctct tagtgccagc 1020
   atgattaaac gttatgatga acaccaccag gacctgacct tactcaaagc gttggttcgc 1080
   cagcaactgc cagagaagta caaagaaatc ttctttgatc agtcaaagaa tggttatgcc 1140
   ggctatattg acgggggtgc aagccaagag gaattctaca aatttatcaa gcctattctg 1200
   gagaaaatgg atggcaccga agagttattg gtgaagctta accgtgaaga cctcctgcgg 1260
   aaacagcgca cattcgataa tggttcgatc ccacaccaaa tccatttggg ggagttacac 1320
   gctattttgc gtcgccagga agacttttac cctttcctga aggataaccg ggagaaaatt 1380
   gagaagatcc ttacctttcg tattccgtat tacgtaggcc ccttagcacg gggtaatagc 1440
   cgtttcgcgt ggatgacacg gaagtcggaa gagacgatca ccccgtggaa cttcgaagag 1500
   gtagtcgaca agggcgcatc agcgcagtct tttattgaac gtatgacgaa tttcgataaa 1560
   aacttgccca atgagaaggt gcttccgaaa cattccttgt tatatgaata ttttacagtt 1620
   tacaacgagc tgaccaaggt taaatacgtg acggaaggaa tgcgcaagcc cgcttttctt 1680
   agcggtgagc aaaaaaaggc gatcgtcgac ctgttattca aaacgaatcg taaggtgact 1740
   gtaaagcaac tcaaagaaga ttacttcaaa aagattgagt gcttcgacag cgtcgaaatc 1800
   tctggggtag aggatcggtt taacgcaagt ttaggtacct accatgacct gcttaaaatc 1860
   attaaggata aagacttctt agataatgaa gagaacgaag atattctcga ggacatcgtc 1920
   ttgacgttaa ccttatttga ggatcgtgaa atgattgagg aacgcctcaa aacttatgcc 1980
   cacctgttcg acgataaggt gatgaagcag ctgaaacgtc ggcgctacac aggatggggc 2040
   cgcttgagtc gcaaacttat taacggaatc cgtgacaagc aatccggcaa aacgattctg 2100
   gatttcttga agtcggacgg atttgctaat cgcaacttca tgcagttgat ccatgatgac 2160
   tccctgactt ttaaagagga tattcaaaag gcgcaggtta gtggtcaagg cgacagctta 2220
   cacgaacaca tcgcaaattt ggctggttcg ccggccatta aaaaggggat cctccagacc 2280
   gtgaaagttg tagatgagct tgttaaggtc atgggtcgtc ataagcccga aaacatcgtg 2340
   attgaaatgg cgcgggagaa tcaaacgacc cagaaaggac aaaagaatag ccgtgaacgg 2400
   atgaagcgga tcgaggaagg cattaaagag ctggggtctc aaatcttgaa ggaacaccct 2460
   gtggagaaca ctcagctcca aaatgaaaaa ctttacctgt actatttgca gaacggacgc 2520
   gatatgtacg tggaccaaga gttggatatt aatcggctga gtgactacga cgttgatcat 2580
   atcgtcccgc agagcttcct caaagacgat tctattgaca ataaggtact gacgcgctct 2640
   gataaaaacc gtggtaagtc ggacaacgtg ccctccgaag aggttgtgaa aaagatgaaa 2700
   aattattggc gccagctttt aaacgcgaag ctgatcacac aacgtaaatt cgataatttg 2760
   accaaggctg aacggggtgg cctgagcgag ttagataagg caggatttat taaacgccag 2820
   ttagtggaga ctcgtcaaat caccaaacat gtcgcgcaga ttttggacag ccggatgaac 2880
   accaagtacg atgaaaatga caaactgatc cgtgaggtga aagtcattac tctgaagtcc 2940
   aaattagtta gtgatttccg gaaggacttt caattctaca aagtccgtga aattaataac 3000
   tatcatcacg cacatgacgc gtacctgaat gcagtggttg ggaccgccct tatcaagaaa 3060
   tatcctaagc tggagtcgga gtttgtctat ggcgactata aggtatacga tgttcgcaaa 3120
   atgattgcga aatctgagca ggagatcggt aaggcaaccg caaaatattt cttttactca 3180
   aacattatga atttctttaa gacagaaatc actctggcca acggggagat tcgcaaacgt 3240
   ccgttgatcg aaacaaacgg cgagactggc gaaattgttt gggacaaagg gcgtgatttc 3300
   gcgacggtgc gcaaggtact gagcatgcct caagtcaata ttgttaagaa aaccgaagtg 3360
   cagacgggcg ggttttccaa ggaaagcatc ttacccaaac gtaattcaga taaacttatt 3420
   gcacgcaaaa aggactggga tccgaaaaag tatggaggct tcgacagtcc aaccgtagcc 3480
   tactctgttc tcgttgtagc gaaagtagaa aagggtaaat ccaagaaact gaaatctgtc 3540
   aaggagttgc ttggaatcac cattatggag cgtagctcct tcgagaagaa cccgattgac 3600
   tttctggaag ccaaaggata taaagaggtc aagaaagatc ttatcattaa gctgcctaag 3660
   tattcactct tcgagctgga aaatggtcgt aaacgcatgc tcgcttctgc cggcgagttg 3720
   cagaagggca atgaattagc acttccatca aagtacgtta acttcctgta tttggccagc 3780
   cattacgaga aactgaaggg gtctccagag gacaacgaac agaaacaatt atttgtagag 3840
   cagcacaagc attatcttga tgaaatcatt gagcaaattt ccgaattcag taaacgcgta 3900
   atcctggccg atgcaaacct cgacaaggtg ctgagcgctt acaataagca tcgcgacaaa 3960
   cctatccgtg agcaggctga aaatatcatt cacctgttca cattaacgaa cctgggcgct 4020
   ccggccgctt ttaaatattt cgacacgaca atcgaccgta agcgctatac cagtacgaaa 4080
   gaagtgttgg atgcgaccct tattcaccag tcaattacag gattatatga gacccgtatc 4140
   gaccttagcc aattaggtgg ggatgcggcc ccgaagaaaa aacgcaaagt ggatccgaag 4200
   aaaaaacgca aagtggcggc cgcactcgag caccaccacc accaccactg a 4251
<210> 410
   <211> 1429
   <212> PRT
   <213> Artificial Sequence
<220>
   <221> source
   <223> /note="Description of Artificial Sequence: Synthetic polypeptide"
<400> 410
   Met Gly Lys Pro Ile Pro Asn Pro Leu Leu Gly Leu Asp Ser Thr Ala
   1               5                   10                  15
   Pro Lys Lys Lys Arg Lys Val Gly Ile His Gly Val Pro Ala Ala Asp
               20                  25                  30
   Lys Lys Tyr Ser Ile Gly Leu Asp Ile Gly Thr Asn Ser Val Gly Trp
           35                  40                  45
   Ala Val Ile Thr Asp Glu Tyr Lys Val Pro Ser Lys Lys Phe Lys Val
       50                  55                  60
   Leu Gly Asn Thr Asp Arg His Ser Ile Lys Lys Asn Leu Ile Gly Ala
   65                  70                  75                  80
   Leu Leu Phe Asp Ser Gly Glu Thr Ala Glu Ala Thr Arg Leu Lys Arg
                   85                  90                  95
   Thr Ala Arg Arg Arg Tyr Thr Arg Arg Lys Asn Arg Ile Cys Tyr Leu
               100                 105                 110
   Gln Glu Ile Phe Ser Asn Glu Met Ala Lys Val Asp Asp Ser Phe Phe
           115                 120                 125
   His Arg Leu Glu Glu Ser Phe Leu Val Glu Glu Asp Lys Lys His Glu
       130                 135                 140
   Arg His Pro Ile Phe Gly Asn Ile Val Asp Glu Val Ala Tyr His Glu
   145                 150                 155                 160
   Lys Tyr Pro Thr Ile Tyr His Leu Arg Lys Lys Leu Val Asp Ser Thr
                   165                 170                 175
   Asp Lys Ala Asp Leu Arg Leu Ile Tyr Leu Ala Leu Ala His Met Ile
               180                 185                 190
   Lys Phe Arg Gly His Phe Leu Ile Glu Gly Asp Leu Asn Pro Asp Asn
           195                 200                 205
   Ser Asp Val Asp Lys Leu Phe Ile Gln Leu Val Gln Thr Tyr Asn Gln
       210                 215                 220
   Leu Phe Glu Glu Asn Pro Ile Asn Ala Ser Gly Val Asp Ala Lys Ala
   225                 230                 235                 240
   Ile Leu Ser Ala Arg Leu Ser Lys Ser Arg Arg Leu Glu Asn Leu Ile
   245 250 255
   Ala Gln Leu Pro Gly Glu Lys Lys Asn Gly Leu Phe Gly Asn Leu Ile
   260 265 270
   Ala Leu Ser Leu Gly Leu Thr Pro Asn Phe Lys Ser Asn Phe Asp Leu
   275 280 285
   Ala Glu Asp Ala Lys Leu Gln Leu Ser Lys Asp Thr Tyr Asp Asp Asp
   290 295 300
   Leu Asp Asn Leu Leu Ala Gln Ile Gly Asp Gln Tyr Ala Asp Leu Phe
   305 310 315 320
   Leu Ala Ala Lys Asn Leu Ser Asp Ala Ile Leu Leu Ser Asp Ile Leu
   325 330 335
   Arg Val Asn Thr Glu Ile Thr Lys Ala Pro Leu Ser Ala Ser Met Ile
   340 345 350
   Lys Arg Tyr Asp Glu His His Gln Asp Leu Thr Leu Leu Lys Ala Leu
   355 360 365
   Val Arg Gln Gln Leu Pro Glu Lys Tyr Lys Glu Ile Phe Phe Asp Gln
   370 375 380
   Ser Lys Asn Gly Tyr Ala Gly Tyr Ile Asp Gly Gly Ala Ser Gln Glu
   385 390 395 400
   Glu Phe Tyr Lys Phe Ile Lys Pro Ile Leu Glu Lys Met Asp Gly Thr
   405 410 415
   Glu Glu Leu Leu Val Lys Leu Asn Arg Glu Asp Leu Leu Arg Lys Gln
   420 425 430
   Arg Thr Phe Asp Asn Gly Ser Ile Pro His Gln Ile His Leu Gly Glu
   435 440 445
   Leu His Ala Ile Leu Arg Arg Gln Glu Asp Phe Tyr Pro Phe Leu Lys
   450 455 460
   Asp Asn Arg Glu Lys Ile Glu Lys Ile Leu Thr Phe Arg Ile Pro Tyr
   465 470 475 480
   Tyr Val Gly Pro Leu Ala Arg Gly Asn Ser Arg Phe Ala Trp Met Thr
   485 490 495
   Arg Lys Ser Glu Glu Thr Ile Thr Pro Trp Asn Phe Glu Glu Val Val
   500 505 510
   Asp Lys Gly Ala Ser Ala Gln Ser Phe Ile Glu Arg Met Thr Asn Phe
   515 520 525
   Asp Lys Asn Leu Pro Asn Glu Lys Val Leu Pro Lys His Ser Leu Leu
   530 535 540
   Tyr Glu Tyr Phe Thr Val Tyr Asn Glu Leu Thr Lys Val Lys Tyr Val
   545 550 555 560
   Thr Glu Gly Met Arg Lys Pro Ala Phe Leu Ser Gly Glu Gln Lys Lys
   565 570 575
   Ala Ile Val Asp Leu Leu Phe Lys Thr Asn Arg Lys Val Thr Val Lys
   580 585 590
   Gln Leu Lys Glu Asp Tyr Phe Lys Lys Ile Glu Cys Phe Asp Ser Val
   595 600 605
   Glu Ile Ser Gly Val Glu Asp Arg Phe Asn Ala Ser Leu Gly Thr Tyr
   610 615 620
   His Asp Leu Leu Lys Ile Ile Lys Asp Lys Asp Phe Leu Asp Asn Glu
   625 630 635 640
   Glu Asn Glu Asp Ile Leu Glu Asp Ile Val Leu Thr Leu Thr Leu Phe
   645 650 655
   Glu Asp Arg Glu Met Ile Glu Glu Arg Leu Lys Thr Tyr Ala His Leu
   660 665 670
   Phe Asp Asp Lys Val Met Lys Gln Leu Lys Arg Arg Arg Tyr Thr Gly
   675 680 685
   Trp Gly Arg Leu Ser Arg Lys Leu Ile Asn Gly Ile Arg Asp Lys Gln
   690 695 700
   Ser Gly Lys Thr Ile Leu Asp Phe Leu Lys Ser Asp Gly Phe Ala Asn
   705 710 715 720
   Arg Asn Phe Met Gln Leu Ile His Asp Asp Ser Leu Thr Phe Lys Glu
   725 730 735
   Asp Ile Gln Lys Ala Gln Val Ser Gly Gln Gly Asp Ser Leu His Glu
   740 745 750
   His Ile Ala Asn Leu Ala Gly Ser Pro Ala Ile Lys Lys Gly Ile Leu
   755 760 765
   Gln Thr Val Lys Val Val Asp Glu Leu Val Lys Val Met Gly Arg His
   770 775 780
   Lys Pro Glu Asn Ile Val Ile Glu Met Ala Arg Glu Asn Gln Thr Thr
   785 790 795 800
   Gln Lys Gly Gln Lys Asn Ser Arg Glu Arg Met Lys Arg Ile Glu Glu
   805 810 815
   Gly Ile Lys Glu Leu Gly Ser Gln Ile Leu Lys Glu His Pro Val Glu
   820 825 830
   Asn Thr Gln Leu Gln Asn Glu Lys Leu Tyr Leu Tyr Tyr Leu Gln Asn
   835 840 845
   Gly Arg Asp Met Tyr Val Asp Gln Glu Leu Asp Ile Asn Arg Leu Ser
   850 855 860
   Asp Tyr Asp Val Asp His Ile Val Pro Gln Ser Phe Leu Lys Asp Asp
   865 870 875 880
   Ser Ile Asp Asn Lys Val Leu Thr Arg Ser Asp Lys Asn Arg Gly Lys
   885 890 895
   Ser Asp Asn Val Pro Ser Glu Glu Val Val Lys Lys Met Lys Asn Tyr
   900 905 910
   Trp Arg Gln Leu Leu Asn Ala Lys Leu Ile Thr Gln Arg Lys Phe Asp
   915 920 925
   Asn Leu Thr Lys Ala Glu Arg Gly Gly Leu Ser Glu Leu Asp Lys Ala
   930 935 940
   Gly Phe Ile Lys Arg Gln Leu Val Glu Thr Arg Gln Ile Thr Lys His
   945 950 955 960
   Val Ala Gln Ile Leu Asp Ser Arg Met Asn Thr Lys Tyr Asp Glu Asn
   965 970 975
   Asp Lys Leu Ile Arg Glu Val Lys Val Ile Thr Leu Lys Ser Lys Leu
   980 985 990
   Val Ser Asp Phe Arg Lys Asp Phe Gln Phe Tyr Lys Val Arg Glu Ile
   995 1000 1005
   Asn Asn Tyr His His Ala His Asp Ala Tyr Leu Asn Ala Val Val
   1010 1015 1020
   Gly Thr Ala Leu Ile Lys Lys Tyr Pro Lys Leu Glu Ser Glu Phe
   1025 1030 1035
   Val Tyr Gly Asp Tyr Lys Val Tyr Asp Val Arg Lys Met Ile Ala
   1040 1045 1050
   Lys Ser Glu Gln Glu Ile Gly Lys Ala Thr Ala Lys Tyr Phe Phe
   1055 1060 1065
   Tyr Ser Asn Ile Met Asn Phe Phe Lys Thr Glu Ile Thr Leu Ala
   1070 1075 1080
   Asn Gly Glu Ile Arg Lys Arg Pro Leu Ile Glu Thr Asn Gly Glu
   1085 1090 1095
   Thr Gly Glu Ile Val Trp Asp Lys Gly Arg Asp Phe Ala Thr Val
   1100 1105 1110
   Arg Lys Val Leu Ser Met Pro Gln Val Asn Ile Val Lys Lys Thr
   1115 1120 1125
   Glu Val Gln Thr Gly Gly Phe Ser Lys Glu Ser Ile Leu Pro Lys
   1130 1135 1140
   Arg Asn Ser Asp Lys Leu Ile Ala Arg Lys Lys Asp Trp Asp Pro
   1145 1150 1155
   Lys Lys Tyr Gly Gly Phe Asp Ser Pro Thr Val Ala Tyr Ser Val
   1160 1165 1170
   Leu Val Val Ala Lys Val Glu Lys Gly Lys Ser Lys Lys Leu Lys
   1175 1180 1185
   Ser Val Lys Glu Leu Leu Gly Ile Thr Ile Met Glu Arg Ser Ser
   1190 1195 1200
   Phe Glu Lys Asn Pro Ile Asp Phe Leu Glu Ala Lys Gly Tyr Lys
   1205 1210 1215
   Glu Val Lys Lys Asp Leu Ile Ile Lys Leu Pro Lys Tyr Ser Leu
   1220 1225 1230
   Phe Glu Leu Glu Asn Gly Arg Lys Arg Met Leu Ala Ser Ala Gly
   1235 1240 1245
   Glu Leu Gln Lys Gly Asn Glu Leu Ala Leu Pro Ser Lys Tyr Val
   1250 1255 1260
   Asn Phe Leu Tyr Leu Ala Ser His Tyr Glu Lys Leu Lys Gly Ser
   1265 1270 1275
   Pro Glu Asp Asn Glu Gln Lys Gln Leu Phe Val Glu Gln His Lys
   1280 1285 1290
   His Tyr Leu Asp Glu Ile Ile Glu Gln Ile Ser Glu Phe Ser Lys
   1295 1300 1305
   Arg Val Ile Leu Ala Asp Ala Asn Leu Asp Lys Val Leu Ser Ala
   1310 1315 1320
   Tyr Asn Lys His Arg Asp Lys Pro Ile Arg Glu Gln Ala Glu Asn
   1325 1330 1335
   Ile Ile His Leu Phe Thr Leu Thr Asn Leu Gly Ala Pro Ala Ala
   1340 1345 1350
   Phe Lys Tyr Phe Asp Thr Thr Ile Asp Arg Lys Arg Tyr Thr Ser
   1355 1360 1365
   Thr Lys Glu Val Leu Asp Ala Thr Leu Ile His Gln Ser Ile Thr
   1370 1375 1380
   Gly Leu Tyr Glu Thr Arg Ile Asp Leu Ser Gln Leu Gly Gly Asp
   1385 1390 1395
   Ser Arg Ala Asp Pro Lys Lys Lys Arg Lys Val Glu Phe His His
   1400 1405 1410
   Thr Gly Leu Val Asp Pro Ser Ser Val Pro Ser Leu Ser Leu Asn
   1415 1420 1425
   Arg
<210> 411
   <211> 4290
   <212> DNA
   <213> Artificial Sequence
<220>
   <221> source
   <223> /note="Description of Artificial Sequence: Synthetic polynucleotide"
<400> 411
   atgggcaagc ccatccctaa ccccctgttg gggctggaca gcaccgctcc caaaaagaaa      60
   aggaaggtgg gcattcacgg cgtgcctgcg gccgacaaaa agtacagcat cggccttgat     120
   atcggcacca atagcgtggg ctgggccgtt atcacagacg aatacaaggt acccagcaag     180
   aagttcaagg tgctggggaa tacagacagg cactctatca agaaaaacct tatcggggct     240
   ctgctgtttg actcaggcga gaccgccgag gccaccaggt tgaagaggac cgcaaggcga     300
   aggtacaccc ggaggaagaa caggatctgc tatctgcagg agatcttcag caacgagatg     360
   gccaaggtgg acgacagctt cttccacagg ctggaggaga gcttccttgt cgaggaggat     420
   aagaagcacg aacgacaccc catcttcggc aacatagtcg acgaggtcgc ttatcacgag     480
   aagtacccca ccatctacca cctgcgaaag aaattggtgg atagcaccga taaagccgac     540
   ttgcgactta tctacttggc tctggcgcac atgattaagt tcaggggcca cttcctgatc     600
   gagggcgacc ttaaccccga caacagtgac gtagacaaat tgttcatcca gcttgtacag     660
   acctataacc agctgttcga ggaaaaccct attaacgcca gcggggtgga tgcgaaggcc     720
   atacttagcg ccaggctgag caaaagcagg cgcttggaga acctgatagc ccagctgccc     780
   ggtgaaaaga agaacggcct cttcggtaat ctgattgccc tgagcctggg cctgaccccc     840
   aacttcaaga gcaacttcga cctggcagaa gatgccaagc tgcagttgag taaggacacc     900
   tatgacgacg acttggacaa tctgctcgcc caaatcggcg accagtacgc tgacctgttc     960
   ctcgccgcca agaacctttc tgacgcaatc ctgcttagcg atatccttag ggtgaacaca    1020
   gagatcacca aggcccccct gagcgccagc atgatcaaga ggtacgacga gcaccatcag    1080
   gacctgaccc ttctgaaggc cctggtgagg cagcaactgc ccgagaagta caaggagatc    1140
   tttttcgacc agagcaagaa cggctacgcc ggctacatcg acggcggagc cagccaagag    1200
   gagttctaca agttcatcaa gcccatcctg gagaagatgg atggcaccga ggagctgctg    1260
   gtgaagctga acagggaaga tttgctccgg aagcagagga cctttgacaa cggtagcatc    1320
   ccccaccaga tccacctggg cgagctgcac gcaatactga ggcgacagga ggatttctac    1380
   cccttcctca aggacaatag ggagaaaatc gaaaagattc tgaccttcag gatcccctac    1440
   tacgtgggcc ctcttgccag gggcaacagc cgattcgctt ggatgacaag aaagagcgag    1500
   gagaccatca ccccctggaa cttcgaggaa gtggtggaca aaggagcaag cgcgcagtct    1560
   ttcatcgaac ggatgaccaa tttcgacaaa aacctgccta acgagaaggt gctgcccaag    1620
   cacagcctgc tttacgagta cttcaccgtg tacaacgagc tcaccaaggt gaaatatgtg    1680
   accgagggca tgcgaaaacc cgctttcctg agcggcgagc agaagaaggc catcgtggac    1740
   ctgctgttca agaccaacag gaaggtgacc gtgaagcagc tgaaggagga ctacttcaag 1800
   aagatcgagt gctttgatag cgtggaaata agcggcgtgg aggacaggtt caacgccagc 1860
   ctgggcacct accacgactt gttgaagata atcaaagaca aggatttcct ggataatgag 1920
   gagaacgagg atatactcga ggacatcgtg ctgactttga ccctgtttga ggaccgagag 1980
   atgattgaag aaaggctcaa aacctacgcc cacctgttcg acgacaaagt gatgaaacaa 2040
   ctgaagagac gaagatacac cggctggggc agactgtcca ggaagctcat caacggcatt 2100
   agggacaagc agagcggcaa gaccatcctg gatttcctga agtccgacgg cttcgccaac 2160
   cgaaacttca tgcagctgat tcacgatgac agcttgacct tcaaggagga catccagaag 2220
   gcccaggtta gcggccaggg cgactccctg cacgaacata ttgcaaacct ggcaggctcc 2280
   cctgcgatca agaagggcat actgcagacc gttaaggttg tggacgaatt ggtcaaggtc 2340
   atgggcaggc acaagcccga aaacatagtt atagagatgg ccagagagaa ccagaccacc 2400
   caaaagggcc agaagaacag ccgggagcgc atgaaaagga tcgaggaggg tatcaaggaa 2460
   ctcggaagcc agatcctcaa agagcacccc gtggagaata cccagctcca gaacgagaag 2520
   ctgtacctgt actacctgca gaacggcagg gacatgtacg ttgaccagga gttggacatc 2580
   aacaggcttt cagactatga cgtggatcac atagtgcccc agagctttct taaagacgat 2640
   agcatcgaca acaaggtcct gacccgctcc gacaaaaaca ggggcaaaag cgacaacgtg 2700
   ccaagcgaag aggtggttaa aaagatgaag aactactgga ggcaactgct caacgcgaaa 2760
   ttgatcaccc agagaaagtt cgataacctg accaaggccg agaggggcgg actctccgaa 2820
   cttgacaaag cgggcttcat aaagaggcag ctggtcgaga cccgacagat cacgaagcac 2880
   gtggcccaaa tcctcgacag cagaatgaat accaagtacg atgagaatga caaactcatc 2940
   agggaagtga aagtgattac cctgaagagc aagttggtgt ccgactttcg caaagatttc 3000
   cagttctaca aggtgaggga gatcaacaac taccaccatg cccacgacgc atacctgaac 3060
   gccgtggtcg gcaccgccct gattaagaag tatccaaagc tggagtccga atttgtctac 3120
   ggcgactaca aagtttacga tgtgaggaag atgatcgcta agagcgaaca ggagatcggc 3180
   aaggccaccg ctaagtattt cttctacagc aacatcatga actttttcaa gaccgagatc 3240
   acacttgcca acggcgaaat caggaagagg ccgcttatcg agaccaacgg tgagaccggc 3300
   gagatcgtgt gggacaaggg cagggacttc gccaccgtga ggaaagtcct gagcatgccc 3360
   caggtgaata ttgtgaaaaa aactgaggtg cagacaggcg gctttagcaa ggaatccatc 3420
   ctgcccaaga ggaacagcga caagctgatc gcccggaaga aggactggga ccctaagaag 3480
   tatggaggct tcgacagccc caccgtagcc tacagcgtgc tggtggtcgc gaaggtagag 3540
   aaggggaaga gcaagaaact gaagagcgtg aaggagctgc tcggcataac catcatggag 3600
   aggtccagct ttgagaagaa ccccattgac tttttggaag ccaagggcta caaagaggtc      3660
   aaaaaggacc tgatcatcaa actccccaag tactccctgt ttgaattgga gaacggcaga      3720
   aagaggatgc tggcgagcgc tggggaactg caaaagggca acgaactggc gctgcccagc      3780
   aagtacgtga attttctgta cctggcgtcc cactacgaaa agctgaaagg cagccccgag      3840
   gacaacgagc agaagcagct gttcgtggag cagcacaagc attacctgga cgagataatc      3900
   gagcaaatca gcgagttcag caagagggtg attctggccg acgcgaacct ggataaggtc      3960
   ctcagcgcct acaacaagca ccgagacaaa cccatcaggg agcaggccga gaatatcata      4020
   cacctgttca ccctgacaaa tctgggcgca cctgcggcat tcaaatactt cgataccacc      4080
   atcgacagga aaaggtacac tagcactaag gaggtgctgg atgccacctt gatccaccag      4140
   tccattaccg gcctgtatga gaccaggatc gacctgagcc agcttggagg cgactctagg      4200
   gcggacccaa aaaagaaaag gaaggtggaa ttccaccaca ctggactagt ggatccgagc      4260
   tcggtaccaa gcttaagttt aaaccgctga                                       4290
<210> 412
   <211> 4601
   <212> DNA
   <213> Artificial Sequence
<220>
   <221> source
   <223> /note="Description of Artificial Sequence: Synthetic polynucleotide"
<400> 412
   taatacgact cactataggg agacccaagc tggctagcgt ttaaacgggc cctctagact 60
   cgagcggccg ccaccatggg caagcccatc cctaaccccc tgttggggct ggacagcacc 120
   gctcccaaaa agaaaaggaa ggtgggcatt cacggcgtgc ctgcggccga caaaaagtac 180
   agcatcggcc ttgatatcgg caccaatagc gtgggctggg ccgttatcac agacgaatac 240
   aaggtaccca gcaagaagtt caaggtgctg gggaatacag acaggcactc tatcaagaaa 300
   aaccttatcg gggctctgct gtttgactca ggcgagaccg ccgaggccac caggttgaag 360
   aggaccgcaa ggcgaaggta cacccggagg aagaacagga tctgctatct gcaggagatc 420
   ttcagcaacg agatggccaa ggtggacgac agcttcttcc acaggctgga ggagagcttc 480
   cttgtcgagg aggataagaa gcacgaacga caccccatct tcggcaacat agtcgacgag 540
   gtcgcttatc acgagaagta ccccaccatc taccacctgc gaaagaaatt ggtggatagc 600
   accgataaag ccgacttgcg acttatctac ttggctctgg cgcacatgat taagttcagg 660
   ggccacttcc tgatcgaggg cgaccttaac cccgacaaca gtgacgtaga caaattgttc 720
   atccagcttg tacagaccta taaccagctg ttcgaggaaa accctattaa cgccagcggg 780
   gtggatgcga aggccatact tagcgccagg ctgagcaaaa gcaggcgctt ggagaacctg 840
   atagcccagc tgcccggtga aaagaagaac ggcctcttcg gtaatctgat tgccctgagc 900
   ctgggcctga cccccaactt caagagcaac ttcgacctgg cagaagatgc caagctgcag 960
   ttgagtaagg acacctatga cgacgacttg gacaatctgc tcgcccaaat cggcgaccag 1020
   tacgctgacc tgttcctcgc cgccaagaac ctttctgacg caatcctgct tagcgatatc 1080
   cttagggtga acacagagat caccaaggcc cccctgagcg ccagcatgat caagaggtac 1140
   gacgagcacc atcaggacct gacccttctg aaggccctgg tgaggcagca actgcccgag 1200
   aagtacaagg agatcttttt cgaccagagc aagaacggct acgccggcta catcgacggc 1260
   ggagccagcc aagaggagtt ctacaagttc atcaagccca tcctggagaa gatggatggc 1320
   accgaggagc tgctggtgaa gctgaacagg gaagatttgc tccggaagca gaggaccttt 1380
   gacaacggta gcatccccca ccagatccac ctgggcgagc tgcacgcaat actgaggcga 1440
   caggaggatt tctacccctt cctcaaggac aatagggaga aaatcgaaaa gattctgacc 1500
   ttcaggatcc cctactacgt gggccctctt gccaggggca acagccgatt cgcttggatg 1560
   acaagaaaga gcgaggagac catcaccccc tggaacttcg aggaagtggt ggacaaagga 1620
   gcaagcgcgc agtctttcat cgaacggatg accaatttcg acaaaaacct gcctaacgag 1680
   aaggtgctgc ccaagcacag cctgctttac gagtacttca ccgtgtacaa cgagctcacc 1740
   aaggtgaaat atgtgaccga gggcatgcga aaacccgctt tcctgagcgg cgagcagaag 1800
   aaggccatcg tggacctgct gttcaagacc aacaggaagg tgaccgtgaa gcagctgaag 1860
   gaggactact tcaagaagat cgagtgcttt gatagcgtgg aaataagcgg cgtggaggac 1920
   aggttcaacg ccagcctggg cacctaccac gacttgttga agataatcaa agacaaggat 1980
   ttcctggata atgaggagaa cgaggatata ctcgaggaca tcgtgctgac tttgaccctg 2040
   tttgaggacc gagagatgat tgaagaaagg ctcaaaacct acgcccacct gttcgacgac 2100
   aaagtgatga aacaactgaa gagacgaaga tacaccggct ggggcagact gtccaggaag 2160
   ctcatcaacg gcattaggga caagcagagc ggcaagacca tcctggattt cctgaagtcc 2220
   gacggcttcg ccaaccgaaa cttcatgcag ctgattcacg atgacagctt gaccttcaag 2280
   gaggacatcc agaaggccca ggttagcggc cagggcgact ccctgcacga acatattgca 2340
   aacctggcag gctcccctgc gatcaagaag ggcatactgc agaccgttaa ggttgtggac 2400
   gaattggtca aggtcatggg caggcacaag cccgaaaaca tagttataga gatggccaga 2460
   gagaaccaga ccacccaaaa gggccagaag aacagccggg agcgcatgaa aaggatcgag 2520
   gagggtatca aggaactcgg aagccagatc ctcaaagagc accccgtgga gaatacccag 2580
   ctccagaacg agaagctgta cctgtactac ctgcagaacg gcagggacat gtacgttgac 2640
   caggagttgg acatcaacag gctttcagac tatgacgtgg atcacatagt gccccagagc 2700
   tttcttaaag acgatagcat cgacaacaag gtcctgaccc gctccgacaa aaacaggggc 2760
   aaaagcgaca acgtgccaag cgaagaggtg gttaaaaaga tgaagaacta ctggaggcaa 2820
   ctgctcaacg cgaaattgat cacccagaga aagttcgata acctgaccaa ggccgagagg 2880
   ggcggactct ccgaacttga caaagcgggc ttcataaaga ggcagctggt cgagacccga 2940
   cagatcacga agcacgtggc ccaaatcctc gacagcagaa tgaataccaa gtacgatgag 3000
   aatgacaaac tcatcaggga agtgaaagtg attaccctga agagcaagtt ggtgtccgac 3060
   tttcgcaaag atttccagtt ctacaaggtg agggagatca acaactacca ccatgcccac 3120
   gacgcatacc tgaacgccgt ggtcggcacc gccctgatta agaagtatcc aaagctggag 3180
   tccgaatttg tctacggcga ctacaaagtt tacgatgtga ggaagatgat cgctaagagc 3240
   gaacaggaga tcggcaaggc caccgctaag tatttcttct acagcaacat catgaacttt 3300
   ttcaagaccg agatcacact tgccaacggc gaaatcagga agaggccgct tatcgagacc 3360
   aacggtgaga ccggcgagat cgtgtgggac aagggcaggg acttcgccac cgtgaggaaa 3420
   gtcctgagca tgccccaggt gaatattgtg aaaaaaactg aggtgcagac aggcggcttt 3480
   agcaaggaat ccatcctgcc caagaggaac agcgacaagc tgatcgcccg gaagaaggac 3540
   tgggacccta agaagtatgg aggcttcgac agccccaccg tagcctacag cgtgctggtg 3600
   gtcgcgaagg tagagaaggg gaagagcaag aaactgaaga gcgtgaagga gctgctcggc 3660
   ataaccatca tggagaggtc cagctttgag aagaacccca ttgacttttt ggaagccaag 3720
   ggctacaaag aggtcaaaaa ggacctgatc atcaaactcc ccaagtactc cctgtttgaa 3780
   ttggagaacg gcagaaagag gatgctggcg agcgctgggg aactgcaaaa gggcaacgaa 3840
   ctggcgctgc ccagcaagta cgtgaatttt ctgtacctgg cgtcccacta cgaaaagctg 3900
   aaaggcagcc ccgaggacaa cgagcagaag cagctgttcg tggagcagca caagcattac 3960
   ctggacgaga taatcgagca aatcagcgag ttcagcaaga gggtgattct ggccgacgcg 4020
   aacctggata aggtcctcag cgcctacaac aagcaccgag acaaacccat cagggagcag 4080
   gccgagaata tcatacacct gttcaccctg acaaatctgg gcgcacctgc ggcattcaaa 4140
   tacttcgata ccaccatcga caggaaaagg tacactagca ctaaggaggt gctggatgcc 4200
   accttgatcc accagtccat taccggcctg tatgagacca ggatcgacct gagccagctt 4260
   ggaggcgact ctagggcgga cccaaaaaag aaaaggaagg tggaattcca ccacactgga 4320
   ctagtggatc cgagctcggt accaagctta agtttaaacc gctgatcagc ctcgactgtg 4380
   ccttctagtt gccagccatc tgttgtttgc ccctcccccg tgccttcctt gaccctggaa 4440
   ggtgccactc ccactgtcct ttcctaataa aatgaggaaa ttgcatcgca ttgtctgagt 4500
   aggtgtcatt ctattctggg gggtggggtg gggcaggaca gcaaggggga ggattgggaa 4560
   gacaatagca ggcatgctgg ggatgcggtg ggctctatgg c 4601
<210> 413
   <211> 4584
   <212> RNA
   <213> Artificial Sequence
<220>
   <221> source
   <223> /note="Description of Artificial Sequence: Synthetic polynucleotide"
<400> 413
<210> 414
   <211> 104
   <212> RNA
   <213> Artificial Sequence
<220>
   <221> source
   <223> /note="Description of Artificial Sequence: Synthetic polynucleotide"
<220>
   <221> source
   <223> /note="5' Triphosphate"
<400> 414
   gcuuauaucc aacacuucgu gguuuuagag cuagaaauag caaguuaaaa uaaggcuagu 60
   ccguuaucaa cuugaaaaag uggcaccgag ucggugcuuu uuuu 104
<210> 415
   <211> 21
   <212> DNA
   <213> Artificial Sequence
<220>
   <221> source
   <223> /note="Description of Artificial Sequence: Synthetic oligonucleotide"
<400> 415
   gactttgctt tccttggtca g 21
<210> 416
   <211> 24
   <212> DNA
   <213> Artificial Sequence
<220>
   <221> source
   <223> /note="Description of Artificial Sequence: Synthetic oligonucleotide"
<400> 416
   ggcttatatc caacacttcg tggg 24
<210> 417
   <211> 9
   <212> DNA
   <213> Artificial Sequence
<220>
   <221> source
   <223> /note="Description of Artificial Sequence: Synthetic oligonucleotide"
<220>
   <221> source
   <223> /note="5' FAM"
<220>
   <221> source
   <223> /note="3' ZEN modifier"
<400> 417
   atggtcaag 9
<210> 418
   <211> 18
   <212> DNA
   <213> Artificial Sequence
<220>
   <221> source
   <223> /note="Description of Artificial Sequence: Synthetic oligonucleotide"
<400> 418
   tgtgcagaag gatggagt 18
<210> 419
   <211> 20
   <212> DNA
   <213> Artificial Sequence
<220>
   <221> source
   <223> /note="Description of Artificial Sequence: Synthetic oligonucleotide"
<400> 419
   ctggtgcttc tctcaggata 20
<210> 420
   <211> 9
   <212> DNA
   <213> Artificial Sequence
<220>
   <221> source
   <223> /note="Description of Artificial Sequence: Synthetic oligonucleotide"
<220>
   <221> source
   <223> /note="5' HEX"
<220>
   <221> source
   <223> /note="3' ZEN modifier"
<400> 420
   tggaatatg 9
<210> 421
   <211> 24
   <212> DNA
   <213> Artificial Sequence
<220>
   <221> source
   <223> /note="Description of Artificial Sequence: Synthetic primer"
<400> 421
   ctcttcttga actggtgctg tctg 24
<210> 422
   <211> 25
   <212> DNA
   <213> Artificial Sequence
<220>
   <221> source
   <223> /note="Description of Artificial Sequence: Synthetic primer "
<400> 422
   caggatgaat ccaatggtca tgagg 25
<210> 423
   <211> 9
   **<212>** DNA
   <213> Artificial Sequence
**<220>**
   <221> source
   <223> /note="Description of Artificial Sequence: Synthetic probe"
<220>
   <221> source
   <223> /note="5' FAM"
**<220>**
   <221> source
   <223> /note="3' ZEN modifier"
<400> 423
   aagtgcctg 9
<210> 424
   <211> 21
   <212> DNA
   <213> Artificial Sequence
<220>
   <221> source
   <223> /note="Description of Artificial Sequence: Synthetic primer"
<400> 424
   ccattgtctg gatttaagcg g 21
<210> 425
   <211> 21
   <212> DNA
   <213> Artificial Sequence
<220>
   <221> source
   <223> /note="Description of Artificial Sequence: Synthetic primer "
<400> 425
   gccacaaaaa atcacaagcc a 21
<210> 426
   <211> 9
   <212> DNA
   <213> Artificial Sequence
<220>
   <221> source
   <223> /note="Description of Artificial Sequence: Synthetic probe"
<220>
   <221> source
   <223> /note="5' HEX modifier"
<220>
   <221> source
   <223> /note="3' ZEN modifier"
<400> 426
   tttctttgc 9
<210> 427
   <211> 64
   <212> RNA
   <213> Artificial Sequence
<220>
   <221> source
   <223> /note="Description of Artificial Sequence: Synthetic oligonucleotide"
<400> 427
   agcauagcaa guuaaaauaa ggcuaguccg ucaacuugaa aaaguggcac cgagucggug 60
   cuuu 64
<210> 428
   <211> 99
   <212> RNA
   <213> Artificial Sequence
<220>
   <221> source
   <223> /note="Description of Artificial Sequence: Synthetic oligonucleotide"
<400> 428
   cuuauaucca acacuucgug guuuuagagc uagaaauagc aaguuaaaau aaggcuaguc 60
   cguuaucaac uugaaaaagu ggcaccgagu cggugcuuu 99
<210> 429
   <211> 36
   <212> RNA
   <213> Artificial Sequence
<220>
   <221> source
   <223> /note="Description of Artificial Sequence: Synthetic oligonucleotide"
<220>
   <221> modified_base
   <222> (1)..(20)
   <223> a, c, u, g, unknown or other
<400> 429
   nnnnnnnnnn nnnnnnnnnn guuuuagagc uaugcu 36
<210> 430
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> modified_base
   <222> (1)..(20)
   <223> a, c, u, g, unknown or other
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 430
   nnnnnnnnnn nnnnnnnnnn guuuuagagc uaugcu 36
<210> 431
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(4)
   <223> 2'-O-methyl nucleotide
<220>
   <221> modified_base
   <222> (1)..(20)
   <223> a, c, u, g, unknown or other
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 431
   nnnnnnnnnn nnnnnnnnnn guuuuagagc uaugcu 36
<210> 432
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(6)
   <223> 2'-O-methyl nucleotide
<220>
   <221> modified_base
   <222> (1)..(20)
   <223> a, c, u, g, unknown or other
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 432
   nnnnnnnnnn nnnnnnnnnn guuuuagagc uaugcu 36
<210> 433
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(8)
   <223> 2'-O-methyl nucleotide
<220>
   <221> modified_base
   <222> (1)..(20)
   <223> a, c, u, g, unknown or other
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 433
   nnnnnnnnnn nnnnnnnnnn guuuuagagc uaugcu 36
<210> 434
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(20)
   <223> a, c, u, g, unknown or other
<220>
   <221> modified_base
   <222> (1)..(10)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 434
   nnnnnnnnnn nnnnnnnnnn guuuuagagc uaugcu 36
<210> 435
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(20)
   <223> a, c, u, g, unknown or other
<220>
   <221> modified_base
   <222> (1)..(12)
   <223> 2'-O-methyl nucleotide
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 435
   nnnnnnnnnn nnnnnnnnnn guuuuagagc uaugcu 36
<210> 436
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(13)
   <223> 2'-O-methyl nucleotide
<220>
   <221> modified_base
   <222> (1)..(20)
   <223> a, c, u, g, unknown or other
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 436
   nnnnnnnnnn nnnnnnnnnn guuuuagagc uaugcu 36
<210> 437
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(15)
   <223> 2'-O-methyl nucleotide
<220>
   <221> modified_base
   <222> (1)..(20)
   <223> a, c, u, g, unknown or other
<220>
   <221> modified_base
   <222> (25)..(36)
   <223> 2'-O-methyl nucleotide
<220>
   <221> misc_feature
   <222> (33)..(36)
   <223> /note="Phosphorothioate linkage"
<400> 437
   nnnnnnnnnn nnnnnnnnnn guuuuagagc uaugcu 36
<210> 438
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' C3 spacer"
<220>
   <221> modified_base
   <222> (1)..(20)
   <223> a, c, u, g, unknown or other
<220>
   <221> source
   <223> /note="3' C3 spacer"
<400> 438
   nnnnnnnnnn nnnnnnnnnn guuuuagagc uaugcu 36
<210> 439
   <211> 36
   <212> RNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> modified_base
   <222> (1)..(20)
   <223> a, c, u, g, unknown or other
<220>
   <221> source
   <223> /note="5' ZEN modifier"
<400> 439
   nnnnnnnnnn nnnnnnnnnn guuuuagagc uaugcu 36
<210> 440
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(20)
   <223> a, c, u, g, unknown or other
<220>
   <221> modified_base
   <222> (1)..(21)
   <223> 2'-O-methyl nucleotide
<220>
   <221> misc_feature
   <222> (33)..(36)
   <223> /note="Phosphorothioate linkage"
<400> 440
   nnnnnnnnnn nnnnnnnnnn guuuuagagc uaugcu 36
<210> 441
   <211> 17
   <212> DNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 441
   gtcgcaagct tgctggt 17
<210> 442
   <211> 16
   <212> DNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 442
   ccctgcgtaa actgga 16
<210> 443
   <211> 18
   <212> DNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 443
   aacatctggg ccctgatt 18
<210> 444
   <211> 18
   <212> DNA
   <213> Artificial Sequence
<220>
   <221> source
   <223> /note="Description of Artificial Sequence: Synthetic oligonucleotide"
<220>
   <221> source
   <223> /note="5' ZEN modifier"
<220>
   <221> source
   <223> /note="3' ZEN modifier"
<400> 444
   ttcccctaag gcaggctg 18
<210> 445
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 445
   aauuaugggg auuacuagga guuuuagagc uaugcu 36
<210> 446
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 446
   uuuuguaauu aacagcuugc guuuuagagc uaugcu 36
<210> 447
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(3)
   <223> 2'-O-methyl nucleotide
<220>
   <221> misc_feature
   <222> (33)..(36)
   <223> /note="Phosphorothioate linkage"
<220>
   <221> modified_base
   <222> (34)..(36)
   <223> 2'-O-methyl nucleotide
<400> 447
   ggucacuuuu aacacaccca guuuuagagc uaugcu 36
<210> 448
   <211> 36
   <212> RNA
   <213> Artificial Sequence
<220>
   <221> source
   <223> /note="Description of Artificial Sequence: Synthetic oligonucleotide"
<220>
   <221> misc_feature
   <222> (1)..(4)
   <223> /note="Phosphorothioate linkage"
<220>
   <221> modified_base
   <222> (1)..(10)
   <223> 2'-fluoro nucleotide
<220>
   <221> modified_base
   <222> (25)..(36)
   <223> 2'-fluoro nucleotide
<220>
   <221> misc_feature
   <222> (33)..(36)
   <223> /note="Phosphorothioate linkage"
<400> 448
   cuuauaucca acacuucgug guuuuagagc uaugcu 36
<210> 449
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1)..(3)
   <223> /note="Phosphorothioate linkage"
<220>
   <221> modified_base
   <222> (1)..(11)
   <223> 2'-fluoro nucleotide
<220>
   <221> modified_base
   <222> (33)..(67)
   <223> 2'-fluoro nucleotide
<220>
   <221> misc_feature
   <222> (65)..(67)
   <223> /note="Phosphorothioate linkage"
<400> 449
   agcauagcaa guuaaaauaa ggcuaguccg uuaucaacuu gaaaaagugg caccgagucg 60
   gugcuuu 67
<210> 450
   <211> 67
   <212> RNA
   <213> Artificial Sequence
<220>
   <221> source
   <223> /note="Description of Artificial Sequence: Synthetic oligonucleotide"
<220>
   <221> misc_feature
   <222> (1)..(3)
   <223> /note="Phosphorothioate linkage"
<220>
   <221> modified_base
   <222> (1)..(9)
   <223> 2'-fluoro nucleotide
<220>
   <221> modified_base
   <222> (58)..(67)
   <223> 2'-fluoro nucleotide
<220>
   <221> misc_feature
   <222> (65)..(67)
   <223> /note="Phosphorothioate linkage"
<400> 450
   agcauagcaa guuaaaauaa ggcuaguccg uuaucaacuu gaaaaagugg caccgagucg 60
   gugcuuu 67
<210> 451
   <211> 62
   <212> RNA
   <213> Artificial Sequence
<220>
   <221> source
   <223> /note="Description of Artificial Sequence: Synthetic oligonucleotide"
<220>
   <221> misc_feature
   <222> (1)..(3)
   <223> /note="Phosphorothioate linkage"
<220>
   <221> modified_base
   <222> (1)..(11)
   <223> 2'-O-methyl nucleotide
<220>
   <221> modified_base
   <222> (14)..(14)
   <223> 2'-O-methyl nucleotide
<220>
   <221> modified_base
   <222> (16)..(17)
   <223> 2'-O-methyl nucleotide
<220>
   <221> modified_base
   <222> (18)..(22)
   <223> 2'-O-methyl nucleotide
<220>
   <221> modified_base
   <222> (25)..(25)
   <223> 2'-O-methyl nucleotide
<220>
   <221> modified_base
   <222> (29)..(29)
   <223> 2'-O-methyl nucleotide
<220>
   <221> modified_base
   <222> (33)..(62)
   <223> 2'-fluoro nucleotide
<220>
   <221> misc_feature
   <222> (60)..(62)
   <223> /note="Phosphorothioate linkage"
<400> 451
   agcauagcaa guuaaaauaa ggcuaguccg uuaucaacuu gaaaaagugg caccgagucg        60
   gu                                                                        62